# FASCICULE DE BREVET EUROPEEN

(11) **EP 2 225 243 B1**
(45) Date de publication et mention de la délivrance du brevet: **16.11.2011**
(21) Numéro de dépôt: 08872982.7
(22) Date de dépôt: 31.12.2008
(51) Int. Cl.: C07D 471/04, A61K 31/437, A61P 35/00, A61P 25/00, A61P 19/10, A61P 9/00, C07F 5/04, C07D 233/70, C07D 233/72, C07D 213/61, C07D 213/24, C07F 5/02, C07F 7/22, A61K 31/444

(54) **Dérivés de N-hétérocyclique-6-hétérocyclique-imidazo[1,2-a]pyridine-2-carboxamides, leur préparation et leur application en thérapeutique**
Derivate aus N-heterozyklischen-6-heterozyklischen Imidazo-[1,2-a]-pyridin-2-carboxamiden, ihre Herstellung und ihre therapeutische Anwendung
Derivatives of N-heterocyclic-6-heterocyclic-imidazo[1,2-a]pyridine-2-carboxamides, preparation thereof and therapeutic application thereof

(30) Priorité: 02.01.2008 FR 0800006
(43) Date de publication de la demande: 08.09.2010
(73) Titulaire: SANOFI, 75013 Paris (FR)
(72) Inventeur: PEYRONEL, Jean-François, F-75013 Paris (FR)
(74) Mandataire: Le Coupanec, Pascale A.M.P.
(86) Numéro de dépôt international: PCT/FR2008/001838
(87) Numéro de publication internationale: WO 2009/106751

(56) Documents cités:
- EP-A1- 1 849 465
- WO-A-2006/099379
- WO-A1-2007/108750
- US-A1- 2005 165 049
- DATABASE CA [Online] CHEMICAL ABSTRACTS SERVICE, COLUMBUS, OHIO, US; IVASHCHENKO, ALEXANDER VASILIEVICH ET AL: "A preparation of combinatorial library of 2-substituted piperidine derivatives" XP002496762 extrait de STN Database accession no. 2004:1037092 & WO 2004/103991 A1 (CHEMICAL DIVERSITY RESEARCH INSTITUTE, LTD., RUSSIA) 2 décembre 2004 (2004-12-02)

## Description

La présente invention se rapporte à des dérivés d'imidazo[1,2-*a*]pyridine-2-carboxamides, à leur préparation et à leur application en thérapeutique dans le traitement ou la prévention de maladies impliquant les récepteurs nucléaires Nurr-1, aussi appelés NR4A2, NOT, TINUR, RNR-1, et HZF3.

La présente invention a pour objet les composés de formule (I): 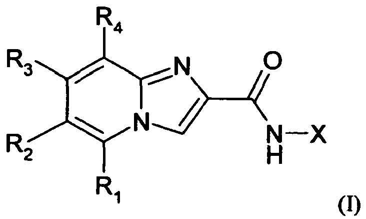 dans laquelle :
X représente un groupe hétérocyclique éventuellement substitué par un ou plusieurs groupes choisis indépendamment les uns des autres parmi les atomes ou groupes suivants : halogène, (C₁-C₆)alcoxy, (C₁-C₆)alkyle, NRaRb, cyano, oxido, COOR₈, les groupes alkyle et alcoxy pouvant éventuellement être substitués par un ou plusieurs atomes d'halogène ;
R₁ représente un atome d'hydrogène, un atome d'halogène, un groupe (C₁-C₆)alcoxy, un groupe (C₁-C₆)alkyle, amino ou NRaRb; les groupes alkyle et alcoxy pouvant éventuellement être substitués par un ou plusieurs atomes d'halogène, un groupe hydroxy, amino, ou un groupe (C₁-C₆₎alcoxy ;
R₂ représente un groupe hétérocyclique, ce groupe étant éventuellement substitué par un ou plusieurs groupes choisis indépendamment les uns des autres parmi les atomes ou groupes suivants : hydroxy, (C₁-C₆)alkyle, (C₁-C₆)alcoxy, halogène, cyano, NRaRb, -CO-R₅, - CO-NR₆R₇, -CO-O-R₈, -NR₉-CO-R₁₀, les groupes (C₁-C₆)alkyle et (C₁-C₆)alkoxy étant éventuellement substitués par un ou plusieurs atomes d'halogène ou groupes hydroxy, NRaRb, oxido ;
R₃ représente un atome d'hydrogène, un groupe (C₁-C₆)alkyle, un groupe (C₁-C₆)alcoxy ou un atome d'halogène ;
R₄ représente un atome d'hydrogène, un groupe (C₁-C₄)alkyle, un groupe (C₁-C₄)alcoxy ou un atome de fluor ;
R₅ représente un atome d'hydrogène, un groupe phényle ou un groupe (C₁-C₆)alkyle ;
R₆ et R₇, identiques ou différents, représentent un atome d'hydrogène ou un groupe (C₁-C₆)alkyle, ou forment avec l'atome d'azote qui les porte un cycle de 4 à 7 chaînons incluant éventuellement un autre hétéroatome choisi parmi N, O ou S ;
R₈ représente un groupe (C₁-C₆)alkyle;
R₉ et R₁₀, identiques ou différents, représentent un atome d'hydrogène ou un groupe (C₁-C₆)alkyle ;
Ra et Rb représentent, indépendamment l'un de l'autre, un atome d'hydrogène, un groupe (C₁-C₆)alkyle ou forment avec l'atome d'azote qui les porte un cycle de 4 à 7 chaînons, incluant éventuellement un autre hétéroatome choisi parmi N, O ou S ;
à l'état de base ou de sel d'addition à un acide.

On connaît de WO 2007/108750 des dérivés de composés pyridopyrimidine, inhibiteurs des protéines kinases PDE4.

EP 1 849 465 décrit des composés qui possèdent une fonction régulatrice sur les récepteurs de type GPR.

US 2005/0165049 décrit des composés qui présentent une activité pour les récepteurs de type VR1.

On connaît également de la demande WO 2006/099379 des composés benzazoles, inhibiteurs de l'activité des BACE.

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels peuvent être préparés avec des acides pharmaceutiquement acceptables, mais les sels d'autres acides utiles, par exemple, pour la purification ou l'isolement des composés de formule (I) font également partie de l'invention.

Les composés de formule (I) peuvent également exister sous forme d'hydrates ou de solvats, à savoir sous forme d'associations ou de combinaisons avec une ou plusieurs molécules d'eau ou avec un solvant. De tels hydrates et solvats font également partie de l'invention.

Dans le cadre de la présente invention, on entend par :
- un atome d'halogène : un fluor, un chlore, un brome ou un iode ;
- un groupe alkyle : un groupe aliphatique saturé linéaire, ramifié ou cyclique, éventuellement substitué par un groupe alkyle saturé alinéaire, ramifié ou cyclique. A titre d'exemples, on peut citer les groupes méthyle, éthyle, propyle, isopropyle, butyle, isobutyle, tertbutyle, cyclopropyle, cyclobutyl, cyclopentyle, cyclohexyle, méthylcyclopropyl etc ;
- un groupe alcoxy : un radical -O-alkyle où le groupe alkyle est tel que précédemment défini ;
- un groupe hétérocyclique : un groupe mono ou bicyclique comportant de 5 à 10 atomes dont de 1 à 4 hétéroatomes choisis parmi N, O et S, ce groupe cyclique est aromatique, insaturé ou partiellement insaturé ou oxydé et est relié par un atome de carbone. A titre d'exemples de groupes hétérocycliques, on peut citer : pyrrole, furane, thiophene, pyrazole, imidazole, triazole, tetrazole, oxazole, isoxazole, oxadiazole, thiazole, isothiazole, thiadiazole, pyridine, pyrimidine, pyrazine, pyridazine, triazine, pyrrolopyrrole, pyrroloimidazole, pyrrolopyrazole, pyrrolotriazole, imidazoimidazole, imidazopyrazole, imidazotriazole, indole, isoindole, benzimidazole, indazole, indolizine, benzofuran, isobenzofuran, benzothiophene, benzo[c]thiophen, pyrrolopyridine" imidazopyridine, pyrazolopyridine, triazolopyridine, tetrazolopyridine, pyrrolopyrimidine, imidazopyrimidine, pyrazolopyrimidine, triazolopyrimidine, tetrazolopyrimidine, pyrrolopyrazine, imidazopyrazine, pyrazolopyrazine, triazolopyrazine, tetrazolopyrazine, pyrrolopyridazine, imidazopyridazine, pyrazolopyridazine, triazolopyridazine, tetrazolopyridazine, pyrrolotriazine, imidazotriazine, pyrazolotriazine, triazolotriazine, tetrazolotriazine, furopyridine, furopyrimidine, furopyrazine, furopyridazine, furotriazine, oxazolopyridine, oxazolopyrimidine, oxazolopyrazine, oxazolopyridazine, oxazolotriazine, isoxazolopyridine, isoxazolopyrimidine, isoxazolopyrazine, isoxazolopyridazine, isoxazolotriazine, oxadiazolopyridine, oxadiazolopyrimidine, oxadiazolopyrazine, oxadiazolopyridazine, oxadiazolotriazine, benzoxazole, benzisoxazole, benzoxadiazole, thienopyridine, thienopyrimidine, thienopyrazine, thienopyridazine, thienotriazine, thiazolopyridine, thiazolopyrimidine, thiazolopyrazine, thiazolopyridazine, thiazolotriazine, isothiazolopyridine, isothiazolopyrimidine, isothiazolopyrazine, isothiazolopyridazine, isothiazolotriazine, thiadiazolopyridine, thiadiazolopyrimidine, thiadiazolopyrazine, thiadiazolopyridazine, thiadiazolotriazine, benzothiazole, benzoisothiazole, benzothiadiazole, quinoline, isoquinoline, cinnoline, phthalazine, quinoxaline, quinazoline, naphthyridine, benzotriazine, pyridopyrimidine, pyridopyrazine, pyridopyridazine, pyridotriazine, pyrimidopyrimidine, pyrimidopyrazine, pyrimidopyridazine, pyrimidotriazine, pyrazinopyrazine, pyrazinopyridazine, pyrazinotriazine, pyridazinopyridazine, pyridazinotriazine.

Le groupe hétérocyclique mis en oeuvre dans les composés selon la présente invention est par exemple un groupe hétérocyclique monocycle.

Différents sous-ensembles de composés sont définis ci-après faisant également partie de l'invention.

Parmi les composés de formule (I) objets de l'invention, un premier groupe de composés est constitué des composés pour lesquels :
X représente un groupe hétérocyclique éventuellement substitué par un ou plusieurs groupes choisis indépendamment les uns des autres parmi les atomes d'halogène ; les autres substituants étant tels que définis précédemment.

Parmi les composés de formule (I) objets de l'invention, un deuxième groupe de composés est constitué des composés pour lesquels :
X représente un groupe pyridine, isoxazole, thiazole, thiadiazole, pyrazole, thiophène, ou oxazole, ces groupes étant éventuellement substitués par un atome d'halogène;
les autres substituants étant tels que définis précédemment.

Parmi les composés de formule (I) objets de l'invention, un troisième groupe de composés est constitué des composés pour lesquels :
R₁, R₃, R₄ représentent un atome d'hydrogène ;
les autres substituants étant tels que définis précédemment.

Parmi les composés de formule (I) objets de l'invention, un quatrième groupe de composés est constitué des composés pour lesquels :
R₂ représente un groupe hétérocyclique, ce groupe étant éventuellement substitué par un ou plusieurs groupes choisis indépendamment les uns des autres parmi les atomes ou groupes suivants : (C₁-C₆)alkyle, NRaRb, le ou les groupes (C₁-C₆)alkyle étant éventuellement substitués par un ou plusieurs atomes d'halogène ou groupes hydroxy,
Ra et Rb représentent, indépendamment l'un de l'autre, un atome d'hydrogène, un groupe (C₁-C₆)alkyle ;
les autres substituants étant tels que définis précédemment.

Parmi les composés de formule (I) objets de l'invention, un cinquième groupe de composés est constitué des composés pour lesquels :
R₂ représente un groupe pyridine, thiophène, imidazole, pyrrole, furane, oxazole, triazole, ou pyrazole, ces groupes étant éventuellement substitués par un groupe NH₂ ou hydroxyméthyle ;
les autres substituants étant tels que définis précédemment.

Parmi les composés de formule (I) objets de l'invention, un sixième groupe de composés est constitué des composés pour lesquels :
X représente un groupe pyridine, isoxazole, thiazole, thiadiazole, pyrazole, thiophène, ou oxazole, ces groupes étant éventuellement substitués par un atome de fluor,
R₂ représente un groupe pyridine, thiophène, imidazole, pyrrole, furane, oxazole, triazole, ou pyrazole, ces groupes étant éventuellement substitués par un groupe NH₂ ou hydroxyméthyle,
R₁, R₃, R₄ représentent un atome d'hydrogène.

Parmi les composés de formule (I) objets de l'invention, un septième groupe de composés est constitué des composés pour lesquels :
X représente un groupe pyridine, isoxazole, thiazole, thiadiazole, pyrazole, thiophène, ces groupes étant éventuellement substitués par un atome de fluor ;
R₂ représente un groupe pyridine, thiophène, imidazole, pyrrole, furanne, oxazole, triazole, ces groupes étant éventuellement substitués par un groupe NH₂ ou hydroxyméthyle ;
R₁, R₃ et R₄ représentent un atome d'hydrogène, à l'état de base ou de sel d'addition à un acide.

Parmi les composés de formule (I) objets de l'invention, on peut notamment citer les composés suivants :
- 6-(6-Aminopyridin-2-yl)-*N*-(pyridin-2-yl)imidazo[1,2-*a*]pyridine-2-carboxamide et son chlorhydrate (2:1)
- 6-(1*H*-Imidazol-4-yl)-*N*-(pyridin-2-yl)imidazo[1,2-*a*]pyridine-2-carboxamide
- *N*,6-Di(pyridin-2-yl)imidazo[1,2-*a*]pyridine-2-carboxamide
- *N*-(Pyridin-2-yl)-6-(thiophèn-3-yl)imidazo[1,2-*a*]pyridine-2-carboxamide
- *N*-(Isoxazol-4-yl)-6-(pyridin-2-yl)imidazo[1,2-*a*]pyridine-2-carboxamide
- *N*-(6-Fluoropyridin-2-yl)-6-(pyridin-2-yl)imidazo[1,2-*a*]pyridine-2-carboxamide
- 6-(Pyridin-2-yl-*N*-(thiazol-2-yl)imidazo[1,2-*a*]pyridine-2-carboxamide
- *N*-(Isoxazol-3-yl)6-(pyridin-2-yl)imidazo[1,2-*a*]pyridine-2-carboxamide
- 6-(Pyridin-2-yl-*N*-(1,3,4-thiadiazol-2-yl)imidazo[1,2-*a*]pyridine-2-carboxamide
- *N*-(1*H*-Pyrazol-3-yl)-6-(pyridin-2-yl)imidazo[1,2-*a*]pyridine-2-carboxamide
- 6-(Pyridin-2-yl-*N*-(thiophén-3-yl)imidazo[1,2-*a*]pyridine-2-carboxamide
- 6-(1*H*-Pyrrol-3-yl)-*N*-(thiazol-2-yl)imidazo[1,2-*a*]pyridine-2-carboxamide
- *N*-(Isoxazol-4-yl)-6-(1*H*-pyrazol-3-yl)imidazo[1,2-*a*]pyridine-2-carboxamide
- 6-(Furan-2-yl)-*N*-thiazol-2-yl)imidazo[1,2-*a*]pyridine-2-carboxamide
- 6-(Furan-2-yl)-*N*-(isoxazol-4-yl)imidazo[1,2-*a*]pyridine-2-carboxamide
- 6-(Oxazol-2-yl)-*N*-(thiazol-2-yl)imidazo[1,2-*a*]pyridine-2-carboxamide
- *N*-(Isoxazol-3-yl)-6-(oxazol-2-yl)imidazo[1,2-*a*]pyridine-2-carboxamide
- *N*-(Isoxazol-4-yl)-6-(oxazol-2-yl)imidazo[1,2-*a*]pyridine-2-carboxamide
- 6-(Furan-3-yl)-*N*-(thiazol-2-yl)imidazo[1,2-*a*]pyridine-2-carboxamide
- 6-(Furan-3-yl)-*N*-(isoxazol-4-yl)imidazo[1,2-*a*]pyridine-2-carboxamide
- 6-[5-(Hydroxyméthyl)furan-2-yl)-*N*-(thiazol-2-yl)imidazo[1,2-*a*]pyridine-2-carboxamide
- 6-[5-(Hydroxyméthyl)furan-2-yl]-*N*-(isoxazol-3-yl)imidazo[1,2-*a*]pyridine-2-carboxamide
- 6-[5-(Hydroxyméthyl)furan-2-yl]-*N*-(isoxazol-4-yl)imidazo[1,2-*a*]pyridine-2-carboxamide
- 6-(1*H*-Imidazol-4-yl)-*N*-(isoxazol-3-yl)imidazo[1,2-*a*]pyridine-2-carboxamide
- 6-(6-Aminopyridin-2-yl)-*N*-(thiazol-2-yl)imidazo[1,2-*a*]pyridine-2-carboxamide
- 6-(1*H*-Pyrrol-3-yl)-*N*-(thiophén-3-yl)imidazo[1,2-*a*]pyridine-2-carboxamide
- 6-(Furan-2-yl)-*N*-(pyridin-2-yl)imidazo[1,2-*a*]pyridine-2-carboxamide
- *N*-(6-Fluoropyridin-2-yl)-6-(furan-2-yl)imidazo[1,2-*a*]pyridine-2-carboxamide
- 6-(Furan-2-yl)-*N*-(thiophén-3-yl)imidazo[1,2-*a*]pyridine-2-carboxamide
- 6-(Furan-2-yl)-*N*-(isoxazol-3-yl)imidazo[1,2-*a*]pyridine-2-carboxamide
- 6-(Furan-2-yl)-*N*-(1,3,4-thiadiazol-2-yl)imidazo[1,2-*a*]pyridine-2-carboxamide
- 6-(Oxazol-2-yl)-*N*-(pyridin-2-yl)imidazo[1,2-*a*]pyridine-2-carboxamide
- *N*-(6-Fluoropyridin-2-yl)-6-(oxazol-2-yl)imidazo[1,2-*a*]pyridine-2-carboxamide
- 6-(Oxazol-2-yl)-*N*-(thiophén-3-yl)imidazo[1,2-*a*]pyridine-2-carboxamide
- 6-(Furan-3-yl)-*N*-(pyridin-2-yl)imidazo[1,2-*a*]pyridine-2-carboxamide
- *N*-(6-Fluoropyridin-2-yl)-6-(furan-3-yl)imidazo[1,2-*a*]pyridine-2-carboxamide
- 6-(Furan-3-yl)-*N*-(thiophén-3-yl)imidazo[1,2-*a*]pyridine-2-carboxamide
- 6-(Furan-3-yl)-*N*-(isoxazol-3-yl)imidazo[1,2-*a*]pyridine-2-carboxamide
- 6-(Furan-3-yl)-*N*-(1,3,4-thiadiazol-2-yl)imidazo[1,2-*a*]pyridine-2-carboxamide
- 6-[5-(Hydroxyméthyl)furan-2-yl]-*N*-(pyridin-2-yl)imidazo[1,2-*a*]pyridine-2-carboxamide
- 6-[5-(Hydroxyméthyl)furan-2-yl]-*N*-(thiophén-3-yl)imidazo[1,2-*a*]pyridine-2-carboxamide
- 6-(Furan-2-yl)-*N*-(1*H*-pyrazol-3-yl)imidazo[1,2-*a*]pyridine-2-carboxamide
- *N*-(Thiophén-3-yl)-6-(1*H*-1,2,4-triazol-3-yl)imidazo[1,2-*a*]pyridine-2-carboxamide
- 6-(6-Aminopyridin-2-yl)-*N*-(6-fluoropyridin-2-yl)imidazo[1,2-*a*]pyridine-2-carboxamide et son chlorhydrate (2:1)
- 6-(6-Aminopyridin-2-yl)-*N*-(thiophén-3-yl)imidazo[1,2-*a*]pyridine-2-carboxamide et son chlorhydrate (2:1)
- 6-(6-Aminopyridin-2-yl)-*N*-(isoxazol-3-yl)imidazo[1,2-*a*]pyridine-2-carboxamide et son chlorhydrate (2:1)
- 6-(6-Aminopyridin-2-yl)-*N*-(1,3,4-thiadiazol-2-yl)imidazo[1,2-*a*]pyridine-2-carboxamide et son chlorhydrate (2:1)
- 6-(6-Aminopyridin-2-yl)-*N*-(isoxazol-4-yl)imidazo[1,2-*a*]pyridine-2-carboxamide et son chlorhydrate (2:1)
- *N*-(Pyridin-2-yl)-6-(1*H*-pyrrol-3-yl)imidazo[1,2-*a*]pyridine-2-carboxamide et son trifluoroacétate (1:1)
- *N*-(Isoxazol-3-yl)-6-(1*H*-pyrrol-3-yl)imidazo[1,2-*a*]pyridine-2-carboxamide
- 6-(1*H*-Pyrrol-3-yl)-*N*-(1,3,4-thiadiazol-2-yl)imidazo[1,2-*a*]pyridine-2-carboxamide
- *N*-(Oxazol-2-yl)-6-(1*H*-pyrrol-3-yl)imidazo[1,2-*a*]pyridine-2-carboxamide
- *N*-(Isoxazol-4-yl)-6-(1*H*-pyrrol-3-yl)imidazo[1,2-*a*]pyridine-2-carboxamide
- 6-(1*H*-Pyrazol-3-yl)-*N*-(pyridin-2-yl)imidazo[1,2-*a*]pyridine-2-carboxamide
- 6-(1*H*-Pyrazol-3-yl)-*N*-(thiophén-3-yl)imidazo[1,2-*a*]pyridine-2-carboxamide et son trifluoroacétate (1:1)
- 6-(1*H*-Pyrazol-3-yl)-*N*-(thiazol-2-yl)imidazo[1,2-*a*]pyridine-2-carboxamide et son trifluoroacétate (1:1)
- *N*-(Isoxazol-3-yl)-6-(1*H*-pyrazol-3-yl)imidazo[1,2-*a*]pyridine-2-carboxamide
- 6-(1*H*-Pyrazol-3-yl)-*N*-(1,3,4-thiadiazol-2-yl)imidazo[1,2-*a*]pyridine-2-carboxamide et son trifluoroacétate (1:1)
- *N*,6-Di(1*H*-pyrazol-3-yl)imidazo[1,2-*a*]pyridine-2-carboxamide
- 6-(Furan-2-yl)-*N*-(1,3-oxazol-2-yl)imidazo[1,2-*a*]pyridine-2-carboxamide et son trifluoroacétate (1:1)
- 6-(Oxazol-2-yl)-*N*-(1,3,4-thiadiazol-2-yl)imidazo[1,2-*a*]pyridine-2-carboxamide
- 6-(Oxazol-2-yl)-*N*-(1*H*-pyrazol-3-yl)imidazo[1,2-*a*]pyridine-2-carboxamide
- 6-(Furan-3-yl)-*N*-(1,3-oxazol-2-yl)imidazo[1,2-*a*]pyridine-2-carboxamide et son trifluoroacétate (1:1)
- 6-[5-(Hydroxyméthyl)furan-2-yl]-*N*-(1*H*-pyrazol-3-yl)imidazo[1,2-*a*]pyridine-2-carboxamide
- 6-(1*H*-Imidazol-4-yl)-*N*-(thiophén-3-yl)imidazo[1,2-*a*]pyridine-2-carboxamide
- 6-(1*H*-Imidazol-4-yl)-*N*-(1,3-thiazol-2-yl)imidazo[1,2-*a*]pyridine-2-carboxamide
- *N*-(Pyridin-2-yl)-6-(1*H*-1,2,3-triazol-4-yl)imidazo[1,2-*a*]pyridine-2-carboxamide
- *N*-(Thiophén-3-yl)-6-(1*H*-1,2,3-triazol-4-yl)imidazo[1,2-*a*]pyridine-2-carboxamide ; et leurs sels d'addition à un acide.

Conformément à l' invention, on peut préparer les composés de formule générale (I) selon le procédé décrit dans le schéma 1.

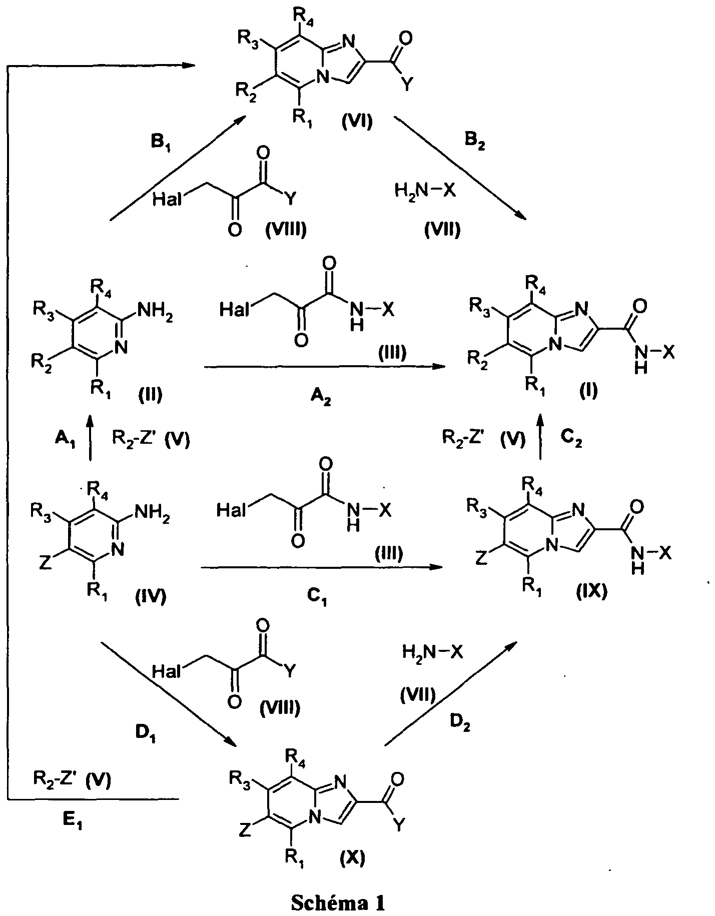

La première voie de synthèse (transformation A₂) consiste à préparer, selon les méthodes connues de l'homme du métier, une 2-amino-pyridine de formule (II), dans laquelle R₁, R₂, R₃ et R₄ sont définis comme précédemment, puis à former le cycle imidazo[1,2-*a*]pyridine par condensation d'un dérivé halogéné de 2-oxo-propionamide (III) dans lequel Hal représente un atome de chlore, de brome ou d'iode et X est défini comme précédemment, par analogie avec les méthodes décrites par J-J. Bourguignon et coll. dans Aust. J. Chem., 50, 719 (1997) et par J.G. Lombardino dans J. Org. Chem., 30, 2403 (1965) par exemple.

Les dérivés halogénés de 2-oxo-*N*-hétéroaryl-propionamide (III) peuvent être obtenus par exemple selon la méthode décrite par R. Kluger et coll. dans J. Am. Chem. Soc., 106, 4017 (1984).

Les 2-amino-pyridines de formule (II) dans laquelle R₁, R₂, R₃ et R₄ sont définis comme précédemment peuvent être préparées par exemple par la transformation A₁, c'est à dire :
- par réaction de couplage d'un dérivé de 2-aminopyridine de formule (IV) dans laquelle R₁, R₃ et R₄ sont définis comme précédemment et Z représente un groupe boryle, stannyle ou silyle et un dérivé R₂-Z' (V), dans lequel R₂ est défini comme précédemment et Z' représente un atome d'halogène tel que brome ou iode ou un groupe sulfonyloxy,
- par réaction de couplage d'un dérivé de 2-aminopyridine de formule (IV) dans laquelle R₁, R₃ et R₄ sont définis comme précédemment et Z représente un atome d'halogène tel que brome ou iode avec un dérivé R₂-Z' (V) dans lequel R₂ est défini comme précédemment et Z' représente un groupe réactif tel qu'un groupe boryle, stannyle ou silyle ou un atome d'hydrogène, ou par toute autre méthode connue de l'homme du métier.

La seconde voie de synthèse (transformation B₂) consiste à coupler un acide imidazopyridine-2-carboxylique ou l'un de ses dérivés de formule (VI), dans laquelle R_{1,} R₂, R₃ et R₄ sont définis comme précédemment, Y représente un hydroxy, halogène ou (C₁-C₆)alcoxy avec une hétéroarylamine X-NH₂ de formule (VII), dans laquelle X est défini comme précédemment, selon des méthodes connues de l'homme du métier. Ainsi, l'acide peut être préalablement converti en l'un de ses dérivés réactifs tel que halogènure d'acide, anhydride, anhydride mixte ou ester activé, puis mis en réaction avec l'amine (VII) en présence d'une base telle que la diisopropyléthylamine, la triéthylamine ou la pyridine, dans un solvant inerte tel que le THF, le DMF ou le dichlorométhane. Le couplage peut également être réalisé en présence d'un agent de couplage tel que CDI, EDCI, HATU ou HBTU dans les mêmes conditions sans isoler d'intermédiaire réactif. Alternativement, on peut faire réagir l'amine (VII) avec un ester de l'acide de formule (VI) en présence d'un catalyseur tel que le triméthylaluminium, selon la méthode de Weinreb, S. et coll (Tet. Lett. (1977), 18, 4171) ou le terbutylate de zirconium.

Les dérivés des acides imidazopyridine-2-carboxyliques de formule (VI), dans laquelle R₁, R₂, R₃ et R₄ sont définis comme précédemment et Y représente un groupe (C₁-C₆)alcoxy, hydroxy ou un atome d'halogène sont préparés par condensation d'une 2-aminopyridine de formule (II), dans laquelle R₁, R₂, R₃ et R₄ sont définis comme précédemment sur un ester d'acide 3-halogèno 2-oxo-propionique de formule (VIII), dans laquelle Hal représente un halogène et Y représente un groupe (C₁-C₆)alcoxy, dans les conditions similaires à celles utilisées pour la condensation d'un dérivé de formule (II) sur un dérivé de formule (III), suivie le cas échéant de la conversion de l'ester en acide puis en chlorure d'acide ou autre dérivé réactif (transformation B₁).

La troisième voie de synthèse (transformation C₂) consiste à coupler un dérivé de formule générale (IX), dans laquelle R₁, R₃, R₄ et X sont définis comme précédemment et Z représente un atome d'halogène tel que brome ou iode, un groupe sulfonyloxy ou un groupe réactif tel que boryle, stannyle ou silyle sur un dérivé de formule R₂-Z' (V) dans laquelle R₂ est défini comme précédemment et
- Z' représente un groupe réactif tel qu'un groupe boryle, stannyle ou silyle ou un atome d'hydrogène lorsque Z représente un atome d'halogène ou un groupe sulfonyloxy, ou
- Z' représente un atome d'halogène tel que brome ou iode lorsque Z représente un groupe réactif tel qu'un groupe boryle, stannyle ou silyle ou un atome d'hydrogène.

Les dérivés de formule générale (IX), dans laquelle R₁, R₃, R₄, X et Z sont définis comme précédemment, peuvent être préparés :
- par condensation d'une 2-amino-pyridine de formule (IV), dans laquelle R₁, R₃, R₄ et Z sont définis comme précédemment, sur un dérivé de 2-oxo-*N*-hétéroaryl-propionamide (III), dans lequel Hal représente un atome de chlore, de brome ou d'iode et X est défini comme précédemment (transformation C₁), selon des méthodes citées pour la conversion d'un composé de formule (II) en composé de formule (I) ou
- par amidification d'un acide imidazopyridine-2-carboxylique ou l'un de ses dérivés de formule (X) dans laquelle R₁, R₂, R₃ et R₄ sont définis comme précédemment, Y représente un hydroxy, un atome d'halogène ou un groupe (C₁-C₆)alcoxy avec une hétéroarylamine X-NH₂ de formule (VII), dans laquelle X est défini comme précédemment (transformation D₂) selon des méthodes citées pour la conversion d'un composé de formule (VI) en composé de formule (I).

Les acides imidazopyridine-2-carboxyliques ou leurs dérivés de formule (X), dans laquelle R₁, R₃ et R₄ sont définis comme précédemment, Y est (C₁-C₆)alcoxy, OH ou halogène et Z représente un groupe boryle, stannyle ou silyle ou un atome d'halogène peuvent être préparés (transformation D₁) par condensation d'une 2-aminopyridine de formule (IV), dans laquelle R₁, R₃ et R₄ sont définis comme précédemment et Z représente un groupe boryle, stannyle ou silyle ou un atome d'halogène avec un ester d'acide 3-halogèno 2-oxo-propionique de formule (VIII), dans laquelle Hal représente un halogène et Y représente un groupe (C₁-C₆)alcoxy, dans les conditions similaires à celles citées précédemment pour la condensation des 2-aminopyridines de formule (II) sur un dérivé de formule (VIII), pour obtenir les acides imidazopyridine-2-carboxyliques ou leurs dérivés de formule (VI) selon la transformation B₁, suivie le cas échéant de la conversion de l'ester en acide puis en chlorure d'acide ou autre dérivé réactif.

Les acides imidazopyridine-2-carboxyliques ou leurs dérivés de formule (VI), dans laquelle R₁, R₂, R₃ et R₄ sont définis comme précédemment, Y est (C₁-C₆)alcoxy, hydroxy ou halogène peuvent également être préparés (transformation E₁) par couplage d'un dérivé de formule générale (X), dans laquelle R₁, R₃, et R₄ sont définis comme précédemment, Y représente un groupe (C₁-C₆)alcoxy et Z représente un atome d'halogène tel que brome ou iode, un groupe sulfonyloxy ou un groupe réactif tel que boryle, stannyle ou silyle, sur un dérivé de formule R₂-Z' (V) dans laquelle R₂ est défini comme précédemment et
- Z' représente un groupe réactif tel qu'un groupe boryle, stannyle ou silyle ou un atome d'hydrogène lorsque Z représente un atome d'halogène ou un groupe sulfonyloxy, ou
- Z' représente un atome d'halogène tel que brome ou iode lorsque Z représente un groupe réactif tel qu'un groupe boryle, stannyle ou silyle ou un atome d'hydrogène,
suivi le cas échéant de la conversion de l'ester en acide puis en chlorure d'acide ou autre dérivé réactif (transformation E₁).

Les couplages des dérivés de formule (IV), (IX) ou (X) avec les produits de formule (V) peuvent être effectués par toute méthode connue de l'homme de métier, en particulier en opérant en présence de catalyseurs à base de cuivre ou de palladium, de ligands tels que des phosphines, selon ou par analogie avec les méthodes décrites par exemple dans les références suivantes et références citées :
- pour les réactions de type Suzuki : N. Miyaura, A. Suzuki, Chem. Rev., 95, 2457, (1995),
- pour les réactions de type Stille : V. Farina et coll., Org. React., 50, 1 (1997),
- pour les réactions de type Hiyama : T. Hiyama et coll., Top. Curr. Chem., 2002, 219, 61 (2002),
- pour les réactions de type Negishi : E. Negishi et coll., Chem. Rev., 103, 1979 (2003),
- pour les réactions de type Bellina : M. Miura et coll., Chem. Lett., 200 (2007).

II est également possible pour effectuer le couplage de former intermédiairement, mais sans les isoler, des dérivés organométalliques tels que des dérivés zinciques.

Conformément à l'invention, on peut également préparer les composés de formule générale (I), (VI) et (II) selon les procédés décrits dans le schéma 2.

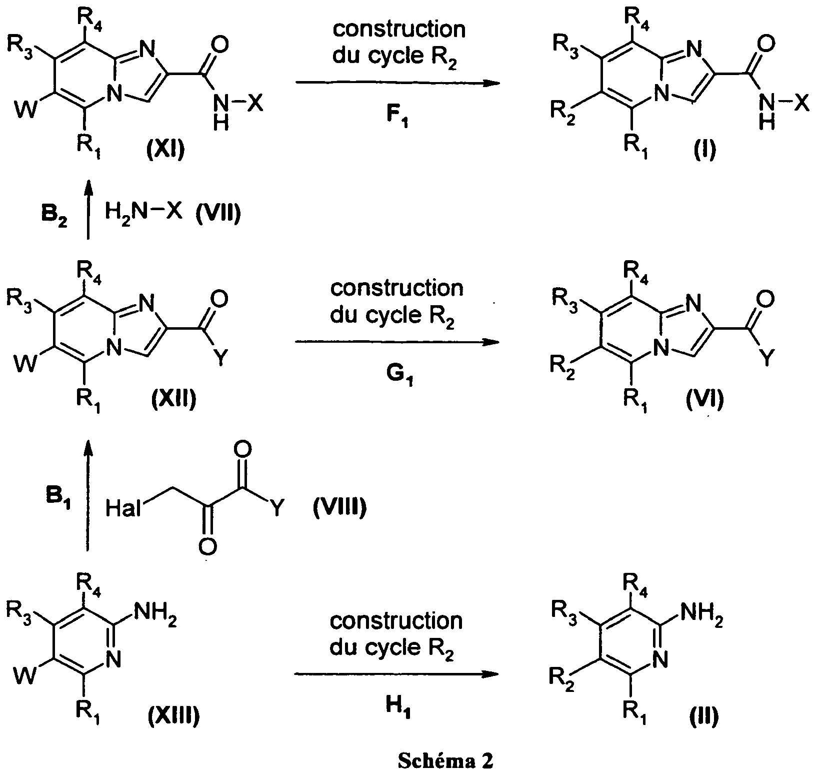

Cette voie de synthèse consiste en la conversion d'un composé de formules générales (XI), (XII) ou (XIII), dans lesquelles R₁, R₃, R₄, X et Y sont définis comme précédemment et W représente un groupement précurseur permettant la construction de l'hétérocycle de formule R_{2,} selon les méthodes connues de l'homme du métier.

A titre d'exemple W peut représenter :
- un groupement 2-halogéno-acyle tel que bromoacétyle, ou 1-halo-2-oxo-alkyle tel que 1-bromo-2-oxo-éthyle qui peut être converti, par exemple, en groupe thiazolyle, imidazolyle, oxazolyle par traitement par des dérivés de thiourée, de thioamide, de guanidine, d'urée ou d'amide,
- un groupement alkynyl, tel qu'éthynyl qui peut être converti en groupe 1,2,3-triazol-4-yl,
- un groupement acyle tel que formyl qui peut être converti, par exemple, en groupe 1,3-dioxolanyl-2 ou oxazolyl
- un groupement cyano qui peut être converti, par exemple, en groupe dihydroimidazolyl(2) ou 1,3,4-triazol-2-yl.

Les composés de formule générale (XI) peuvent être obtenus à partir des composés de formule (XII) dans les conditions décrites pour la préparation des composés (I) à partir des dérivés d'acide imidazopyridine-2-carboxyliques de formule (VI) par les transformations B₂.

Les dérivés d'acide imidazopyridine-2-carboxyliques de formule générale (XII) peuvent être obtenus à partir des amino-pyridines de formule (XIII), dans les conditions décrites pour la conversion des amino-pyridines de formule (II) en composés de formule générale (I) par la transformation A₂.

Les produits de formule (I) et leurs précurseurs de formule (II), (IV), (VI), (IX) ou (X), peuvent être soumis, si désiré et si nécessaire, pour obtenir des produits de formule (I) ou être tranformés en d'autres produits de formule (I) à l'une ou plusieurs des réactions de transformations suivantes, dans un ordre quelconque :
a) une réaction d'estérification ou d'amidification de fonction acide,
b) une réaction d'amidification de fonction amine,
c) une réaction d'hydrolyse de fonction ester en fonction acide,
d) une réaction de transformation de fonction hydroxyle en fonction alcoxy,
e) une réaction d'oxydation de fonction alcool en fonction aldéhyde ou cétone,
f) une réaction de réduction des fonctions aldéhyde ou cétone en fonction alcool, par réduction ou action d'un organométallique tel qu'un organomagnésien,
g) une réaction de transformation de radical nitrile en fonction aldéhyde,
h) une réaction de transformation de radical nitrile en fonction cétone,
i) une réaction d'oxydation de groupe alcènyle en fonction aldéhyde ou cétone,
j) une réaction de couplage catalytique d'un dérivé organométallique tel qu'un dérivé du bore, de l'étain ou du silicium avec un dérivé halogéné pour introduire un substituant alkyle, alcènyle, alcynyles ou aryle,
k) une réaction de conversion d'un groupe amino primaire ou secondaire en un groupe amino secondaire ou tertiaire par amination réductrice ou alkylation,
l) une réaction de protection des fonctions réactives,
m) une réaction d'élimination des groupements protecteurs que peuvent porter les fonctions réactives protégées,
n) une réaction de salification par un acide minéral ou organique ou par une base pour obtenir le sel correspondant,
o) une réaction de dédoublement des formes racémiques en énantiomères,
lesdits produits de formule (I) ainsi obtenus étant le cas échéant sous toutes les formes isomères possibles : racémiques, énantiomères et diastéréoisomères.

Dans le schéma 1, les composés de départ et les réactifs, quand leur mode de préparation n'est pas décrit, sont disponibles dans le commerce ou décrits dans la littérature, ou bien peuvent être préparés selon des méthodes qui y sont décrites ou qui sont connues de l'Homme du métier.

Les exemples suivants décrivent la préparation de certains composés conformes à l'invention. Les numéros des composés exemplifiés renvoient à ceux donnés dans le tableau ci-après, qui illustre les structures chimiques et les propriétés physiques de quelques composés selon l'invention.

### Exemple 1 : 6-(6-Aminopyridin-2-yl)-N-(pyridin-2-yl)imidazo[1,2-a]pyridine-2-carboxamide et son chlorhydrate (2:1)

Dans un tube à microondes on charge 276 mg de 6-triméthylstannyl-*N*-(pyridin-2-yl)imidazo[1,2-*a*]pyridine-2-carboxamide (intermédiaire 18), 476 mg de 6-brome-2-aminopyridine, 111 mg de tétrakis(triphénylphosphine)palladium et 4 mL de *N,N-*diméthylfornamide. Le mélange est chauffé 45 minutes dans l'appareil à microondes réglé sur 150°C, puis refroidi et versé dans 100 mL de dichlorométhane. L'insoluble est filtré puis repris jusqu'à dissolution dans un grand volume de mélange de méthanol, dichlorométhane et acétate d'éthyle. La solution est évaporée en présence de silice et le produit brut ainsi déposé sur silice est chromatographié sur une cartouche de silice en éluant par un mélange 90/10 de dichlorométhane et de méthanol ammoniacal. Les fractions contenant le produit attendu sont réunies et concentrées à sec sous pression réduite. Le résidu est trituré avec du pentane, puis filtré pour donner 108 mg de 6-(6-aminopyridin-2-yl)-*N*-(pyridin-2-yl)imidazo[1,2-*a*]pyridine-2-carboxamide sous la forme d'un solide écru.

Ce produit est solubilisé dans du dioxanne bouillant et on ajoute à la solution chaude 150 µL d'une solution 4N d'acide chlorhydrique dans le dioxanne. Le précipité est essoré et rincé par du pentane puis séché pour donner 130 mg de chlorhydrate (2:1) de 6-(6-aminopyridin-2-yl)-*N*-(pyridin-2-yl)imidazo[1,2-*a*]pyridine-2-carboxamide sous la forme d'un solide écru.

### Exemple 2 : 6-(1H-imidazol-4-yl)-N-(pyridin-2-yl)imidazo[1,2-a]pyridine-2-carboxamide

### 2.1 : 6-(1-Benzyl-imidazol-4-yl)-N-(pyridin-2-yl)imidazo[1,2-a]pyridine-2-carboxamide

A une solution de 405 mg de (1-benzyl-imidazol-4-yl)tributylstannane dans 25 mL de toluène, on ajoute 300 mg de 6-iodo-*N*-(pyridin-2-yl)imidazo[1,2-*a*]pyrridine-2-carboxamide (intermédiaire 17) et 48 mg de tétrakis(triphénylphosphine)palladium(0). Le mélangé réactionnel est chauffé 3,5 heures au reflux puis concentré sous pression réduite. Le résidu est repris dans du dichlorométhane et lavé deux fois par une solution aqueuse saturée de fluorure de potassium. La phase organique est séchée et évaporée à sec sous pression réduite et le résidu concrété avec du dichlorométhane pour donner 220 mg de 6-(1-benzyl-imidazol-4-yl)-*N*-(pyridin-2-yl)imidazo[1,2-*a*]pyridine-2-carboxamide sous la forme d'un solide blanc.

Spectre RMN ¹H (DMSO-d6, δ en ppm) : 5,26 (s, 2H), 7,18 (m, 1H), 7,30 - 7,43 (m, 5H), 7,68 (d, J=9,3 Hz, 1H), 7,74 - 7,80 (m, 2H), 7,88 (m, 1H), 7,91 (d, J=1,0 Hz, 1H), 8,24 (d, J=8,1 Hz, 1H), 8,38 (ddd, J=4,9, 1,9, 1,0 Hz, 1H), 8,63 (s, 1H), 8,99 (s large, 1H), 9,78 (s, 1H). Spectre de masse (IE) : m/z 395 [M]⁺.

### 2.2 : 6-(1H-imidazol-4-yl)-N-(pyridin-2-yl)imidazo[1,2-a]pyridine-2-carboxamide

Un mélange de 10 mL d'éthanol, 5 mL de dichlorométhane, 220 mg de 6-(1-benzyl-imidazol-4-yl)-*N*-(pyridin-2-yl)imidazo[1,2-*a*]pyridine-2-carboxamide, 50 mg de palladium à 10 % sur charbon et 6,8 mL de cyclohexène est chauffé 10 minutes dans l'appareil à microondes réglé sur 150°C et de nouveau 2 fois 10 minutes à 150°C après ajout de 20 mg de palladium sur charbon et 2 mL de cyclohexène. Le mélange réactionnel est filtré, l'insoluble est lavé à l'éthanol et les filtrats réunis sont concentrés à sec en présence de silice. Le produit brut ainsi déposé sur silice est chromatographié sur une cartouche de silice en éluant par des mélanges de dichlorométhane et de méthanol (95/5 puis 90/10). Les fractions contenant le produit attendu sont réunies et concentrées à sec sous pression réduite pour donner 13 mg de 6-(1*H*-imidazol-4-yl)-*N*-(pyridin-2-yl)imidazo[1,2-*a*]pyridine-2-carboxamide sous la forme d'un solide blanc cassé.

### Exemple 3 : N,6-di(pyridin-2-yl)imidazo[1,2-a]pyridine-2-carboxamide

A un mélange de 280 mg d'acide 6-(pyridin-2-yl)imidazo[1,2-*a*]pyridine-2-carboxylique (intermédiaire 2), 239 mg de 1-hydroxy-7-azabenzotriazole (HOAt), 667 mg d'hexafluorophosphate de 1-oxyde de 1-[bis(diméthylamino)méthylène]-1*H*-1,2,3-triazolo[4,5-*b*]pyridinium (HATU) et 600 µl de diisopropyléthylamine dans 2 mL de *N*,*N* diméthylformamide, on ajoute 165 mg de 2-pyridylamine. Le mélange réactionnel est agité à 20°C pendant 16 heures puis filtré. L'insoluble est lavé à l'eau puis repris dans du dichlorométhane. La phase organique est lavée par une solution saturée d'hydrogénocarbonate de sodium, séchée sur sulfate de sodium et concentrée sous pression réduite. Le résidu est trituré avec du méthanol pour donner 70 mg de *N*,6-di(pyridin-2-yl)imidazo[1,2-*a*]pyridine-2-carboxamide sous la forme d'un solide blanc.

### Exemple 4 : N-(pyridin-2-yl)-6-(thiophén-3-yl)imidazo[1,2-a]pyridine-2-carboxamide

Dans un tube à microondes, on charge 0,55 mL d'une solution aqueuse 2M de carbonate de potassium et 3 mL de diméthoxy-1,2-éthane, puis après dégazage à l'argon 200 mg de 6-iodo-*N*-(pyridin-2-yl)imidazo[1,2-*a*]pyridine-2-carboxamide (intermédiaire 17), 84 mg d'acide 3-thiophène-boronique et 39 mg de dichloro bis(triphenylphosphine)palladium(II). Le mélange réactionnel est chauffé 20 minutes dans l'appareil à microondes réglé sur 120°C, puis dilué dans un mélange d'acétate d'éthyle et d'eau et concentré. Le résidu est repris par un mélange d'éther éthylique et d'eau. Le solide est lavé successivement par de l'eau (2 fois), de l'éther (2 fois), par un mélange de 2 mL de méthanol et 5 mL d'éther, puis par de l'isopropanol (2 fois) et enfin par du méthanol. Le produit brut ainsi obtenu étant contaminé par environ 7% de 6-iodo-*N*-(pyridin-2-yl)imidazo[1,2-*a*]pyridine-2-carboxamide inchangé, il est recyclé dans des conditions similaires pour achever la conversion (mélange de 0,22 mL de carbonate de potassium 2M, 3 mL de diméthoxy-1,2-éthane, 28 mg d'acide 3-thiophène-boronique et 30 mg de dichloro bis(triphenylphosphine)palladium(II) chauffé 10 minutes sous microondes à 100°C). Le solide obtenu après évaporation du mélange réactionnel est lavé successivement par de l'eau (2 fois), du méthanol (2 fois), de l'éther et enfin par du pentane et séché pour donner 103 mg de *N*-(pyridin-2-yl)-6-(thiophèn-3-yl)imidazo[1,2-*a*]pyridine-2-carboxamide sous la forme d'un solide gris clair.

### Exemple 5 : N-(isoxazol-4-yl)-6-(pyridin-2-yl)imidazo[1,2-a]pyridine-2-carboxamide

A une suspension de 65 mg d'acide 6-(pyridin-2-yl)imidazo[1,2-*a*]pyridine-2-carboxylique (intermédiaire 2) et 104 mg de chlorhydrate de 1-(3-diméthylaminopropyl)-3-éthylcarbodiimide dans 2 mL de pyridine anhydre, placée sous argon, on ajoute 68 mg d'isoxazol-4-yl-amine. Le mélange réactionnel est agité pendant 16 heures à 50 °C puis concentré à sec sous pression réduite. Le résidu est repris par du dichlorométhane et lavé à l'eau. La phase organique est séchée sur sulfate de magnésium et concentrée à sec sous pression réduite pour donner 49 mg de *N*-(isoxazol-4-yl)-6-(pyridin-2-yl)imidazo[1,2-*a*]pyridine-2-carboxamide sous la forme d'un solide beige.

### Exemples 6 à 23 : Couplage des acides 6-(hétérocyclyl)imidazo[1,2-a]pyridine-2-carboxyliques avec les amines hétéroaromatiques

Les composés des exemples 6 à 23 sont obtenus par couplage des acides 6-(hétérocyclyl)imidazo[1,2-*a*]pyridine-2-carboxyliques (intermédiaires 1 à 14) avec les amines hétéroaromatiques appropriées selon le mode opératoire de l'exemple 5. Si cela est nécessaire, le produit obtenu peut être repurifié par chromatographie sur colonne de silice.

### Exemple 24 : 6-(1H-imidazol-4-yl)-N-(isoxazol-3-yl)imidazo[1,2-a]pyridine-2-carboxamide

### 24.1 : 6-(1-Triphénylméthyl-1H-imidazol-4-yl)-N-(isoxazol-3-yl)imidazo[1,2-a]pyridine-2-carboxamide

Ce composé est obtenu par couplage de l'acide 6-(1-triphénylméthyl-1*H*-imidazol-4-yl)imidazo[1,2-*a*]pyridine-2-carboxylique avec l'isoxazol-3-ylamine selon le mode opératoire de l'exemple 5.

### 24.2 : 6-(1H-imidazol-4-yl)-N-(isoxazol-3-yl)imidazo[1,2-a]pyridine-2-carboxamide

A une solution de 137 mg de 6-(1-triphénylméthyl-1*H*-imidazol-4-yl)-*N*-(isoxazol-3-yl)imidazo[1,2-*a*]pyridine-2-carboxamide dans 3 mL d'éthanol, on ajoute 3 mL d'une solution aqueuse 4N d'acide chlorhydrique . Le mélange réactionnel est agité à 25°C pendant 16 heures puis traité par une solution aqueuse 2N de carbonate de sodium jusqu'à atteindre un pH de 8-9. Le résidu obtenu après évaporation sous pression réduite est chromatographié sur silice en éluant par des mélanges de dichlorométhane et de méthanol (de 96/4 à 90/10). Les fractions contenant le produit attendu sont réunies et concentrées à sec sous pression réduite pour donner 13 mg de 6-(1*H*-imidazol-4-yl)-*N*-(isoxazol-3-yl)imidazo[1,2-*a*]pyridine-2-carboxamide sous la forme d'un solide beige.

### Exemples 25 : 6-(6-Aminopyridin-2-yl)-N-(thiazol-2-yl)imidazo[1,2-a]pyridine-2-carboxamide

### 25.1 : 6-(6-{[(1,1-diméthyléthoxy)carbonyl]amino}pyridin-2-yl)-N-(thiazl-2-yl)imidazo[1,2-a]-pyridine-2-carboxamide

Ce composé est obtenu par couplage de l'acide 6-(6-{[(1,1-diméthyléthoxy)carbonyl]-amino}pyridin-2-yl)imidazo[1,2-*a*]pyridine-2-carboxylique avec la thiazol-2-ylamine selon le mode opératoire de l'exemple 5.

### 25.2 : 6-(6-Aminopyridin-2-yl)-N-(thiazol-2-yl)imidazo[1,2-a]pyridine-2-carboxamide

A une solution de 124 mg de 6-(6-{[(1,1-diméthyléthoxy)carbonyl]amino}pyridin-2-yl)-*N*-(thiazol-2-yl)imidazo[1,2-*a*]pyridine-2-carboxamide dans 0,5 mL de dioxanne refroidie à 0 °C, on ajoute 1,5 mL d'une solution aqueuse 4N d'acide chlorhydrique. Le mélange réactionnel est agité à 25 °C pendant 3 heures puis concentré à sec. Le solide est lavé par 5 mL d'éther éthylique, repris par 20 mL de dichlorométhane que l'on évapore sous pression réduite (3 fois) puis lavé à nouveau avec par 5 mL d'éther éthylique et séché pour donner 114 mg de 6-(6-aminopyridin-2-yl)-*N*-(thiazol-2-yl)imidazo[1,2-*a*]pyridine-2-carboxamide sous la forme d'un solide blanc.

Les intermédiaires décrits ci-dessous sont utiles à la préparation des composés de la présente invention.

### Intermédiaire 1 : Acide 6-(6-{[(1,1-diméthyléthoxy)carbonyl]amino}pyridin-2-yl)imidazo[1,2-a]pyridine-2-carboxylique

### 1.1 : 6-(6-Aminopyridin-2-yl)imidazo[1,2-a]pyridine-2-carboxylate d'éthyle

350 mg de 2-amino-6-bromopyridine, 750 mg d'acide 2-éthoxycarbonyl-imidazo[1,2-*a*]pyridine-6-boronique et 57 mg de [1,1'-bis(diphenylphosphino)ferrocène]dichloropalladium sont dégazés sous vide puis mis en suspension, sous argon, dans 20 mL de dioxanne dégazé. Après ajout de 2 mL de solution aqueuse 2N de carbonate de sodium, le mélange est dégazé sous vide puis placé sous argon et chauffé 5 heures à 90°C, puis refroidi, dilué et agité dans un mélange de 50 mL de solution saturée de bicarbonate de sodium et 50 mL de dichlorométhane. La phase organique est séchée sur sulfate de sodium, filtrée et concentrée à sec sous pression réduite. Le résidu est chromatographié sur silice en éluant par un mélange d'acétate d'éthyle et d'hexane. Les fractions contenant le produit attendu sont réunies et concentrées à sec sous pression réduite pour donner 446 mg de 6-(6-aminopyridin-2-yl)imidazo[1,2-*a*]pyridine-2-carboxylate d'éthyle.

Spectre RMN ¹H (DMSO-d6, δ en ppm) : 9,13 (dd, J = 1,0, 1,6 1H), 8,61 (d, J = 0,7, 1H), 7,94 (dd, J = 1,8, 9,6, 1H), 7,65 (d, J = 9,6, 1H), 7,50 (t, J = 8,1, 1H), 7,07 (d, J = 7,0, 1H), 6,48 (dd, J = 0,3, 8,1, 1H), 6,08 (s large, 2H), 4,33 (q, J = 7,1, 2H), 1,33 (t, J = 7,1, 3H).
Spectre de masse (APCI): m/z= 283 [M+H]⁺.

### 1.2 : 6-(6-{[(1,1-Diméthyléthoxy)carbonyl]amino} pyridin-2-yl)imidazo[1,2-a]pyridine-2-carboxylate d'éthyle et 6-(6-{bis[(1,1-diméthyléthoxy)carbonyl]amino}pyridin-2-yl)-imidazo[1,2-a]pyridine-2-carboxylate d'éthyle

A une suspension de 700 mg de 6-(6-aminopyridin-2-yl)imidazo[1,2-*a*]pyridine-2-carboxylate d'éthyle et 25 mg de 4-diméthylaminopyridine dans 5 mL d'acétonitrile, on ajoute 1,14 mL de ditertbutyledicarbonate. Le mélange est agité pendant 16 heures à 25 °C puis concentré. Le résidu est chromatographié sur silice en éluant par un gradient d'acétate d'éthyle et d'hexane (de 50/50 à 100/0) pour donner 370 mg de 6-(6-{bis[(1,1-diméthyléthoxy)carbonyl]amino}pyridin-2-yl)imidazo[1,2-*a*]pyridine-2-carboxylate d'éthyle Spectre RMN ¹H (DMSO-d6, δ en ppm) : 9,23 (s, 1H), 8,65 (s, 1H), 8,06,-7,98 (m, 2H), 7,95 (d, J = 7,7, 1H), 7,76 (d, J = 9,6, 1H), 7,43 (d, J = 7,8, 1H), 4,33 (q, J = 7,0, 2H), 1,43 (s, 18H), 1,34 (t, J = 7,1, 3H).

Spectre de masse (APCI): m/z= 483 [M+H]⁺.
et 163 mg de 6-(6-{[(1,1-diméthyléthoxy)carbonyl]amino}pyridin-2-yl)imidazo[1,2-*a*]pyridine-2-carboxylate d'éthyle

Spectre RMN ¹H (DMSO-d6, δ en ppm) : 9,28 (s, 1H), 8,50 (s, 1H), 8,04-8,00 (m, 2H), 7,95 (d, J = 7,8, 1H), 7,70 (d, J = 9,6, 1H), 7,38 (d, J = 7,9, 1H), 4,31 (q, J = 7,0, 2H), 1,39 (s, 9H), 1,33 (t, J = 7,1, 3H).
Spectre de masse (APCI): m/z= 383 [M+H]⁺.

### 1.3 : Acide 6-(6-{[(1,1-diméthyléthoxy)carbonyl]amino}pyridin-2-yl)imidazo[1,2-a]pyridine-2-carboxylique

0,9 mL d'une solution aqueuse 2 M de lithine sont ajoutés à une solution de 292 mg de 6-(6-{bis[(1,1-diméthyléthoxy)carbonyl]amino}pyridin-2-yl)imidazo[1,2-*a*]pyridine-2-carboxylate d'éthyle dans 4,73 mL de mélange 50:1 de tétrahydrofuranne et de méthanol. Le mélange réactionnel est agité pendant 7 heures à 25 °C, puis traité goutte à goutte à 0 °C par de l'acide chlorhydrique 2 N jusqu'à atteindre un pH de 3. Le précipité formé après 20 minutes est essoré et lavé par de l'eau (20 mL) et de l'éther diéthylique (20 ml) puis séché sous pression réduite pour donner 195 mg d'acide 6-(6-{[(1,1-diméthyléthoxy)carbonyl]amino}pyridin-2-yl)imidazo[1,2-*a*]pyridine-2-carboxylique sous la forme d'un solide beige.

Spectre RMN ¹H (DMSO-d6, δ en ppm) : 13,5-12,0 (br, 1H), 9,80 (s, 1H), 9,24 (s, 1H), 8,51 (s, 1H), 8,03 (dd, J = 1,5, 9,6 1H), 7,88 (app, t, J = 8,0, 7,8, 1H), 7,77 (d, J = 8,2, 1H), 7,73 (d, J = 9,6, 1H), 7,62 (d, J = 7,5, 1H), 1,50 (s, 9H)

### Intermédiaire 2 : Acide 6-(pyridin-2-yl)imidazo[1,2-a]pyridine-2-carboxylique

### 2.1 : 6-(Pyridin-2-yl)imidazo[1,2-a]pyridine-2-carboxylate d'éthyle

Un mélange de 3,18 g de carbonate de césium, 25 mL de dioxanne, 9,3 mL d'eau, 500 mg de 2-iodopyridine, 89 mg de [1,1'-bis(diphenylphosphino)ferrocène]dichloropalladium et 848 mg de bromhydrate (1:1) de 6-(4,4,5,5-tétraméthyl-1,3,2-dioxaborolan-2-yl)imidazo[1,2-*a*]pyridine-2-carboxylate d'éthyle est chauffé 2 heures à 110 °C, puis partiellement concentré et dilué avec du dichlorométhane et filtré. La phase organique est lavée à l'eau et séchée sur sulfate de magnésium, filtrée et concentrée à sec sous pression réduite. Le résidu est chromatographié sur une cartouche de silice en éluant par un mélange de dichlorométhane et de cyclohexane (80/20). Les fractions contenant le produit attendu sont réunies et concentrées à sec sous pression réduite pour donner 317 mg de 6-(pyridin-2-yl)imidazo[1,2-*a*]pyridine-2-carboxylate d'éthyle sous la forme d'une huile brune.

Spectre RMN ¹H (DMSO-d6, δ en ppm) : 1,34 (t, J=7,0 Hz, 3H), 4,33 (q, J=7,0 Hz, 2H), 7,42 (ddd, J=7,5, 5,5, 2,0 Hz, 1H), 7,73 (d, J=9,3 Hz, 1H), 7,85 - 8,02 (m, 2H), 8,07 (dd, J=9,3, 2,0 Hz, 1H), 8,64 (s, 1H), 8,70 (d large, J=5,5 Hz, 1H), 9,36 (s large, 1H).

Spectre de masse (LC-MS-DAD-ELSD) : m/z 268 [M+H]⁺.

### 2.2 : Acide 6-(pyridin-2-yl)imidazo[1,2-a]pyridine-2-carboxylique

317 mg de 6-(pyridin-2-yl)imidazo[1,2-*a*]pyridine-2-carboxylate d'éthyle sont saponifiés dans des conditions analogues à celles décrites pour la préparation de l'intermédiaire 1 (étape 1.3) pour donner 280 mg d'acide 6-(pyridin-2-yl)imidazo[1,2-*a*]pyridine-2-carboxylique sous la forme d'un solide rosé pâteux.

Spectre RMN ¹H (DMSO-d6, δ en ppm) : 7,47 (m, 1H), 7,83 (d, J=9,8 Hz, 1H), 7,99 (dt, J=8,5, 2,0 Hz, 1H), 8,06 (d, J=8,5 Hz, 1H), 8,31 (d large, J=9,8 Hz, 1H), 8,73 (m, 2 H), 9,52 (s large, 1H).

Spectre de masse (LC-MS-DAD-ELSD) : m/z 240 [M+H]⁺.

### Intermédiaire 3 : Acide 6-(1-triphénylméthyl-1H-imidazol-4-yl)imidazo[1,2-a]pyridine-2-carboxylique

### 3.1 : 6-(1-Triphénylméthyl-1H-imidazol-4-yl)imidazo[1,2-a]pyridine-2-carboxylate d'éthyle

873 mg de 4-iodo-1-triphénylméthyl-imidazole, 750 mg d'acide 2-éthoxycarbonyl-imidazo[1,2-*a*]pyridine-6-boronique, 23 mg d'acétate de palladium et 70 mg de (2-biphényl)-dicyclohexylphosphine sont dégazés sous vide puis mis en suspension, sous argon, dans un mélange dégazé de 15 mL de toluène, 5 mL d'eau et 5 mL de *N*-méthylpyrrolidone. Après ajout de 950 mg de phosphate de potassium, le mélange est dégazé sous vide puis placé sous argon et chauffé 15 minutes à 100 °C sous microondes, puis refroidi, dilué et agité dans un mélange de 50 mL de solution saturée de bicarbonate de sodium et 50 mL de dichlorométhane. La phase organique est séchée sur sulfate de sodium, filtrée et concentrée à sec sous pression réduite. Le résidu est chromatographié sur silice en éluant par un mélange d'acétate d'éthyle et d'hexane. Les fractions contenant le produit attendu sont réunies et concentrées à sec sous pression réduite pour donner 508 mg de 6-(1-triphénylméthyl-1*H*-imidazol-4-yl)imidazo[1,2-*a*]pyridine-2-carboxylate d'éthyle.

Spectre RMN ¹H (DMSO-d6, δ en ppm): 8,97 (s, 1H), 8,54 (s, 1H), 7,76-7,72 (m, 1H), 7,56-7,52 (m, 3H), 7,47-7,37 (m, 9H), 7,20-7,17 (m, 6H), 4,31-4,27 (m, 2H), 1,34-1,20 (m, 3H).

Spectre de masse (APCI): m/z= 499 [M+H]⁺.

### 3.2 : Acide 6-(1-triphénylméthyl-1H-imidazol-4-yl)imidazo[1,2-a]pyridine-2-carboxylique

500 mg de 6-(1-triphénylméthyl-1*H*-imidazol-4-yl)imidazo[1,2-*a*]pyridine-2-carboxylate d'éthyle sont saponifiés dans des conditions analogues à celles décrites pour la préparation de l'intermédiaire 1 (étape 1.3) pour donner 346 mg d'acide 6-(1-triphénylméthyl-1*H* imidazol-4-yl)imidazo[1,2-*a*]pyridine-2-carboxylique.

Spectre RMN ¹H (DMSO-d6, δ en ppm) : 9,01 (s, 1H), 8,51 (s, 1H), 7,83 (d, J = 9,5, 1H), 7,59-7,56 (m, 3H), 7,47-7,37 (m, 9H), 7,20-7,17 (m, 6H). Un proton échangeable n'est pas observé

Spectre de masse (APCI): m/z= 471 [M+H)⁺.

### Intermédiaire 4 : Acide 6-(2-{[(1,1-diméthyléthoxy)carbonyl]amino}-thiazol-4-yl)imidazo[1,2-a]pyridine-2-carboxylique

### 4.1 : 6-(2-{[(1,1-Diméthyléthoxy)carbonyl]amino}-thiazol-4-yl)imidazo[1,2-a]pyridine-2-carboxylate d'éthyle

465 mg de 4-iodothiazol-2-ylcarbamate de tert-butyle, 434 mg d'acide 2-éthoxycarbonyl-imidazo[1,2-*a*]pyridine-6-boronique et 104 mg de [1,1'-bis(diphenylphosphino)-ferrocène]dichloropalladium sont dégazés sous vide. Après ajout de 10 mL de tétrahydrofuranne dégazé et 0,66 mL de solution aqueuse 2N de carbonate de sodium, le mélange réactionnel est chauffé 2 heures à 100 °C, puis refroidi, dilué dans du dichlorométhane et lavé par une solution aqueuse demi-saturée de bicarbonate de. La phase organique est séchée sur sulfate de magnésium, filtrée et concentrée à sec sous pression réduite. Le résidu est chromatographié sur silice en éluant par un mélange de dichlorométhane : méthanol (99:1 à 99:2). Les fractions contenant le produit attendu sont réunies et concentrées à sec sous pression réduite. Le solide obtenu est lavé par 5 mL d'éther diéthylique pour donner 125 mg de 6-(2-{[(1,1-diméthyléthoxy)carbonyl]amino}-thiazol-4-yl)imidazo[1,2-*a*]pyridine-2-carboxylate d'éthyle sous la forme d'un solide blanc cassé.

Spectre RMN ¹H (DMSO-d6, δ en ppm) : 11,65 (s, 1H), 8,84 (s, 1H), 8,47 (s, 1H), 7,84 (s, 1H), 7,68-7,71 (m, 2H), 4,32 (q, J = 7,1, 2H), 1,51 (s, 9H), 1,33 (t, J = 7,1, 3H).

Spectre de masse (APCI): m/z= 389 [M+H]⁺.

### 4.2 : Acide 6-(2-{[(1,1-diméthyléthoxy)carbonyl]amino}-thiazol-4-yl)imidazo[1,2-a]pyridine-2-carboxylique

125 mg de 6-(2-{[(1,1-diméthyléthoxy)carbonyl]amino}-thiazol-4-yl)imidazo[1,2-*a*]pyridine-2-carboxylate d'éthyle sont saponifiés dans des conditions analogues à celles décrites pour la préparation de l'intermédiaire 1 (étape 1.3) pour donner 90 mg d'acide 6-(2-{[(1,1-diméthyléthoxy)carbonyl]amino}-thiazol-4-yl)imidazo[1,2-*a*]pyridine-2-carboxylique sous la forme d'un solide brun.

Spectre RMN ¹H (DMSO-d6, δ en ppm) : 11,66 (s, 1H), 8,86 (s, 1H), 8,42 (s, 1H), 7,84 (s, 1H), 7,67-7,69 (m, 2H), 1,51 (s, 9H).

Spectre de masse (APCI): m/z= 361 [M+H]⁺.

### Intermédiaire 5 : Acide 6-(1H-pyrrol-3-yl)imidazo[1,2-a]pyridine-2-carboxylique

### 5.1 6-[1-(Triisopropylsilyl)-1H-pyrrol-3-yl]imidazo[1,2-a]pyridine-2-carboxylate d'éthyle

100 mg de 6-iodo-imidazo[1,2-*a*]pyridine-2-carboxylate d'éthyle, 135 mg d'acide 1-(triisopropylsilyl)-pyrrole-3-boronique et 18 mg de tétrakis(triphénylphosphine)palladium(0) sont dégazés sous vide puis mis en suspension, sous argon, dans un mélange dégazé de 1,5 mL de 1,2-diméthoxyéthane, 1,5 mL d'éthanol et 316 µL de solution aqueuse 2N de carbonate de sodium. Le mélange réactionnel est chauffé au reflux pendant 4 heures, puis refroidi, dilué et agité avec un mélange de 5 mL de solution aqueuse demi-saturée de bicarbonate de sodium et 5 mL de dichlorométhane. La phase organique est séchée sur sulfate de sodium, filtrée et concentrée à sec sous pression réduite. Le résidu est chromatographié sur silice en éluant par un mélange d'acétate d'éthyle et d'hexane (50/50). Les fractions contenant le produit attendu sont réunies et concentrées à sec sous pression réduite pour donner 121 mg de 6-[1-(triisopropylsilyl)-1*H*-pyrrol-3-yl]imidazo[1,2-*a*]pyridine-2-carboxylate d'éthyle.

Spectre RMN ¹H (DMSO-d6, δ en ppm) : 8,76 (s, 1H), 8,42 (s, 1H), 7,70 (dd, J = 1,9, 9,7 1H), 7,59 (d, J = 9,7 1H), 7,37 (s large, 1H), 6,94 (m, 1H), 6,63 (m, 1H), 4,33 (q, J = 6,9, 2H), 1,61-1,50 (m, 3H), 1,33 (t, J = 6,9, 3H), 1,10-1,03 (m, 18H).

Spectre de masse (APCI): m/z= 412 [M+H]⁺.

### 5.2 : Chlorhydrate (1,1) de l'acide 6-(1H-pyrrol-3-yl)imidazo[1,2-a]pyridine-2-carboxylique

292 mg de 6-[1-(triisopropylsilyl)-1*H*-pyrrol-3-yl]imidazo[1,2-*a*]pyridine-2-carboxylate d'éthyle sont saponifiés dans des conditions analogues à celles décrites pour la préparation de l'intermédiaire 1 (étape 1.3) pour donner 140 mg de chlorhydrate (1:1) de l'acide 6-(1*H*-pyrrol-3-yl)imidazo[1,2-*a*]pyridine-2-carboxylique sous la forme d'un solide blanc.

Spectre RMN ¹H (DMSO-d6, δ en ppm) :11,07 (s large, 1H), 8,73 (s, 1H), 8,39 (s, 1H), 7,69 (dd, J = 1,3, 9,5, 1H), 7,59 (d, J = 9,5, 1H), 7,31 (s, 1H), 6,86 (s, 1H), 6,46 (s, 1H).

Spectre de masse (APCI): m/z= 228 [M+H]⁺.

### Intermédiaire 6 : Acide 6-(1H-pyrazol-3-yl)imidazo[1,2-a]pyridine-2-carboxylique

### 6.1 : 6-(1H-pyrazol-3-yl)imidazo[1,2-a]pyridine-2-carboxylate d'éthyle

Ce produit est préparé dans les conditions analogues à celles décrites pour la préparation de l'intermédiaire 5 (étape 5.1) en remplaçant l'acide 1-(triisopropylsilyl)-pyrrole-3-boronique par l'acide-pyrrazole-3-boronique.

Spectre RMN ¹H (MeOD-d4, δ en ppm) : 8,89 (t, J = 1,2 et 2,4, 1H), 8,45 (d, J = 0,6, 1H), 7,89 (d, J = 9,0, 1H), 7,76 (s large, 1H), 7,67 (d, J = 9,5, 1H), 6,77 (d, J = 2,4, 1H), 4,42 (q, J = 7,1, 2H), 1,43 (t, J = 7,1, 3H).

Spectre de masse (APCI): m/z= 257 [M+H]⁺.

### 6.2 : Acide 6-(1H-pyrazol-3-yl)imidazo[1,2-a]pyridine-2-carboxylique

128 mg de 6-(1*H* pyrazol-3-yl)imidazo[1,2-α]pyridine-2-carboxylate d'éthyle sont saponifiés dans des conditions analogues à celles décrites pour la préparation de l'intermédiaire 1 (étape 1.3) pour donner 113 mg d'acide 6-(1*H*-pyrazol-3-yl)imidazo[1,2-a]pyridine-2-carboxylique.

Spectre RMN ¹H (DMSO-d6, δ en ppm) : 13,50-12,50 (s large, 1H), 9,03 (s, 1H), 8,40 (s, 1H), 7,83-7,80 (m, 2H), 7,63 (d, J = 9,4, 1H), 6,74 (s, 1H).

### Intermédiaire 7 : Acide 6-(1H-pyrazol-4-yl)imidazo[1,2-a]pyridine-2-carboxylique

### 7.1 : 6-(1H-pyrazol-4-yl)imidazo[1,2-a]pyridine-2-carboxylate d'éthyle

Ce produit est préparé dans les conditions analogues à celles décrites pour la préparation de l'intermédiaire 5 (étape 5.1) en remplaçant l'acide 1-(triisopropylsilyl)-pyrrole-3-boronique par l'acide-pyrrazole-4-boronique et en chauffant à 90°C sous microondes pendant 37 minutes.

Spectre RMN ¹H (DMSO-d6, δ en ppm) : 13,10 (s large, 1H), 8,83 (s, 1H), 8,43 (s, 1H), 8,25 (s large, 1H), 7,94 (s large, 1H), 7,69-7,61 (m, 2H), 4,31 (q, J = 7,1, 2H), 1,32 (t, J = 7,1, 3H).

Spectre de masse (APCI): m/z= 257 [M+H]⁺.

### 7.2 : Acide 6-(1H-pyrazol-4-yl)imidazo[1,2-a]pyridine-2-carboxylique

128 mg de 6-(1*H*-pyrazol-4-yl)imidazo[1,2-*a*]pyridine-2-carboxylate d'éthyle sont saponifiés dans des conditions analogues à celles décrites pour la préparation de l'intermédiaire 1 (étape 1.3) pour donner 60 mg d'acide 6-(1*H*-pyrazol-4-yl)imidazo[1,2-*a*]yridine-2-carboxylique.

Spectre RMN ¹H (DMSO-d6, δ en ppm) : 14,0-12,0 (s large, 1H), 8,84 (s, 1H), 8,36 (s, 1H), 8,10 (s, 2H), 7,64 (s, 2H).

### Intermédiaire 8 : Acide 6-(furan-2-yl)imidazo[1,2-a]pyridine-2-carboxylique

### 8.1 : 6-Furan-2-yl)imidazo[1,2-a]pyridine-2-carboxylate d'éthyle

Ce produit est préparé dans les conditions analogues à celles décrites pour la préparation de l'intermédiaire 5 (étape 5.1) en remplaçant l'acide 1-(triisopropylsilyl)-pyrrole-3-boronique par l'acide-furanne-2-boronique.

Spectre RMN ¹H (DMSO-d6, δ en ppm) : 8,78 (s, 1H), 8,44 (s, 1H), 7,72 (dd, J = 1,8, 9,6, 1H), 7,63-7,60 (m, 2H), 6,89 (d, J = 3,4, 1H), 6,57 (dd, J = 1,8, 3,4, 1H), 4,42 (q, J = 7,1, 2H), 1,42 (t, J = 7,1, 3H).

Spectre de masse (APCI): m/z= 257 [M+H]⁺.

### 8.2 : Acide 6-furan-2-yl)imidazo[1,2-a]pyridine-2-carboxylique

384 mg de 6-furan-2-yl)imidazo[1,2-*a*]pyridine-2-carboxylate d'éthyle sont saponifiés dans des conditions analogues à celles décrites pour la préparation de l'intermédiaire 1 (étape 1.3) pour donner 256 mg d'acide 6-furan-2-yl)imidazo[1,2-*a*]pyridine-2-carboxylique.

Spectre RMN ¹H (DMSO-d6, δ en ppm) : 8,86 (s, 1H), 8,38 (s, 1H), 7,80 (dd, J = 1,7, 9,5, 1H), 7,67-7,64 (m, 2H), 6,90 (d, J = 3,4, 1H), 6,60 (dd, J = 1,8, 3,4, 1H).

### Intermédiaire 9 : Acide 6-(furan-3-yl)imidazo[1,2-a]pyridine-2-carboxylique

### 9.1 : 6-Furan-3-yl)imidazo[1,2-a]pyridine-2-carboxylate d'éthyle

Ce produit est préparé dans les conditions analogues à celles décrites pour la préparation de l'intermédiaire 5 (étape 5.1) en remplaçant l'acide 1-(triisopropylsilyl)-pyrrole-3-boronique par l'acide-furanne-3-boronique.

Spectre RMN ¹H (DMSO-d6, δ en ppm) : 8,86 (s, 1H), 8,45 (s, 1H), 8,28 (s, 1H), 7,82 (s, 1H), 7,66 (s, 2H), 6,95 (s, 1H), 4,31 (q, J = 7,1, 2H), 1,33 (t, J = 7,1, 3H).

Spectre de masse (APCI): m/z= 257 [M+H]⁺.

### 9.2 : Acide 6-furan-3-yl)imidazo[1,2-a]pyridine-2-carboxylique

384 mg de 6-furan-3-yl)imidazo[1,2-*a*]pyridine-2-carboxylate d'éthyle sont saponifiés dans des conditions analogues à celles décrites pour la préparation de l'intermédiaire 1 (étape 1.3) pour donner 287 mg d'acide 6-furan-3-yl)imidazo[1,2-*a*]pyridine-2-carboxylique.

Spectre RMN ¹H (DMSO-d6, δ en ppm) : 8,86 (s, 1H), 8,38 (s, 1H), 8,27 (s, 1H), 7,81 (s, 1H), 7,64 (s, 2H), 6,95 (s, 1H).

Spectre de masse (APCI): m/z= 229 [M+H]⁺.

### Intermédiaire 10 : Acide 6-[5-(hydroxyméthyl)furan-2-yl]imidazo[1,2-a]pyridine-2-carboxylique

### 10.1 : 6-(5-Formylfuran-2-yl)imidazo[1,2-a]pyridine-2-carboxylate d'éthyle

2 g de 6-iodo-imidazo[1,2-*a*]pyridine-2-carboxylate d'éthyle, 1,42 g d'acide 5-formyl-furanne-2-boronique et 231 mg de [1,1'-bis(diphenylphosphino)ferrocène]dichloropalladium sont dégazés sous vide puis mis en suspension, sous argon, dans un mélange dégazé de 30 mL de dioxanne et 9,4 mL d'une solution aqueuse 2N de carbonate de sodium. Le mélange réactionnel est chauffé 5 heures à 90 °C, puis agité 16 heures à 20 °C et concentré à sec. Le résidu est chromatographié sur silice en éluant par un mélange d'acétate d'éthyle et d'hexane (90/10), par de l'acétate d'éthyle puis par un mélange (99/1) d'acétate d'éthyle et de méthanol. Les fractions contenant le produit attendu sont réunies et concentrées à sec sous pression réduite pour donner 884 mg de 6-(5-formylfuran-2-yl)imidazo[1,2-*a*]pyridine-2-carboxylate d'éthyle.

Spectre RMN ¹H (DMSO-d6, δ en ppm) : 9,64 (s, 1H), 9,20 (s, 1H), 8,66 (s, 1H), 7,86-7,74 (m, 2H), 7,72 (d, J = 3,8, 1H), 7,37 (d, J = 3,8, 1H), 4,33 (q, J = 7,0, 2H), 1,33 (t, J = 7,1, 3H).

Spectre de masse (APCI): m/z= 285 [M+H]⁺.

### 10.2 : 6-[5-(Hydroxyméthyl)furan-2-yl]imidazo[1,2-a]pyridine-2-carboxylate d'éthyle

A une suspension de 770 mg de 6-(5-formylfuran-2-yl)imidazo[1,2-*a*]pyridine-2-carboxylate d'éthyle dans 15 mL d'éthanol, on ajoute 123 mg de borohydrure de sodium. Le mélange réactionnel est agité à 25 °C pendant 90 minutes puis dilué et agité avec 10 mL de dichlorométhane et 3 mL d'une solution aqueuse demi-saturée de carbonate de sodium. La phase organique est séparée, séchée sur sulfate de magnésium, filtrée et concentrée sous pression réduite. Le résidu est chromatographié sur silice en éluant par un mélange de dichlorométhane et de méthanol (98/2). Les fractions contenant le produit attendu sont réunies et concentrées à sec sous pression réduite. Le solide obtenu est trituré dans 5 mL de dichlorométhane, filtré et séché pour donner 403 mg de 6-(5-formylfuran-2-yl)imidazo[1,2-*a*]pyridine-2-carboxylate d'éthyle sous la forme d'un solide blanc.

Spectre RMN ¹H (DMSO-d6, δ en ppm) : 8,89 (s, 1H), 8,60 (s, 1H), 7,70 (m, 2H), 6,98 (d, J = 3,3, 1H), 6,45 (d, J = 3,3, 1H), 5,30 (t, J = 5,3, 1H), 4,47 (d, J = 5,6, 2H), 4,32 (q; J = 7,1, 2H), 1,32 (t, J = 7,1, 3H).

Spectre de masse (APCI): m/z= 287 [M+H]⁺.

### 10.3 : Acide 6-[5-(hydroxyméthyl)furan-2-yl]imidazo[1,2-a]pyridine-2-carboxylique

400 mg de 6-[5-(hydroxyméthyl)furan-2-yl]imidazo[1,2-*a*]pyridine-2-carboxylate d'éthyle sont saponifiés dans des conditions analogues à celles décrites pour la préparation de l'intermédiaire 1 (étape 1.3) pour donner 346 mg d'acide 6-[5-(hydroxyméthyl)furan-2-yl]imidazo[1,2-*a*]pyridine-2-carboxylique sous la forme d'un solide blanc.

Spectre RMN ¹H (DMSO-d6, δ en ppm) : 9,06 (s, 1H), 8,73 (s, 1H), 8,03 (d, J = 9,5, 1H), 7,82 (d, J = 9,5, 1H), 7,09 (d, J = 3,3, 1H), 6,49 (d, J = 3,2, 1H), 4,49 (s, 2H).

Spectre de masse (APCI): m/z= 259 [M+H]⁺.

### Intermédiaire 11 : Acide 6-(thiophén-3-yl)imidazo[1,2-a]pyridine-2-carboxylique

### 11.1 : 6-(Thiophén-3-yl)imidazo[1,2-a]pyridine-2-carboxylate d'éthyle

Ce produit est préparé dans les conditions analogues à celles décrites pour la préparation de l'intermédiaire 5 (étape 5.1) en remplaçant l'acide 1-(triisopropylsilyl)-pyrrole-3-boronique par l'acide-thiophène-3-boronique (catalyseur : dichloro-bis(triphénylphosphine)-palladium.

Spectre RMN ¹H (DMSO-d6, δ en ppm) : 1,34 (d, J=7,1 Hz, 3H), 4,32 (q, J=7,1 Hz, 2H), 7,56 (dd, J=5,0, 1,4 Hz, 1H), 7,68 (d, J=9,8 Hz, 1H), 7,73 (dd, J=5,0, 3,0 Hz, 1H), 7,78 (dd, J=9,8, 1,8 Hz, 1H), 7,97 (dd, J=3,0, 1,4 Hz, 1H), 8,48 (s, 1H), 8,98 (s large, 1H).

Spectre de masse (LC-MS-DAD-ELSD) : m/z 273 [M+H]⁺.

### 11.2 : Acide 6-(thiophèn-3-yl)imidazo[1,2-a]pyridine-2-carboxylique

310 mg de 6-(thiophén-3-yl)imidazo[1,2-*a*]pyridine-2-carboxylate d'éthyle sont saponifiés dans des conditions analogues à celles décrites pour la préparation de l'intermédiaire 1 (étape 1.3) pour donner 250 mg d'acide 6-(thiophèn-3-yl)imidazo[1,2-*a*]pyridine-2-carboxylique.

Spectre RMN ¹H (DMSO-d6, δ en ppm) : 7,57 (d, J=5,4 Hz, 1H), 7,66 (d, J=9,8 Hz, 1H), 7,73 (dd, J=5,4, 2,8 Hz, 1H), 7,76 (dd, J=9,8, 2,0 Hz, 1H), 7,97 (d large, J=2,0 Hz, 1H), 8,41 (s, 1H), 8,99 (s large, 1H).

Spectre de masse (LC-MS-DAD-ELSD) : m/z 245 [M+H]⁺.

### Intermédiaire 12 : Acide 6-(oxazol-2-yl)imidazo[1,2-a]pyridine-2-carboxylique

### 12.1 : 6-(Oxazol-2-yl)imidazo[1,2-a]pyridine-2-carboxylate d'éthyle

1 g de 6-iodo-imidazo[1,2-*a*]pyridine-2-carboxylate d'éthyle, 350 mg de tétrakis-(triphénylphosphine)palladium(0) et 360 mg de chlorure de lithium sont dégazés sous vide puis mis en suspension, sous argon, dans 15 mL de dioxanne dégazé. Après ajout de 5 g de 2-(tri-n-butylstannyl)oxazole, le mélange réactionnel est chauffé à 90°C pendant 3,5 heures, puis refroidi, dilué et agité avec un mélange de 100 mL de solution aqueuse 1M de fluorure de potassium et 200 mL d'acétate d'éthyle. La phase aqueuse est extraite par 200 mL d'acétate d'éthyle et les phases organiques réunies sont lavées par de la saumure et séchées sur sulfate de sodium, filtrées et concentrées à sec sous pression réduite. Le résidu est chromatographié sur silice en éluant par un gradient d'acétate d'éthyle et d'hexane (de 80/20 à 100/0). Les fractions contenant le produit attendu sont réunies et concentrées à sec sous pression réduite pour donner 530 mg de 6-(oxazol-2-yl)imidazo[1,2-*a*]pyridine-2-carboxylate d'éthyle sous la forme d'une poudre jaune.

Spectre RMN ¹H (DMSO-d6, δ en ppm) : 9,30 (d, J = 0,8, 1H), 8,68 (s, 1H), 8,30 (s, 1H), 7,85 (dd, J = 1,7, 9,5, 1H), 7,79 (d, J = 9,5, 1H), 7,44 (d, J = 0,6, 1H), 4,33 (q, J = 7,0, 2H), 1,33 (t, J = 7,1, 3H).

Spectre de masse (APCI): m/z= 258 [M+H]⁺.

### 12.2 : Acide 6-(oxazol-2-yl)imidazo[1,2-a]pyridine-2-carboxylique

512 mg de 6-(oxazol-2-yl)imidazo[1,2-*a*]pyridine-2-carboxylate d'éthyle sont saponifiés dans des conditions analogues à celles décrites pour la préparation de l'intermédiaire 1 (étape 1.3) pour donner 365 mg d'acide 6-(oxazol-2-yl)imidazo[1,2-*a*]pyridine-2-carboxylique sous la forme d'un solide blanc.

Spectre RMN ¹H (DMSO-d6, δ en ppm) : 9,41 (s, 1H), 8,73 (s, 1H), 8,34 (s, 1H), 8,05 (dd, J = 1,5, 9,5, 1H), 7,86 (d, J = 9,5, 1 H), 7,48 (s, 1H).

### Intermédiaire 13 : Acide 6-(1H-1,2,4-triazol-3-yl)imidazo[1,2-a]pyridine-2-carboxylique

### 13.1 : 6-[éthoxy(imino)méthyl]imidazo[1,2-a]pyridine-2-carboxylate d'éthyle

470 mg d'éthanethiolate de sodium sont ajoutés à une solution de 1 g de 6-cyanoimidazo[1,2-*a*]pyridine-2-carboxylate d'éthyle (J. Med. Chem. (1998), 41(22), 4317) dans un mélange de 15 mL d'éthanol et de 10 mL de dichlorométhane refroidie à 0°C. Le mélange réactionnel est agité 5 heures à 25°C, filtré et le filtrat évaporé à sec. Le résidu est chromatographié sur silice en éluant par un mélange de dichlorométhane et de méthanol (98/2) pour donner 625 mg de 6-[éthoxy(imino)méthyl]imidazo[1,2-*a*]pyridine-2-carboxylate d'éthyle sous la forme d'un solide jaune pâle.

Spectre RMN ¹H (DMSO-d6, δ en ppm) : 9,17 (s, 1H), 9,04 (s, 1H), 8,64 (s, 1H), 7,84 (m, 1H), 7,68 (m, 1H), 4,33 (q, J = 7,1, 4H), 1,34 (t = 7,2, 6H).

Spectre de masse (APCI): m/z= 262 [M+H]⁺.

### 13.2 : 6-[Hydrazino(imino)méthyl]imidazo[1,2-a]pyridine-2-carboxylate d'éthyle

A une solution de 625 mg de 6-[éthoxy(imino)méthyl]imidazo[1,2-*a*]pyridine-2-carboxylate d'éthyle dans 12 mL d'éthanol, on ajoute goutte à goutte à 0-5°C 0,2 mL d'hydrate d'hydrazine. Le mélange réactionnel est agité 2 heures puis on ajoute 73 µL d'hydrate d'hydrazine et on agite encore 2 heures en laissant remonter la température à 25°C. Le mélange réactionnel est concentré à sec sous pression réduite et le résidu séché pour donné 600 mg de 6-[hydrazino(imino)méthyl]imidazo[1,2-*a*]pyridine-2-carboxylate d'éthyle qui est utilisé sans autre purification dans la suite de la synthèse.

Spectre RMN ¹H (DMSO-d6, δ en ppm): 8,77 (s large, 1H), 8,49 (s, 1H), 7,70 (m, 1H), 7,53 (d, J = 9,6, 1H), 5,67 (s, 2H), 5,15 (s large, 2H), 4,33 (q, J = 7,1, 2H), 1,32 (t = 7,1, 3H).

Spectre de masse (APCI): m/z= 248 [M+H]⁺.

### 13.3 : 6-(1H-1,2,4-triazol-3-yl)imidazo[1,2-a]pyridine-2-carboxylate d'éthyle

Une suspension de 580 mg de 6-[hydrazino(imino)méthyl]imidazo[1,2-*a*]pyridine-2-carboxylate d'éthyle dans 6 mL d'acide formique est chauffée 20 heures à 85°C. Le mélange réactionnel est concentré à moins de 20 % de son volume initial et dilué par 20 mL d'eau. On ajoute à 0-5°C du carbonate de sodium solide jusqu'à atteindre pH 8-9. Le précipité est essoré puis purifié par chromatographie sur silice en éluant par un mélange de dichlorométhane et de méthanol (98/2) pour donner 320 mg de : 6-(1H-1,2,4-triazol-3-yl)imidazo[1,2-*a*]pyridine-2-carboxylate d'éthyle.

Spectre RMN ¹H (DMSO-d6, δ en ppm) : 14,5-14,0 (s large, 1H), 9,25 (s, 1H), 8,69 (s, 1H), 8,63 (s large, 1H), 7,94 (dd, J = 9,5, 1,5, 1H), 7,73 (d, J = 9,5, 1H), 4,33 (q, J = 7,0, 2H), 1,33 (t = 7,0, 3H)

Spectre de masse (APCI): m/z= 258 [M+H]⁺.

### 13.4 : Acide 6-(1H-1,2,4-triazol-3-yl)imidazo[1,2-a]pyridine-2-carboxylique

320 mg de 6-(1H-1,2,4-triazol-3-yl)imidazo[1,2-*a*]pyridine-2-carboxylate d'éthyle sont saponifiés dans des conditions analogues à celles décrites pour la préparation de l'intermédiaire 1 (étape 1.3) pour donner 238 mg d'acide 6-(1*H*-1,2,4-triazol-3-yl)imidazo[1,2-*a*]pyridine-2-carboxylique sous la forme d'un solide blanc cassé.

Spectre RMN ¹H (DMSO-d6, δ en ppm) : 14,5-14,2 (s large, 1H), 9,26 (s, 1H), 8,66-8,62 (m, 2H), 7,91 (d, J = 9,1, 1H), 7,73 (d, J = 9,6, 1H).

### Intermédiaire 14 : Acide 6-(1H-1,2,3-triazol-4-yl)imidazo[1,2-a]pyridine-2-carboxylique

### 14.1 : 6-[(Triméthylsilyl)éthynyl]imidazo[1,2-a]pyridine-2-carboxylate d'éthyle

Un mélange de 4 g de 6-iodo-imidazo[1,2-*a*]pyridine-2-carboxylate d'éthyle, 2,63 mL d'éthynyl-trimethylsilane, 888 mg de dichloro-bis(triphénylphosphine)palladium est dégazé sous vide. On ajoute 240 mg de N,N-diméthylformamide dégazé, 3,52 mL de triéthylamine. Le mélange réactionnel est dégazé sous argon puis agité à 50°C pendant 50 heures puis refroidi, dilué par 20 mL d'eau. Le précipité est essoré et lavé par 5 mL d'eau puis chromatographié sur silice en éluant par des mélanges d'acétate d'éthyle et d'hexane (de 50/50 à 90/10). Les fractions contenant le produit attendu sont réunies et concentrées à sec sous pression réduite pour donner 3,6 g de 6-[(triméthylsilyl)éthynyl]imidazo[1,2-*a*]pyridine-2-carboxylate d'éthyle sous la forme d'un solide blanc cassé.

Spectre RMN ¹H (DMSO-d6, δ en ppm) : 8,61 (s, 1H), 8,22 (s, 1H), 7,36 (d, J = 9,5, 1H), 7,07 (dd, J = 9,5, 1,7, 1H), 4,07 (q, J = 7,1, 2H), 1,08 (t, J = 7,1, 3H), 0,01 (s, 9H).

Spectre de masse (APCI): m/z= 287 [M+H]⁺.

### 14.2 : 6-Ethynylimidazo[1,2-a]pyridine-2-carboxylate d'éthyle

A une solution de 500 mg de 6-[(triméthylsilyl)éthynyl]imidazo[1,2-*a*]pyridine-2-carboxylate d'éthyle dans 10 mL de tétrahydrofuranne anhydre, refroidie à 0°C on ajoute goutte à goutte 1,58 mL dune solution 1M de fluorure de tétrabutylammonium dans le tétrahydrofuranne. Le mélange réactionnel est agité pendant 30 minutes puis on ajoute 5 mL d'eau et extrait 3 fois par 20 mL de dichlorométhane. Le produit est purifié par chromatographie sur silice en éluant par des mélanges d'acétate d'éthyle et d'hexane (de 1/3 à 1/1). Les fractions contenant le produit attendu sont réunies et concentrées à sec sous pression réduite pour donner 280 mg de 6-éthynylimidazo[1,2-*a*]pyridine-2-carboxylate d'éthyle sous la forme d'un solide jaune.

Spectre RMN ¹H (DMSO-d6, δ en ppm) : 8,86 (d, J = 1,0, 1H), 8,50 (d, J = 0,6, 1H), 7,63 (d, J = 9,4, 1H), 7,37 (d, J = 1,7, 9,4, 1H), 4,32 (m, 3H), 1,32 (t, J = 7,1 Hz, 3H).

Spectre de masse (APCI): m/z= 215 [M+H]⁺.

### 14.3 : 6-(1H-1,2,3-triazol-4-yl)imidazo[1,2-a]pyridine-2-carboxylate d'éthyle

A une solution de 220 mg de 6-éthynylimidazo[1,2-*a*]pyridine-2-carboxylate d'éthyle et 0,21 mL d'azidotriméthylsilane dans 4 mL d'un mélange (9/1) de *N*,*N*-diméthylformamide et de méthanol on ajoute 9,8 mg d'iodure cuivreux. Le mélange réactionnel est agité 2 heures à 100°C puis refroidi et dilué par 4 mL de dichlorométhane puis filtré sur alumine et concentré à sec. Le résidu est chromatographié sur silice en éluant par un mélange de dichlorométhane et d'éthanol (97/3). Les fractions contenant le produit attendu sont réunies et concentrées à sec sous pression réduite pour donner 125 mg de : 6-(1*H*-1,2,3-triazol-4-yl)imidazo[1,2-*a*]pyridine-2-carboxylate d'éthyle sous la forme d'un solide blanc cassé.

Spectre RMN ¹H (DMSO-d6, δ en ppm) : 15,5-15,0 (s large, 1H), 9,14 (dd, J = 1,1, 1,5, 1H), 8,60 (d, J = 0,5, 1H), 8,40 (s large, 1H), 7,82 (dd, J = 1,7, 9,5, 1H), 7,75 (d, J = 9,5, 1H), 4,33 (q, J = 7,1, 2H), 1,33 (t, J = 7,1, 3H).

Spectre de masse (APCI): m/z= 258 [M+H]⁺.

### 14.4 : Acide 6-(1H-1,2,3-triazol-4-yl)imidazo[1,2-a]pyridine-2-carboxylique

125 mg de 6-(1*H*-1,2,3-triazol-3-yl)imidazo[1,2-*a*]pyridine-2-carboxylate d'éthyle sont saponifiés dans des conditions analogues à celles décrites pour la préparation de l'intermédiaire 1 (étape 1.3) pour donner 72 mg d'acide 6-(1*H*-1,2,3-triazol-3-yl)imidazo[1,2-*a*]pyridine-2-carboxylique sous la forme d'un solide blanc.

Spectre RMN ¹H (DMSO-d6, δ en ppm) : 16,0-15,0 (s large, 1H), 9,23 (s, 1H), 8,62 (s, 1H), 8,46 (s large, 1H), 7,96 (dd, J = 1,4, 9,5, 1H), 7,80 (d, J = 9,5, 1H).

### Intermédiaire 15 : 6-iodo-N-(isoxazol-4-yl)imidazo[1,2-a]pyridine-2-carboxamide

A une suspension de 200 mg d'acide 6-iodoimidazo[1,2-*a*]pyridine-2-carboxylique et 266 mg de chlorhydrate de 1-(3-diméthylaminopropyl)-3-éthylcarbodiimide dans 1 mL de pyridine anhydre, on ajoute 175 mg d'isoxazol-4-yl-amine. Le mélange réactionnel est agité pendant 16 heures à 50 °C puis concentré à sec sous pression réduite. Le résidu est repris par 10 mL d'un mélange de chloroforme et d'eau (1/1). Le solide est trituré avec 3 mL d'eau, essoré et lavé 3 mL d'eau puis avec 3 mL d'éther éthylique et séché pour donner 280 mg de 6-iodo-*N-*(isoxazol-4-yl)imidazo[1,2-*a*]pyridine-2-carboxamide sous la forme d'un solide beige.

Spectre RMN ¹H (DMSO-d6, δ en ppm) : 10,93 (s large, 1H), 9,24 (s large, 1H), 9,02 (s large, 1H), 8,81 (s large, 1H), 8,43 (s large, 1H), 7,60-7,48 (m, 2H).

Spectre de masse (APCI): m/z= 258 [M+H]⁺.

### Intermédiaire 16 : 6-iodo-N-(thiazol-2-yl)imidazo[1,2-a]pyridine-2-carboxamide

Ce produit est préparé dans les conditions analogues à celles décrites pour la préparation de l'intermédiaire 15 en remplaçant l'isoxazol-4-yl-amine par la thiazol-2-ylamine.

Spectre RMN ¹H (DMSO-d6, δ en ppm) : 11,95 (brs, 1H), 9,04 (brs, 1H), 8,59 (brs, 1H), 7,60-7,51 (m, 3H), 7,30 (d, J = 3,4, 1H).

Spectre de masse (APCI): m/z= 371 [M+H]⁺.

### Intermédiaire 17 : 6-Iodo-N-(pyridin-2-yl)imidazo[1,2-a]pyridine-2-carboxamide

Une suspension de 1 g de 6-iodo-imidazo[1,2-*a*]pyridine-2-carboxylate d'éthyle, 330 mg de 2-pyridylamine, 92 mg de 1-hydroxy-7-azabenzotriazole (HOAt) et 787 mg de tertbutylate de zirconium dans 12 mL de toluène est agitée 16 heures à température ambiante puis chauffée au reflux pendant 6 heures. Après refroidissement, le milieu est dilué dans de l'acétate d'éthyle et filtré. D'une part, le solide est repris par du dichlorométhane et une solution aqueuse saturée d'hydrogénocarbonate de sodium. D'autre part, le filtrat est concentré à sec, repris par de l'eau et du dichlorométhane, la phase organique est séparée, séchée et concentrée à sec. Les solides obtenu de part et d'autre sont réunis et trituré avec du dichlorométhane pour donner 1,42 g de 6-iodo-*N*-(pyridin-2-yl)imidazo[1,2-*a*]pyridine-2-carboxamide sous la forme d'un solide jaune pâle.

Spectre RMN 1H (DMSO-d6, δ en ppm) : 7,19 (dd, J = 5,0 et 8,0 Hz, 1H), 7,55 (d, J = 9,5 Hz, 1H), 7,60 (dd, J = 2,0 et 9,5 Hz, 1H), 7,89 (dt, J = 2,0 et 8,0 Hz, 1H), 8,22 (d, J = 8,0 Hz, 1H), 8,38 (dd, J = 2,0 et 5,0 Hz, 1H), 8,51 (s, 1H), 9,01 (s large, 1H), 9,79 (s, 1H).

Spectre de masse (IC) : m/z 365 [M+H]⁺.

### Intermédiaire 18 : 6-Triméthylstannyl-N-(pyridin-2-yl)imidazo[1,2-α]pyridine-2-carboxamide

A une suspension de 300 mg de 6-iodo-*N*-(pyridin-2-yl)imidazo[1,2-*a*]pyridine-2-carboxamide dans 16 mL de tolène on ajoute 423 µL d'hexaméthyldistannane et 50 mg de tétrakis-(triphénylphosphine)palladium(0). Le mélange réactionnel est chauffé 2 heures au reflux puis agité 16 h à température ambiante et filtré. Le filtrat est concentré sous pression réduite et le résidu est chromatographié sur une cartouche de silice en éluant par du un mélange (1/1) de dichlorométhane et d'acétate d'éthyle. Les fractions contenant le produit attendu sont réunies et évaporées à sec sous pression réduite et le résidu concrété avec un peu de dichlorométhane, de pentane et d'éther éthylique pour donner 276 mg de 6-triméthylstannanyl-*N*-(pyridin-2-yl)imidazo[1,2-*a*]pyridine-2-carboxamide sous la forme d'un solide écru.

Spectre RMN ¹H (DMSO-d6, δ en ppm) : 0,37 (m, 9H), 7,19 (dd large, J = 5,0 et 8,0 Hz, 1H), 7,42 (d large, J = 9,5 Hz, 1H), 7,67 (d, J = 9,5 Hz, 1H), 7,89 (m, 1H), 8,24 (d, J = 8,0 Hz, 1H), 8,39 (d large, J = 5,0 Hz, 1H), 8,53 (m, 2H), 9,80 (s, 1H).

Spectre de masse (LC-MS-DAD-ELSD) : m/z 403, [M+H]⁺

### Intermédiaire 19 : Bromhydrate (1:1) de 6-(4,4,5,5-tétraméthyl-1,3,2-dioxaborolan-2-yl)imidazo[1,2-a]pyridine-2-carboxylate d'éthyle

A une solution de 4 g de 2-amino-5-(4,4,5,5-tétraméthyl-1,3,2-dioxaborolan-2-yl)pyridine dans 40 mL de 1,2-diméthoxyéthane on ajoute 4,26 g de 3-bromo-2-oxo-propionate d'éthyle. Le mélange réactionnel est agité 40 heures à 20°C. Le précipité est essoré, lavé par un peu de 1,2-diméthoxyéthane et du pentane puis repris dans 50 mL d'éthanol et chauffé au reflux pendant 1 heure. Le mélange réactionnel est concentré à sec sous pression réduite. L'huile obtenue est redissoute dans de l'éther éthylique et la solution concentrée sous pression réduite. Le solide est essoré et lavé par un peu d'éther éthylique pour donner 3,78 g de bromhydrate (1:1) de 6-(4,4,5,5-tétraméthyl-1,3,2-dioxaborotan-2-yl)imidazo[1,2-*a*]pyridine-2-carboxylate d'éthyle sous la forme d'un solide blanc.

Spectre RMN ¹H (DMSO-d6, δ en ppm) : 1,27 - 1,38 (m, 15H), 4,36 (q, J=7,3 Hz, 2H), 7,59 (d, J=9,3 Hz, 1H), 7,67 (d, J=9,3 Hz, 1H), 8,68 (s, 1H), 8,97 (s, 1H).

Spectre de masse (IE) : m/z 316 [M]⁺, 244 [M- CO₂Et+H]⁺.

### Intermédiaire 20 : Acide 2-éthoxycarbonyl-imidazo[1,2-a]pyridine-6-boronique

A une solution de 2,5 g de 2-amino-5-(4,4,5,5-tétraméthyl-1,3,2-dioxaborolan-2-yl)pyridine dans 50 mL de 1,2-diméthoxyéthane on ajoute 2,14 mL de 3-bromo-2-oxo-propionate d'éthyle. Le mélange réactionnel est agité 3,5 heures à 25°C puis on ajoute 50 mL d'éthanol et chauffe au reflux pendant 16 heures. Le mélange réactionnel est refroidi et concentré à sec. Le résidu est mis en suspension dans 100 mL d'eau à 0°C et traité en agitant vigoureusement par du carbonate de sodium solide jusqu'à atteindre un pH de 8-9. Le précipité est essoré et lavé par 100 mL d'eau à 0 °C puis dissout dans 150 mL de méthanol. La solution est séchée sur sulfate de magnésium, filtrée, concentrée et séchée sous vide pour donner 2,36 g d'acide 2-éthoxycarbonyl-imidazo[1,2-*a*]pyridine-6-boronique sous la forme d'un solide crème.

Spectre RMN ¹H (DMSO-d6, δ en ppm) : 8,82 (d, J = 0,9, 1H), 8,58 (s, 1H), 8,35 (s, 2H) 7,61 (m, 2H), 4,33 (m, 2H), 1,32 (m, 3H)

Spectre de masse (APCI): m/z= 235 [M+H]⁺.

Les tableaux qui suivent illustrent les structures chimiques (tableau 1) et les caractéristiques spectroscopiques (tableau 2) de quelques exemples de composés selon l'invention.

Dans ce tableaux,- « 2 HCl » signifie chlorhydrate (2:1); « TFA » signifie trifluoroacétate (1 :1 ) ; « - » signifie que le composé se présente sous forme base.

**Tableau 1**

| | | | | | | |
|---|---|---|---|---|---|---|
| 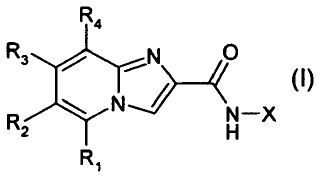 | | | | | | |

| **Ex** | **R₁** | **R₂** | **R₃** | **R₄** | **X** | **sel** |
|---|---|---|---|---|---|---|
| **1** | H | 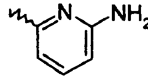 | H | H | 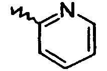 | 2 HCl |
| **2** | H | 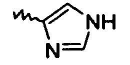 | H | H | 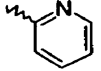 | - |
| **3** | H | 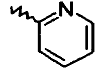 | H | H | 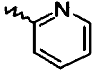 | - |
| **4** | H | 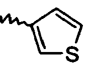 | H | H | 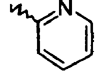 | - |
| **5** | H | 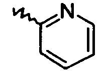 | H | H | 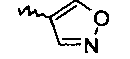 | - |
| **6** | H | 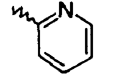 | H | H | 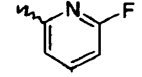 | - |
| **7** | H | 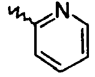 | H | H | 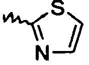 | - |
| **8** | H | 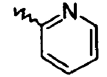 | H | H | 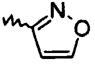 | - |
| **9** | H | 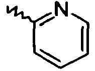 | H | H | 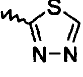 | - |
| **10** | H | 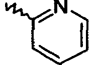 | H | H | 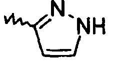 | - |
| **11** | H | 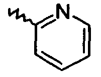 | H | H | 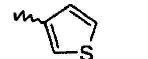 | - |
| **12** | H | 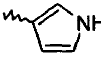 | H | H | 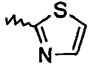 | - |
| **13** | H | 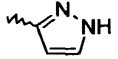 | H | H | 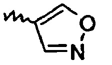 | - |
| **14** | H | 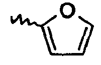 | H | H | 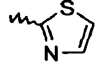 | - |
| **15** | H | 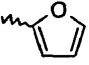 | H | H | 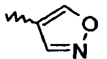 | - |
| **16** | H | 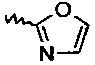 | H | H | 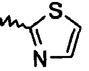 | - |
| 17 | H | 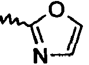 | H | H | 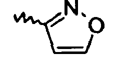 | - |
| **18** | H | 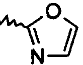 | H | H | 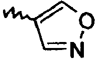 | - |
| **19** | H | 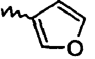 | H | H | 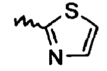 | - |
| **20** | H | 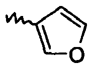 | H | H | 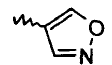 | - |
| **21** | H | 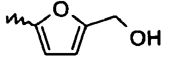 | H | H | 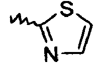 | - |
| **22** | H | 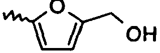 | H | H | 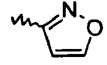 | - |
| **23** | H | 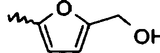 | H | H | 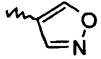 | - |
| **24** | H | 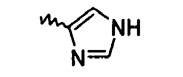 | H | H | 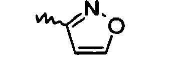 | - |
| **25** | H | 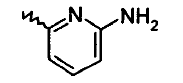 | H | H | 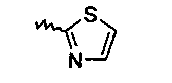 | - |
| **26** | H | 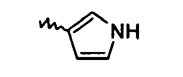 | H | H | 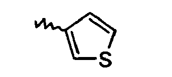 | - |
| **27** | H | 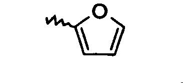 | H | H | 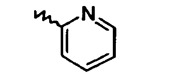 | - |
| **28** | H | 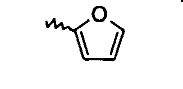 | H | H | 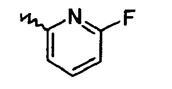 | - |
| **29** | H | 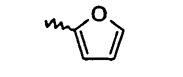 | H | H | 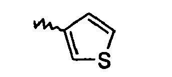 | - |
| **30** | H | 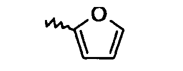 | H | H | 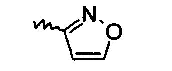 | - |
| **31** | H | 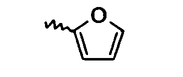 | H | H | 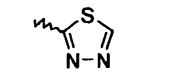 | - |
| **32** | H | 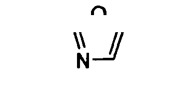 | H | H | 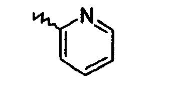 | - |
| **33** | H | 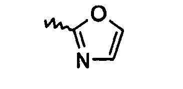 | H | H | 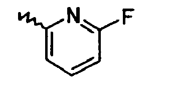 | - |
| **34** | H | 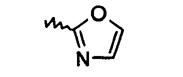 | H | H | 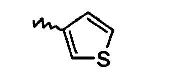 | - |
| **35** | H | 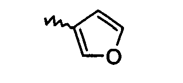 | H | H | 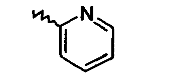 | - |
| **36** | H | 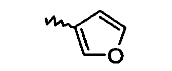 | H | H | 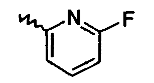 | - |
| **37** | H | 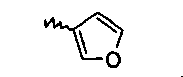 | H | H | 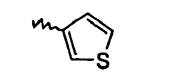 | - |
| **38** | H | 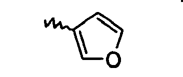 | H | H | 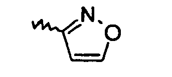 | - |
| **39** | H | 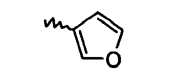 | H | H | 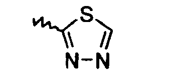 | - |
| **40** | H | 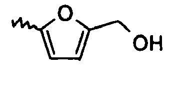 | H | H | 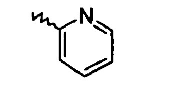 | - |
| **41** | H | 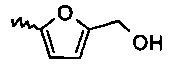 | H | H | 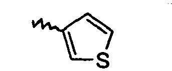 | - |
| **42** | H | 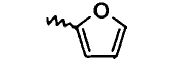 | H | H | 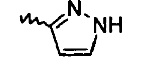 | - |
| **43** | H | 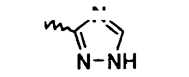 | H | H | 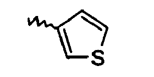 | - |
| **44** | H | 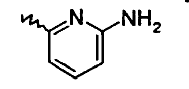 | H | H | 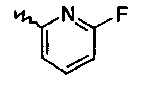 | 2 HCl |
| **45** | H | 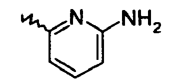 | H | H | 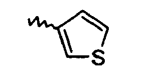 | 2 HCl |
| **46** | H | 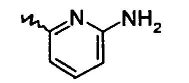 | H | H | 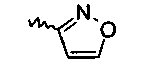 | 2 HCl |
| **47** | H | 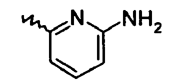 | H | H | 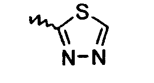 | 2 HCl |
| **48** | H | 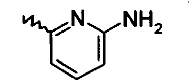 | H | H | 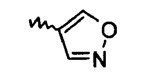 | 2 HCl |
| **49** | H | 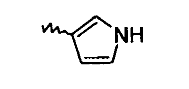 | H | H | 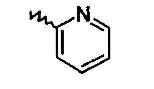 | TFA |
| **50** | H | 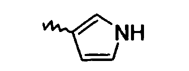 | H | H | 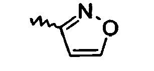 | - |
| **51** | H | 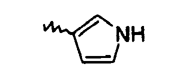 | H | H | 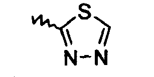 | - |
| **52** | H | 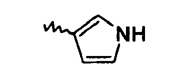 | H | H | 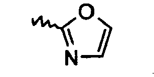 | - |
| **53** | H | 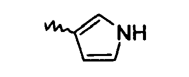 | H | H | 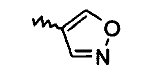 | - |
| **54** | H | 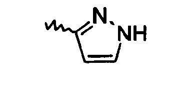 | H | H | 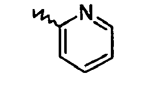 | - |
| **55** | H | 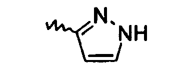 | H | H | 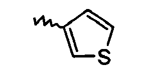 | TFA |
| **56** | H | 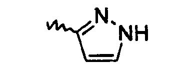 | H | H | 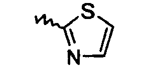 | TFA |
| **57** | H | 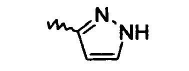 | H | H | 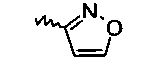 | - |
| **58** | H | 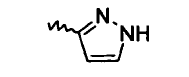 | H | H | 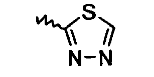 | TFA |
| **59** | H | 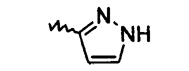 | H | H | 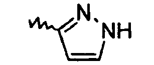 | - |
| **60** | H | 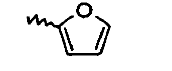 | H | H | 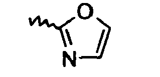 | TFA |
| **61** | H | 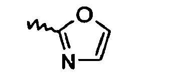 | H | H | 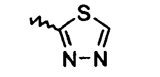 | - |
| **62** | H | 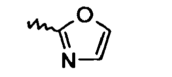 | H | H | 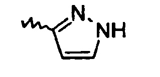 | - |
| **63** | H | 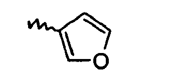 | H | H | 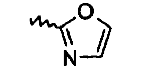 | TFA |
| **64** | H | 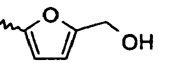 | H | H | 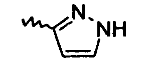 | - |
| **65** | H | 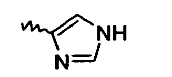 | H | H | 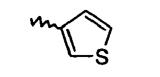 | - |
| **66** | H | 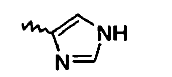 | H | H | 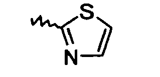 | - |
| **67** | H | 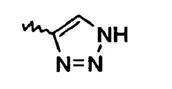 | H | H | 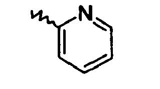 | - |
| **68** | H | 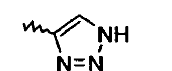 | H | H | 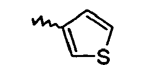 | - |

**Tableau 2**

| **Ex** | **Caractérisations** |
|---|---|
| **1** | Spectre RMN ¹H (DMSO-d6, δ en ppm) : 6,90 (m large, 1H), de 7,30 à 7,39 (m, 2H), de 7,84 à 8,00 (m, 4H), 8,25 (d, J = 8,0 Hz, 1H), 8,42 (d large, J = 5,0 Hz, 1H), 8,78 (s, 1H), 9,28 (s large, 1H), 10,25 (m étalé, 1H). Spectre de masse (LC-MS-DAD-ELSD) : m/z 331 [M+H]⁺ |
| **2** | Spectre RMN ¹H (DMSO-d6, δ en ppm) : 7,09 (m, 1H), de 7,64 à 7,95 (m, 5H), 8,26 (m, 1H), 8,39 (m, 1H), 8,65 (s, 1H), 9,01 (s, 1H), 9,79 (s, 1H), 12,3 (m étalé, 1H). Spectre de masse (LC-MS-DAD-ELSD) : m/z 305 [M+H]⁺, m/z 303 [M-H]⁻. |
| **3** | Spectre RMN ¹H (DMSO-d6, δ en ppm) : 7,20 (ddd, J=7,3, 4,9, 1,2 Hz, 1H), 7,43 (ddd, J=7,3, 4,8, 1,2 Hz, 1H), 7,81 (dt, J=9,6, 0,9 Hz, 1H), 7,89 (m, 1H), 7,96 (m, 1H), 8,02 (dt, J=8,1, 1,2 Hz, 1H), 8,14 (dd, J=9,6, 1,8 Hz, 1H), 8,26 (dt, J=8,1, 1,2 Hz, 1H), 8,39 (ddd, J=4,9, 2,0, 1,2 Hz, 1H), 8,71 (d, J=0,9 Hz, 1H), 8,73 (m, 1H), 9,43 (dd, J=1,8, 0,9 Hz, 1H), 9,83 (s, 1H). Spectre de masse (LC-MS-DAD-ELSD) : m/z 316 [M+H]⁺ |
| **4** | Spectre RMN ¹H (DMSO-d6, δ en ppm) : 7,19 (dd large, J=7,3, 4,9 Hz, 1H), 7,59 (dd, J=5,9, 1,0 Hz, 1H), 7,72 - 7,79 (m, 2H), 7,82 - 7,92 (m, 2H), 8,01 (d large, J=3,4 Hz, 1H), 8,25 (d large, J=8,5 Hz, 1H), 8,39 (d large, J=4,9 Hz, 1H), 8,55 (s, 1H), 9,05 (s large, 1H), 9,82 (s, 1H). Spectre de masse (LC-MS-DAD-ELSD) : m/z 321 [M+H]⁺. |
| **5** | Spectre RMN ¹H (DMSO-d6, δ en ppm) : 7,43 (ddd, J=7,6, 4,8, 1,3 Hz, 1H), 7,75 (dt, J=9,6, 1,0 Hz, 1H), 7,96 (td, J=7,6, 1,9 Hz, 1H), 8,02 (dt, J=7,6, 1,3 Hz, 1H), 8,12 (dd, J=9,6, 1,9 Hz, 1H), 8,62 (d, J=0,8 Hz, 1H), 8,71 (ddd, J=4,8, 1,9, 1,3 Hz, 1H), 8,84 (s, 1H), 9,25 (s, 1H), 9,43 (dd, J=1,9, 1,0 Hz, 1H), 10,94 (s, 1H). Spectre de masse (LC-MS-DAD-ELSD) : m/z 306 [M+H]⁺, m/z 350 [M+HCO₂H-H]⁻. |
| **6** | Spectre RMN ¹H (DMSO-d6, δ en ppm) : 6,95 (dd, J=8,0, 2,5 Hz, 1H), 7,43 (m, 1H), 7,80 (d, J=9,6 Hz, 1H), 7,88 - 8,16 (m, 5H), 8,68 (s, 1H), 8,70 (d large, J=5,0 Hz, 1H), 9,35 (s large, 1H), 9,80 (s large, 1H). Spectre de masse (LC-MS-DAD-ELSD) : m/z 334 [M+H]⁺, m/z 378 [M+HCO₂H-H]⁻. |
| **7** | Spectre RMN ¹H (DMSO-d6, δ en ppm) : 7,30 (d, J=3,6 Hz, 1H), 7,43 (m, 1H), 7,55 (d, J=3,6 Hz, 1H), 7,79 (d, J=9,8 Hz, 1H), 7,97 (td, J=7,7, 1,2 Hz, 1H), 8,03 (d large, J=7,7 Hz, 1H), 8,14 (dd, J=9,8, 1,8 Hz, 1H), 8,72 (d large, J=4,9 Hz, 1H), 8,78 (s, 1H), 9,44 (s large, 1H), 11,91 (s large, 1H). Spectre de masse (LC-MS-DAD-ELSD) : m/z 322 [M+H]⁺. |
| **8** | Spectre RMN ¹H (DMSO-d6, δ en ppm) : 7,01 (d, J=1,7 Hz, 1H), 7,43 (ddd, J=7,5, 4,8, 1,3 Hz, 1H), 7,77 (dt, J=9,6, 1,0 Hz, 1H), 7,96 (td, J=7,5, 1,8 Hz, 1H), 8,03 (dt, J=7,5, 1,3 Hz, 1H), 8,13 (dd, J=9,6, 1,9 Hz, 1H), 8,70 - 8,73 (m, 2H), 8,84 (d, J=1,7 Hz, 1H), 9,42 (dd, J=1,9, 0,9 Hz, 1H), 10,95 (s, 1H). Spectre de masse (LC-MS-DAD-ELSD) : m/z 306 [M+H]⁺. |
| **9** | Spectre RMN ¹H (DMSO-d6, δ en ppm) : 7,41 (m, 1H), 7,77 (d, J=9,5 Hz, 1H), 7,87 - 8,02 (m, 2H), 8,11 (dd, J=9,5, 1,7 Hz, 1H), 8,70 (d large, J=4,9 Hz, 1H), 8,78 (s, 1H), 9,18 (s, 1H), 9,36 (s large, 1H), 11,90 (m étalé, 1H). Spectre de masse (LC-MS-DAD-ELSD) : m/z 323 [M+H]⁺. |
| **10** | Spectre RMN ¹H (DMSO-d6, δ en ppm) : 6,64 (s large, 1H), 7,42 (ddd, J=7,6, 4,8, 1,3 Hz, 1H), 7,68 (s large, 1H), 7,77 (d, J=9,5 Hz, 1H), 7,96 (td, J=7,6, 1,9 Hz, 1H), 8,02 (d large, J=7,6 Hz, 1H), 8,11 (dd, J=9,5, 1,8 Hz, 1H), 8,62 (s, 1H), 8,71 (d large, J=4,8 Hz, 1H), 9,42 (s large, 1H), 9,90 (s, 1H), 12,46 (s large, 1H). Spectre de masse (LC-MS-DAD-ELSD) : m/z 305 [M+H]⁺. |
| **11** | Spectre RMN ¹H (DMSO-d6, δ en ppm) : 7,39 - 7,49 (m, 2H), 7,52 (dd, J=5,1, 1,4 Hz, 1H), 7,75 (d, J=9,6 Hz, 1H), 7,80 (dd, J=3,2, 1,4 Hz, 1H), 7,96 (td, J=7,9, 1,9 Hz, 1H), 8,02 (d large, J=7,9 Hz, 1H), 8,11 (dd, J=9,6, 1,9 Hz, 1H), 8,60 (s, 1H), 8,71 (d large, J=5,1 Hz, 1H), 9,42 (s large, 1H), 10,83 (s large, 1H). Spectre de masse (LC-MS-DAD-ELSD) : m/z 321 [M+H]⁺. |
| **12** | Spectre RMN ¹H (DMSO-d6, δ en ppm) : 6,49 (q, J=2,5 Hz, 1H), 6,87 (dd, J=2,5, 1,9 Hz, 1H), 7,29 (d, J=3,6 Hz, 1H), 7,33 (dd, J=2,5, 1,9 Hz, 1H), 7,54 (d, J=3,6 Hz, 1H), 7,62 (d large, J=9,6 Hz, 1H), 7,70 (dd, J=9,6, 1,9 Hz, 1H), 8,56 (s, 1H), 8,77 (s large, 1H), 11,06 (s large, 1H), 11,75 (s large, 1 H). Spectre de masse (LC-MS-DAD-ELSD) : m/z 310 [M+H]⁺, m/z 308 [M-H]⁻. |
| **13** | Spectre RMN ¹H (DMSO-d6, δ en ppm) : (tous les signaux sont larges) 6,71 (s, 1H), 7,66 (d, J=9,5 Hz, 1H), 7,78 (s, 1H), 7,85 (d, J=9,5 Hz, 1H), 8,49 (s, 1H), 8,82 (s, 1H), 9,02 (s, 1H), 9,16 (s, 1H), 10,60 (s, 1H), 12,86 (m étalé, 1H). Spectre de masse (LC-MS-DAD-ELSD) : m/z 295 [M+H]⁺, m/z 293 [M-H]⁻. |
| **14** | Spectre RMN ¹H (DMSO-d6, δ en ppm) : 6,66 (dd, J=3,4, 1,9 Hz, 1H), 7,07 (dd, J=3,4, 0,9 Hz, 1H), 7,30 (d, J=3,6 Hz, 1H), 7,55 (d, J=3,6 Hz, 1H), 7,74 (d large, J=9,6 Hz, 1H), 7,78 (dd, J=9,6, 1,7 Hz, 1H), 7,83 (dd, J=1,9, 0,9 Hz, 1H), 8,72 (s large, 1H), 9,00 (s large, 1H), 11,86 (s large, 1H). Spectre de masse (LC-MS-DAD-ELSD) : m/z 311 [M+H]⁺. |
| **15** | Spectre RMN ¹H (DMSO-d6, δ en ppm) : (tous les signaux sont larges) 6,62 (m, 1H), 6,96 (d, J=2,8 Hz, 1H), 7,66 (d, J=9,6 Hz, 1H), 7,70 (d, J=9,6 Hz, 1H), 7,76 (m, 1H), 8,51 (s, 1H), 8,82 (s, 1H), 8,94 (s, 1H), 9,15 (s, 1H), 10,59 (s, 1H). Spectre de masse (LC-MS-DAD-ELSD) : m/z 295 [M+H]⁺, m/z 293 [M-H]⁻. |
| **16** | Spectre RMN ¹H (DMSO-d6, δ en ppm) : 7,31 (d, J=3,6 Hz, 1H), 7,46 (d, J=0,9 Hz, 1H), 7,55 (d, J=3,6 Hz, 1H), 7,83 (dt, J=9,6, 0,9 Hz, 1H), 7,92 (dd, J=9,6, 1,7 Hz, 1H), 8,32 (d, J=0,9 Hz, 1H), 8,82 (d, J=0,9 Hz, 1H), 9,40 (dd, J=1,7, 0,9 Hz, 1H), 11,99 (s large, 1H). Spectre de masse (LC-MS-DAD-ELSD) : m/z 312 [M+H]⁺. |
| **17** | Spectre RMN ¹H (DMSO-d6, δ en ppm) : 7,00 (d, J=1,9 Hz, 1H), 7,46 (d, J=0,9 Hz, 1H), 7,81 (dt, J=9,6, 0,9 Hz, 1H), 7,92 (dd, J=9,6, 1,9 Hz, 1H), 8,31 (d, J=0,9 Hz, 1H), 8,74 (d, J=0,9 Hz, 1H), 8,84 (d, J=1,9 Hz, 1H), 9,38 (dd, J=1,9, 0,9 Hz, 1H), 11,02 (s, 1H). Spectre de masse (LC-MS-DAD-ELSD) : m/z 296 [M+H]⁺. |
| **18** | Spectre RMN ¹H (DMSO-d6, δ en ppm) : 7,45 (d, J=1,0 Hz, 1H), 7,79 (dt, J=9,6, 0,9 Hz, 1H), 7,92 (dd, J=9,6, 1,9 Hz, 1H), 8,31 (d, J=1,0 Hz, 1H), 8,68 (d, J=0,9 Hz, 1H), 8,83 (s, 1H), 9,26 (s, 1H), 9,38 (dd, J=1,9, 0,9 Hz, 1H), 10,97 (s, 1H). Spectre de masse (LC-MS-DAD-ELSD) : m/z 296 [M+H]⁺. |
| **19** | Spectre RMN ¹H (DMSO-d6, δ en ppm) : 7,00 (dd, J=1,8, 0,9 Hz, 1 H), 7,29 (d, J=3,6 Hz, 1H), 7,55 (d, J=3,6 Hz, 1H), 7,72 (d, J=1,4 Hz, 2H), 7,82 (t, J=1,8 Hz, 1H), 8,31 (dd, J=1,8, 0,9 Hz, 1H), 8,61 (s, 1H), 8,94 (t, J=1,4 Hz, 1H), 11,87 (m étalé, 1H). Spectre de masse (LC-MS-DAD-ELSD) : m/z 311 [M+H]⁺. |
| **20** | Spectre RMN ¹H (DMSO-d6, δ en ppm) : 6,97 (dd, J=1,8, 0,9 Hz, 1H), 7,61 - 7,74 (m, 2H), 7,82 (t, J=1,8 Hz, 1H), 8,29 (dd, J=1,8, 0,9 Hz, 1H), 8,43 (s, 1H), 8,83 (s, 1H), 8,93 (t, J=1,4 Hz, 1H), 9,24 (s, 1H), 10,89 (s, 1H). Spectre de masse (LC-MS-DAD-ELSD) : m/z 295 [M+H]⁺, m/z 293 [M-H]⁻. |
| **21** | Spectre RMN ¹H (DMSO-d6, δ en ppm) : 4,49 (d, J=5,6 Hz, 2H), 5,31 (t, J=5,6 Hz, 1H), 6,47 (d, J=3,4 Hz, 1H), 6,99 (d, J=3,4 Hz, 1H), 7,30 (d, J=3,6 Hz, 1H), 7,55 (d, J=3,6 Hz, 1H), 7,69 - 7,83 (m, 2H), 8,73 (s, 1H), 8,96 (s large, 1H), 11,84 (m étalé, 1H). Spectre de masse (LC-MS-DAD-ELSD) : m/z 341 [M+H]⁺, m/z 339 [M-H]⁻. |
| **22** | Spectre RMN ¹H (DMSO-d6, δ en ppm) : 4,49 (d large, J=5,6 Hz, 2H), 5,05 étalé (m, 1H), 6,43 (d, J=3,3 Hz, 1H), 6,91 (d, J=3,3 Hz, 1H), 6,99 (s large, 1H), 7,69 (m, 2H), 8,61 (s, 1H), 8,76 (s large, 1H), 8,90 (s large, 1H), 10,49 (m étalé, 1H). Spectre de masse (LC-MS-DAD-ELSD) : m/z 325 [M+H]⁺. |
| **23** | Spectre RMN ¹H (DMSO-d6, δ en ppm) : 4,48 (d, J=5,6 Hz, 2H), 5,30 (t, J=5,6 Hz, 1H), 6,46 (d, J=3,3 Hz, 1H), 6,97 (d, J=3,3 Hz, 1H), 7,68 (dt, J=9,5, 1,0 Hz, 1H), 7,74 (dd, J=9,5, 1,7 Hz, 1H), 8,59 (d, J=1,0 Hz, 1H), 8,83 (s, 1H), 8,96 (s large, 1H), 9,24 (s, 1 H), 10,89 (s large, 1 H). Spectre de masse (LC-MS-DAD-ELSD) : m/z 325 [M+H]⁺, m/z 323 [M-H]⁻. |
| **24** | Spectre RMN ¹H (DMSO-d6, δ en ppm) : 7,01 (d, J=1,7 Hz, 1H), 7,66 (dt, J=9,5, 0,9 Hz, 1H), 7,72 (dd, J=1,9, 1,0 Hz, 1H), 7,78 (t, J=1,0 Hz, 1H), 7,82 (dd, J=9,5, 1,7 Hz, 1H), 8,64 (d, J=0,9 Hz, 1H), 8,83 (d, J=1,7 Hz, 1H), 8,99 (dd, J=1,7, 0,9 Hz, 1H), 10,83 (s large, 1 H), 12,29 (m étalé, 1H). Spectre de masse (LC-MS-DAD-ELSD) : m/z 295 [M+H]⁺, m/z 293 [M-H]⁻. |
| **25** | Spectre RMN ¹H (DMSO-d6, δ en ppm) : 7,01 (dd, J=8,8, 1,0 Hz, 1H), 7,28 (dd, J=7,4, 1,0 Hz, 1H), 7,33 (d, J=3,6 Hz, 1H), 7,58 (d, J=3,6 Hz, 1H), 7,92 (dt, J=9,5, 1,0 Hz, 1H), 7,98 (dd, J=8,8, 7,4 Hz, 1H), 8,06 (dd, J=9,5, 1,7 Hz, 1H), 8,10 (m très étalé, 2H), 8,87 (d, J=1,0 Hz, 1H), 9,47 (dd, J=2,0, 1,0 Hz, 1H), 10,67 (m étalé, 1H). Spectre de masse (LC-MS-DAD-ELSD) : m/z 337 [M+H]⁺, m/z 335 [M-H]⁺. |
| **26** | Spectre RMN ¹H (DMSO-d6, δ en ppm) : 6,47 (m, 1H), 6,87 (m, 1H), 7,30 (m, 1H), 7,45 (dd, J=5,1, 3,1 Hz, 1H), 7,50 (dd, J=5,1, 1,4 Hz, 1H), 7,58 (d, J=9,5 Hz, 1H), 7,66 (dd, J=9,5, 1,7 Hz, 1H), 7,78 (dd, J=3,1, 1,4 Hz, 1H), 8,38 (s, 1H), 8,76 (s large, 1H), 10,71 (s large, 1H), 11,04 (m étalé, 1H) Spectre de masse (LC-MS-ES+/-) : m/z 307 [M+H]⁻, m/z 309 [M+H]⁺ |
| **27** | Spectre RMN ¹H (DMSO-d6, δ en ppm) : 6,66 (dd, J=3,4, 1,9 Hz, 1H), 7,07 (d, J=3,4 Hz, 1H), 7,19 (ddd, J=7,4, 4,9, 0,9 Hz, 1H), 7,73-7,92 (m, 4H), 8,25 (d, J=8,4 Hz, 1H), 8,38 (ddd, J=4,9, 1,9, 0,9 Hz, 1H), 8,65 (s, 1H), 8,99 (s large, 1H), 9,79 (s large, 1H) Spectre de masse (LC-MS-ES+/-) : m/z 305 [M+H]⁺ |
| **28** | Spectre RMN ¹H (DMSO-d6, δ en ppm) : 6,66 (dd, J=3,4, 1,9 Hz, 1H), 6,94 (ddd, J=7,8, 2,5, 0,8 Hz, 1H), 7,07 (dd, J=3,4, 0,8 Hz, 1H), 7,75 (d, J=9,5 Hz, 1H), 7,80 (dd, J=9,5, 1,7 Hz, 1H), 7,83 (dd, J=1,9, 0,8 Hz, 1H), 8,06 (q, J=8,1 Hz, 1H), 8,14 (ddd, J=8,1, 2,5, 0,8 Hz, 1H), 8,67 (s, 1H), 8,98 (s large, 1H), 9,89 (s large, 1H) Spectre de masse (LC-MS-ES+/-) : m/z 323 [M+H]⁺ |
| **29** | Spectre RMN ¹H (DMSO-d6, δ en ppm): 3,65 (dd, J=3,5, 1,9 Hz, 1H), 7,04 (dd, J=3,5, 0,8 Hz, 1H), 7,46 (dd, J=5,1, 3,3 Hz, 1H), 7,51 (dd, J=5,1, 1,4 Hz, 1H), 7,69 (d, J=9,6 Hz, 1H), 7,75 (dd, J=9,6, 1,7 Hz, 1H), 7,79 (dd, J=3,3, 1,4 Hz, 1H), 7,82 (dd, J=1,9, 0,8 Hz, 1H), 8,55 (s, 1H), 8,99 (s large, 1H), 10,78 (s large, 1H) Spectre de masse (LC-MS-ES+/-) : m/z 310 [M+H]⁺ |
| **30** | Spectre RMN ¹H (DMSO-d6, δ en ppm) : 6,66 (dd, J=3,4, 1,9 Hz, 1H), 7,01 (d, J=1,7 Hz, 1H), 7,06 (dd, J=3,4, 0,8 Hz, 1H), 7,72 (d, J=9,5 Hz, 1H), 7,77 (dd, J=9,5, 1,7 Hz, 1H), 7,83 (dd, J=1,9, 0,8 Hz, 1H), 8,64 (s, 1H), 8,83 (d, J=1,7 Hz, 1H), 8,99 (s large, 1H), 10,90 (s large, 1H) Spectre de masse (LC-MS-ES+/-) : m/z 295 [M+H]⁺ |
| **31** | Spectre RMN ¹H (DMSO-d6, δ en ppm) : 6,66 (dd, J=3,4, 1,9 Hz, 1H), 7,08 (d, J=3,4 Hz 1H), 7,69-7,86 (m, 3H), 8,77 (s, 1H), 9,01 (s large, 1H), 9,24 (s large, 1H), 12,47-12,80 (m étalé, 1 H) Spectre de masse (LC-MS-ES+/-) : m/z 310 [M+H]⁺, m/z 312 [M+H]⁺ |
| **32** | Spectre RMN ¹H (DMSO-d6, δ en ppm) : 7,17-7,23 (m, 1H), 7,44-7,47 (m, 1H), 7,82-7,96 (m, 3H), 8,24 (d, J=8,3 Hz, 1H), 8,30-8,32 (m, 1H), 8,37-8,41 (m, 1H), 8,75 (s, 1H), 9,37 (s large, 1 H), 9,83 (s large, 1H) Spectre de masse (LC-MS-ES+/-) : m/z 306 [M+H]⁺ |
| **33** | Spectre RMN ¹H (DMSO-d6, δ en ppm) : 6,95 (dd, J=7,9, 2,3 Hz, 1H), 7,44-7,47 (m, 1H), 7,84 (d, J=9,5 Hz, 1H), 7,93 (dd, J=9,5, 1,7 Hz, 1H), 8,01-8,17 (m, 2H), 8,3-8,33 (m, 1H), 8,77 (s, 1H), 9,37 (s large, 1H), 9,95 (s, 1H) Spectre de masse (LC-MS-ES+/-) : m/z 324 [M+H]⁺ |
| **34** | Spectre RMN ¹H (DMSO-d6, δ en ppm) : 7,44-7,53 (m, 3H), 7,76-7,82 (m, 2H), 7,9 (dd, J=9,5, 1,7 Hz, 1H), 8,30-8,31 (m, 1H), 8,65 (s, 1H), 9,38 (s large, 1H), 10,85 (s large, 1 H) Spectre de masse (LC-MS-ES+/-) : m/z 311 [M+H]⁺ |
| **35** | Spectre RMN ¹H (DMSO-d6, δ en ppm) : 6,98 (dd, J=1,9, 0,9 Hz, 1H), 7,19 (ddd, J=7,3, 4,9, 1,1 Hz, 1 H), 7,73-7,74 (m, 2H), 7,82 (t, J=1,9 Hz, 1H), 7,89 (m, 1H), 8,25 (dt, J=8,4, 0,9 Hz, 1H), 8,3 (s large, 1H), 8,39 (ddd, J=4,9, 1,9, 0,9 Hz, 1H), 8,52 (s, 1 H), 8,92 (s large, 1H), 9,8 (s, 1H) Spectre de masse (LC-MS-ES+/-) : m/z 305 [M+H]⁺ |
| **36** | Spectre RMN ¹H (DMSO-d6, δ en ppm) : 6,94 (ddd, J=8,1, 2,6, 0,9 Hz, 1H), 6,98 (dd, J=1,9, 0,9 Hz, 1H), 7,74 (m, 2H), 7,82 (t, J=1,9 Hz, 1H), 8,06 (q, J=8,1 Hz, 1H), 8,15 (ddd, J=8,1, 2,6, 0,9 Hz, 1H), 8,3 (s large, 1H), 8,55 (s, 1H), 8,92 (s large, 1H), 9,91 (s large, 1H) Spectre de masse (LC-MS-ES+/-) : m/z 323 [M+H]⁺ |
| **37** | Spectre RMN ¹H (DMSO-d6, δ en ppm) : 6,98 (dd, J=1,8, 0,9 Hz, 1H), 7,46 (dd, J=5,3, 3,3 Hz, 1H), 7,5 (dd, J=5,3, 1,4 Hz, 1H), 7,68 (m, 2H), 7,78 (dd, J=3,3, 1,4 Hz, 1H), 7,82 (t, J=1,8 Hz, 1H), 8,29 (s large, 1H), 8,42 (s, 1H), 8,93 (s large, 1H), 10,84 (s large, 1H) Spectre de masse (LC-MS-ES+/-) : m/z 310 [M+H]⁺ |
| **38** | Spectre RMN ¹H (DMSO-d6, δ en ppm) : 6,99 (dd, J=1,9, 0,9 Hz, 1H), 7,01 (d, J=1,9 Hz, 1H), 7,7 (m, 2H), 7,82 (t, J=1,9 Hz, 1H), 8,30 (s large, 1H), 8,52 (s, 1H), 8,83 (d, J=1,9 Hz, 1H), 8,93 (s large, 1H), 10,9 (s large, 1H) Spectre de masse (LC-MS-ES+/-) : m/z 295 [M+H]⁺ |
| **39** | Spectre RMN ¹H (DMSO-d6, δ en ppm) : 7,00 (dd, J=1,9, 0,9 Hz, 1H), 7,73 (m, 2H), 7,82 (t, J=1,9 Hz, 1H), 8,31 (s large, 1H), 8,67 (s, 1H), 8,95 (s large, 1H), 9,23 (s, 1H), 12,58 (m étalé, 1 H) Spectre de masse (LC-MS-ES+/-) : m/z 310 [M+H]⁻, m/z 312 [M+H]⁺ |
| **40** | Spectre RMN ¹H (DMSO-d6, δ en ppm) : 4,49 (d, J=5,6 Hz, 2H), 5,30 (t, J=5,6 Hz, 1H), 6,47 (d, J=3,3 Hz, 1H), 6,99 (d, J=3,3 Hz, 1H), 7,14-7,24 (m, 1H), 7,76 (s, 2H), 7,83-7,95 (m, 1H), 8,23 (d, J=8,2 Hz, 1H), 8,34-8,43 (m, 1H), 8,66 (s, 1H), 8,95 (s large, 1H), 9,79 (s large, 1H) Spectre de masse (LC-MS-ES+/-) : m/z 335 [M+H]⁺ |
| **41** | Spectre RMN ¹H (DMSO-d6, δ en ppm) : 4,48 (d, J=5,6 Hz, 2H), 5,30 (t, J=5,6 Hz, 1H), 6,46 (d, J=3,5 Hz, 1H), 6,97 (d, J=3,5 Hz, 1H), 7,46 (dd, J=5,6, 3,2 Hz, 1H), 7,50 (dd, J=5,6, 1,5 Hz, 1H), 7,65-7,75 (m, 2H), 7,78 (dd, J=3,2, 1,5 Hz, 1H), 8,58 (s, 1H), 8,95 (s large, 1 H), 10,78 (s large, 1 H) Spectre de masse (LC-MS-ES+/-) : m/z 340 [M+H]⁺ |
| **42** | Spectre RMN ¹H (DMSO-d6, δ en ppm) : 6,61 (m étalé, 1H), 6,66 (dd, J=3,5, 1,2 Hz, 1H), 7,05 (d large, J=3,5 Hz, 1H), 7,66 (m étalé, 1H), 7,69-7,80 (m, 2H), 7,82 (d large, J=1,2 Hz, 1H), 8,56 (s, 1H), 8,98 (s large, 1H), 9,89 (m étalé, 1H), 12,47 (m étalé, 1H) Spectre de masse (LC-MS-ES+/-) : m/z 292 [M+H]⁻, m/z 294 [M+H]⁺ |
| **43** | Spectre RMN ¹H (DMSO-d6, δ en ppm) : 7,46 (dd, J=5,2, 3,2 Hz, 1H), 7,52 (dd, J=5,2, 1,3 Hz, 1H), 7,75 (d, J=9,5 Hz, 1H), 7,79 (dd, J=3,2, 1,3 Hz, 1H), 7,97 (dd, J=9,5, 1,6 Hz, 1H), 8,65 (s, 1H), 8,69 (m étalé, 1H), 9,31 (s large, 1H), 10,81 (s large, 1H), 14,27 (m étalé, 1H) Spectre de masse (LC-MS-ES+/-) : m/z 309 [M+H]⁻, m/z 311 [M+H]⁺ |
| **44** | Spectre RMN ¹H (DMSO-d6, δ en ppm) : 6,82-6,89 (m, 1H), 6,93-6,99 (m, 1H), 7,21 (d, J=7,1 Hz, 1H), 7,50 (m étalé, 2H), 7,82-7,98 (m, 3H), 8,02-8,18 (m, 2H), 8,78 (s, 1H), 9,26 (s, 1H), 10,13 (s large, 1H) Spectre de masse (LC-MS-ES+/-) : m/z 349 [M+H]⁺ |
| **45** | Spectre RMN ¹H (DMSO-d6, δ en ppm) : 6,98 (d, J=8,6 Hz, 1H), 7,25 (d, J=7,3 Hz, 1H), 7,45-7,54 (m, 2H), 7,76 (m étalé, 2H), 7,77-7,90 (m, 2H), 7,90-8,04 (m, 2H), 8,66 (s, 1H), 9,35 (s large, 1H), 11,01 (s large, 1H) Spectre de masse (LC-MS-ES+/-) : m/z 336 [M+H]⁺ |
| **46** | Spectre RMN ¹H (DMSO-d6, δ en ppm) : 6,98 (d, J=8,7 Hz, 1H), 7,02 (dd, J=1,6 Hz, 1H), 7,26 (d, J=7,3 Hz, 1H), 7,88 (d, J=9,5 Hz, 1H), 7,93-8,01 (m, 2H), 8,14 (m étalé, 3 H ), 8,77 (s, 1H), 8,86 (d, J=1,6 Hz, 1H), 9,38 (s large, 1H), 11,23 (s, 1H) Spectre de masse (LC-MS-ES+/-) : m/z 321 [M+H]⁺, m/z = 365 [M+HCO₂H-H]⁻ |
| **47** | Spectre RMN ¹H (DMSO-d6, δ en ppm) : 7,01 (d large, J=8,6 Hz, 1H), 7,28 (d large, J=7,6 Hz, 1H), 7,84-8,04 (m, 3H), 8,20 (m étalé, 3H), 8,90 (s, 1H), 9,25 (s, 1H), 9,44 (s large, 1H) Spectre de masse (LC-MS-ES+/-) : m/z 336 [M+H]⁻, m/z 338 [M+H]⁺ |
| **48** | Spectre RMN ¹H (DMSO-d6, δ en ppm) : 6,98 (d, J=8,8 Hz, 1H), 7,24 (d, J=7,3 Hz, 1H), 7,84 (d, J=9,5 Hz, 1H), 7,88-8,02 (m, 2H), 8,08 (m étalé, 3 H), 8,63 (s, 1H), 8,84 (s, 1H), 9,26 (s, 1H), 9,32 (s, 1H), 11,05 (s, 1H) (signaux larges) Spectre de masse (LC-MS-ES+/-) : m/z 321 [M+H]⁺, m/z = 365 [M+HCO₂H-H]⁻ |
| **49** | Spectre RMN ¹H (DMSO-d6, δ en ppm) : 6,51 (m, 1H), 6,89 (m, 1H), 7,23 (ddd, J=7,4, 5,0, 1,1 Hz, 1H), 7,37 (m, 1H), 7,69 (d, J=9,5 Hz, 1H), 7,86 (dd, J=9,5, 1,7 Hz, 1H), 7,93 (ddd, J=8,4, 7,4, 2,0 Hz, 1H), 8,24 (dt, J=8,4, 1,1 Hz, 1H), 8,41 (ddd, J=5,0, 2,0, 1,1 Hz, 1H), 8,65 (s, 1H), 8,87 (s large, 1H), 10,42 (m étalé, 1H), 11,12 (m étalé, 1H) Spectre de masse (LC-MS-ES+/-) : m/z 302 [M+H]⁻, m/z 304 [M+H]⁺ |
| **50** | Spectre RMN ¹H (DMSO-d6, δ en ppm) : 6,47 (m, 1H), 6,87 (m, 1H), 7,01 (d, J=1,6 Hz, 1H), 7,31 (m, 1H), 7,61 (d, J=9,5 Hz, 1H), 7,69 (dd, J=9,5, 1,7 Hz, 1H), 8,48 (s, 1H), 8,76 (s large, 1H), 8,83 (d, J=1,6 Hz, 1H), 10,81 (s large, 1H), 11,05 (m étalé, 1H) Spectre de masse (LC-MS-ES+/-) : m/z 292 [M+H]⁻, m/z 294 [M+H]⁺ |
| **51** | Spectre RMN ¹H (DMSO-d6, δ en ppm) : 6,49 (m, 1H), 6,87 (m, 1H), 7,33 (m, 1H), 7,63 (d, J=9,5 Hz, 1H), 7,72 (dd, J=9,5, 1,7 Hz, 1H), 8,62 (s, 1H), 8,78 (s large, 1H), 9,22 (s, 1H), 11,06 (m étalé, 1H), 12,49 (m très étalé, 1H) Spectre de masse (LC-MS-ES+/-) : m/z 309 [M+H]⁻, m/z 311 [M+H]⁺ |
| **52** | Spectre RMN ¹H (DMSO-d6, δ en ppm) : 6,47 (m, 1H), 6,87 (m, 1H), 7,17 (s, 1H), 7,32 (m, 1H), 7,59 (d, J=9,5 Hz, 1H), 7,69 (dd, J=9,5, 1,2 Hz, 1H), 7,95 (s, 1H), 8,46 (s, 1H), 8,75 (s, 1H), 10,95-11,16 (m, 2H) (signaux larges) Spectre de masse (LC-MS-ES+/-) : m/z 292 [M+H]⁻, m/z 294 [M+H]⁺ |
| **53** | Spectre RMN ¹H (DMSO-d6, δ en ppm) : 6,46 (m, 1H), 6,87 (m, 1H), 7,30 (m, 1H), 7,58 (d, J=9,7 Hz, 1H), 7,68 (dd, J=9,7, 1,7 Hz, 1H), 8,40 (s, 1H), 8,77 (s large, 1H), 8,83 (s, 1H), 9,23 (s, 1H), 10,83 (s large, 1H), 11,04 (m étalé, 1H) Spectre de masse (LC-MS-ES+/-) : m/z 292 [M+H]⁻, m/z 294 [M+H]⁺ |
| **54** | Spectre RMN ¹H (DMSO-d6, δ en ppm) : 6,77 (d, J=2,3 Hz, 1H), 7,19 (m, 1H), 7,75 (d, J=9,5 Hz, 1H), 7,79-7,99 (m, 3H), 8,25 (d, J=8,2 Hz, 1H), 8,39 (m, 1H), 8,61 (s, 1H), 9,09 (s large, 1H), 9,81 (s large, 1H), 13,06 (m étalé, 1H) Spectre de masse (LC-MS-ES+/-) : m/z 303 [M+H]⁻, m/z 305 [M+H]⁺ |
| **55** | Spectre RMN ¹H (DMSO-d6, δ en ppm) : 6,77 (d, J=2,3 Hz, 1H), 7,46 (dd, J=5,2, 3,2 Hz, 1H), 7,50 (dd, J=5,2, 1,4 Hz, 1H), 7,70 (d, J=9,5 Hz, 1H), 7,79 (dd, J=3,2, 1,4 Hz, 1H), 7,84 (d, J=2,3 Hz, 1H), 7,90 (dd, J=9,5, 1,7 Hz, 1H), 8,52 (s, 1H), 9,10 (s large, 1H), 10,81 (s, 1H), 13,03 (m étalé, 1H) Spectre de masse (LC-MS-ES+/-) : m/z 308 [M+H]⁻, m/z 310 [M+H]⁺ |
| **56** | Spectre RMN ¹H (DMSO-d6, δ en ppm) : 6,81 (d, J=2,3 Hz, 1H), 7,31 (d, J=3,7 Hz, 1H), 7,57 (d, J=3,7 Hz, 1H), 7,78 (d, J=9,6 Hz, 1H), 7,86 (d, J=2,3 Hz, 1H), 8,02 (dd, J=9,6, 1,7 Hz, 1H), 8,74 (s, 1H), 9,18 (s large, 1H), 11,55-12,61 (m étalé, 1H) Spectre de masse (LC-MS-ES+/-) : m/z 309 [M+H]⁻, m/z 311 [M+H]⁺ |
| **57** | Spectre RMN ¹H (DMSO-d6, δ en ppm) : 6,77 (s large, 1H), 7,01 (d, J=1,7 Hz, 1H), 7,71 (d, J=9,6 Hz, 1H), 7,86 (s large, 1H), 7,90 (d large, J=9,6 Hz, 1H), 8,61 (s, 1H), 8,83 (d, J=1,7 Hz, 1H), 9,09 (s large, 1H), 10,89 (s, 1H), 13,05 (s large, 1H) Spectre de masse (LC-MS-ES+/-) : m/z 293 [M+H]⁻, m/z 295 [M+H]⁺ |
| **58** | Spectre RMN ¹H (DMSO-d6, δ en ppm) : 6,79 (d large, J=2,2 Hz, 1H), 7,74 (d, J=9,5 Hz, 1H), 7,85 (s large, 1H), 7,91 (d large, J=9,5 Hz,1H), 8,74 (s, 1H), 9,11 (s large, 1H), 9,23 (s, 1H), 12,36-12,78 (m étalé, 1H), 12,97-13,16 (s large, 1H) Spectre de masse (LC-MS-ES+/-) : m/z 310 [M+H]⁻, m/z 312 [M+H]⁺ |
| **59** | Spectre RMN ¹H (DMSO-d6, δ en ppm) : 6,61 (s , 1H), 6,76 (d, J=1,9 Hz, 1H), 7,70 (m, 2H), 7,80-7,94 (m, 2H), 8,53 (s, 1H), 9,08 (s, 1H), 9,79-10,01 (m étalé, 1H), 12,38-12,54 (m étalé, 1H), 13,04 (s, 1H), (signaux larges) Spectre de masse (LC-MS-ES+/-) : m/z 292 [M+H]⁻, m/z 294 [M+H]⁺ |
| **60** | Spectre RMN ¹H (DMSO-d6, δ en ppm) : 6,67 (dd, J=3,4, 1,7 Hz, 1H), 7,08 (dd, J=3,4, 0,6 Hz, 1H), 7,20 (d, J=0,8 Hz, 1H), 7,73 (d, J=9,6 Hz, 1H), 7,79-7,87 (m, 2H), 7,95 (d, J=0,8 Hz, 1H), 8,65 (s, 1H), 9,01 (s large, 1H), 11,09 (m très étalé, 1H) Spectre de masse (LC-MS-ES+/-) : m/z 295 [M+H]⁺ |
| **61** | Spectre RMN ¹H (DMSO-d6, δ en ppm) : 7,46 (m, 1H), 7,84 (d, J=9,5 Hz, 1H), 7,90-7,97 (dd, J=9,5, 1,7 Hz, 1H), 8,32 (m, 1H), 8,87 (s, 1H), 9,24 (s, 1H), 9,41 (s large, 1H) Spectre de masse (LC-MS-ES+/-) : m/z 311 [M+H]⁻, m/z 313 [M+H]⁺ |
| **62** | Spectre RMN ¹H (DMSO-d6, δ en ppm) : 6,49-6,68 (m étalé, 1H), 7,43 (s, 1H), 7,58-7,74 (m étalé, 1H), 7,75-7,85 (d, J= 9,5 Hz, 1H), 7,85-7,96 (dd, J= 9,5, 1,7 Hz, 1H), 8,30 (s, 1H), 8,67 (s, 1H), 9,38 (s large, 1H), 9,79-10,11 (m étalé, 1H), 12,36-12,56 (m étalé, 1H) Spectre de masse (LC-MS-ES+/-) : m/z 293 [M+H]⁻, m/z 295 [M+H]⁺ |
| **63** | Spectre RMN ¹H (DMSO-d6, δ en ppm) : 7,02 (dd, J=1,9, 0,9 Hz, 1H), 7,29 ( s large, 1H), 7,80 (d, J=9,5 Hz, 1H), 7,86 (t, J=1,9 Hz, 1H), 7,94 (s large, 1H), 8,00 (dd, J=9,5, 1,1 Hz, 1H), 8,37 (s large, 1H), 8,64 (s, 1H), 9,09 (s large, 1H) Spectre de masse (LC-MS-ES+/-) : m/z 295 [M+H]⁺ |
| **64** | Spectre RMN ¹H (DMSO-d6, δ en ppm) : 4,48 (d, J=5,7 Hz, 2H), 5,29 (t, J=5,7 Hz, 1H), 6,46 (d, J=3,3 Hz, 1H), 6,58-6,66 (m étalé, 1H), 6,97 (d, J=3,3 Hz, 1H), 7,56-7,80 (m, 3H), 8,58 (s, 1H), 8,94 (s large, 1H), 9,77-9,95 (m étalé, 1H), 12,33-12,57 (m étalé, 1H) Spectre de masse (LC-MS-ES+/-) : m/z 322 [M+H]⁻, m/z 324 [M+H]⁺ |
| **65** | Spectre RMN ¹H (DMSO-d6, δ en ppm) : 7,41-7,53 (m, 2H), 7,63 (d, J=9,6 Hz, 1H), 7,70 (s large, 1H), 7,74-7,84 (m, 3H), 8,53 (s, 1H), 9,00 (s large, 1H), 10,74 (s large, 1H), 12,27 (m étalé, 1H) Spectre de masse (LC-MS-ES+/-) : m/z 308 [M+H]⁻, m/z 310 [M+H]⁺ |
| **66** | Spectre RMN ¹H (DMSO-d6, δ en ppm) : 6,62-6,72 (m, 1H), 7,22 (d, J=3,8 Hz, 1H), 7,55 (d, J=9,4 Hz, 1H), 7,60-7,69 (m, 2H), 7,76 (s, 1H), 8,27 (s large, 1H), 8,92 (s large, 1H), 12,27 (m étalé, 1H) Spectre de masse (LC-MS-ES+/-) : m/z 309 [M+H]⁻, m/z 311 [M+H]⁺ |
| **67** | Spectre RMN ¹H (DMSO-d6, δ en ppm) : 7,19 (ddd, J=7,4, 4,9, 1,0 Hz, 1H), 7,81 (d, J=9,6 Hz, 1H), 7,85-7,94 (m, 2H), 8,25 (dt, J=8,3, 1,0 Hz, 1H), 8,39 (ddd, J=4,9, 2,0, 1,0 Hz, 1H), 8,45 (m étalé, 1H), 8,67 (s, 1H), 9,20 (s large, 1H), 9,82 (s large, 1H), 15,29 (m étalé, 1 H) Spectre de masse (LC-MS-ES+/-) : m/z 306 [M+H]⁺ |
| **68** | Spectre RMN ¹H (DMSO-d6, δ en ppm): 7,46 (dd, J=5,1, 3,2 Hz, 1H), 7,51 (dd, J=5,1, 1,5 Hz, 1H), 7,75 (d, J=9,5 Hz, 1H), 7,79 (dd, J=3,2, 1,5 Hz, 1H), 7,86 (dd, J=9,5, 1,7 Hz, 1H), 8,42 (m étalé, 1H), 8,55 (s, 1H), 9,20 (s large, 1H), 10,80 (s large, 1H), 15,26 (m étalé, 1H) Spectre de masse (LC-MS-ES+/-) : m/z 309 [M+H]⁻, m/z 311 [M+H]^{+x} |

Les composés selon l'invention ont fait l'objet d'essais pharmacologiques permettant de déterminer leur effet modulateur sur NOT.

### Evaluation de l'activité in vitro sur cellules N2A

L'activité des composés selon l'invention a été évaluée sur une lignée cellulaire (N2A) exprimant de manière endogène le récepteur de souris Nurr1 et transfectée de manière stable avec l'élément de réponse liant NOT (NBRE) couplé au gène rapporteur luciférase. Les EC₅₀ sont comprises entre 0,01 et 1000 nM. Les essais ont été réalisés selon le mode opératoire décrit ci dessous.

La lignée cellulaire Neuro-2A provient de source commerciale standard (ATCC). Le clone Neuro-2A a été obtenu à partir d'une tumeur spontanée provenant d'une souche de souris A albino par R.J Klebe et col. Cette lignée Neuro-2A est ensuite stablement transfectée avec 8NBRE-luciférase. Les cellules N2A-8NBRE sont cultivées jusqu'à confluence dans des flacons de culture de 75 cm² contenant du DMEM supplémenté par 10% de sérum foetal de veau, 4,5 g/L de glucose et 0,4 mg/ml de Généticine. Après une semaine de culture les cellules sont récupérées par de la trypsine 0,25% pendant 30 secondes puis remises en suspension dans du DMEM sans rouge de phénol contenant 4,5g/L de glucose, 10% de sérum délipidé Hyclone et déposées dans des plaques blanches 96 puits à fond transparent. Les cellules sont déposées à raison de 60.000 par puits dans 75 µL pendant 24 heures avant l'addition des produits. Les produits sont appliqués dans 25µL et incubés 24 heures supplémentaires. Le jour de la mesure, on ajoute à chaque puits un volume équivalent (100µL) de Steadylite, puis on attend 30 minutes pour obtenir une lyse complète des cellules et la production maximale du signal. Les plaques sont ensuite mesurées dans un compteur de luminescence pour microplaques après avoir été scellées par un film adhésif. Les produits sont préparés sous forme de solution stock à 10⁻² M, puis dilués dans 100% de DMSO. Chaque concentration de produit est préalablement diluée dans du milieu de culture avant incubation avec les cellules contenant ainsi 0,625% final de DMSO.

Par exemple, les composés n° 1, 2, 7, 10, 22, 36, 51, 56 et 58 ont montré une CE₅₀ de respectivement 16 nM, 1,5 nM, 0,8 nM, 21 nM, 2 nM, 0,4 nM, 1,5 nM, 1 nM et 5,3 nM.

Il apparaît donc que les composés selon l'invention ont un effet modulateur de NOT.

Les composés selon l'invention peuvent donc être utilisés pour la préparation de médicaments pour leur application en thérapeutique dans le traitement ou la prévention de maladies impliquant les récepteurs NOT.

Ainsi, selon un autre de ses aspects, l'invention a pour objet des médicaments qui comprennent un composé de formule (I), ou un sel d'addition de ce dernier à un acide pharmaceutiquement acceptable.

Ces médicaments trouvent leur emploi en thérapeutique, notamment dans le traitement et la prévention des maladies neurodégénératives telles que par exemple la maladie de Parkinson, d'Alzheimer, les tauopathies (ex. la paralysie progressive supranucléaire, la démence fronto temporale, la dégénérescence corticobasale, la maladie de Pick); les traumatismes cérébraux comme l'ischémie et les traumatismes crâniens et l'épilepsie ; les maladies psychiatriques comme la schizophrénie, la dépression, la dépendance à une substance, les troubles du déficit de l'attention et de l'hyperactivité ; les maladies inflammatoires du système nerveux central comme la sclérose en plaque, encéphalite, myélite et encéphalomyélite et autres maladies inflammatoires comme les pathologies vasculaires, l'athérosclérose, les inflammations des articulations, l'arthrose, l'arthrite rhumatoïde ; l'ostéoarthrite, la maladie de Crohn, colite ulcéreuse; les maladies inflammatoires allergiques telle que l'asthme, les maladies auto-immunes comme le diabète de type 1, lupus, sclérodermies, Syndrome de Guillain-Barré, maladie d'Addison et autres maladies immuno-médiées; l'ostéoporose; les cancers.

Ainsi, un objet de la présente invention vise un composé de formule (I) tel que défini précédemment pour le traitement des maladies, troubles et désordres précités.

Selon un autre de ses aspects, la présente invention concerne l'utilisation d'un composé de formule (I) tel que défini précédemment pour la préparation d'un médicament destiné au traitement et à la prévention de l'un(e) des maladies, troubles ou désordres cités ci-dessus.

Ces composés pourraient être aussi utilisés comme traitement associé à des greffes et/ou transplantations de cellules souches.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'Homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, peut être administré sous forme unitaire d'administration, en mélange avec des excipients pharmaceutiques classiques, aux animaux et aux êtres humains pour la prophylaxie ou le traitement des troubles ou des maladies ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants:

| | |
|---|---|
| Composé selon l'invention | 50,0 mg |
| Mannitol | 223,75 mg |
| Croscarmellose sodique | 6,0 mg |
| Amidon de maïs | 15,0 mg |
| Hydroxypropyl-méthylcellulose | 2,25 mg |
| Stéarate de magnésium | 3,0 mg |

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

1. **Composés répondant à la formule (I) :** 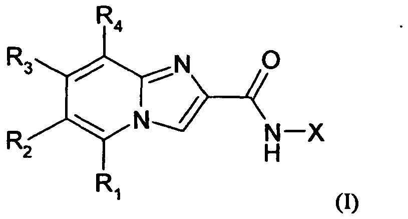 dans laquelle :
X représente un groupe hétérocyclique éventuellement substitué par un ou plusieurs groupes choisis indépendamment les uns des autres parmi les atomes ou groupes suivants : halogène, (C₁-C₆)alcoxy, (C₁-C₆)alkyle, NRaRb, cyano, oxido, COOR₈, les groupes alkyle et alcoxy pouvant éventuellement être substitués par un ou plusieurs atomes d'halogène ;
R₁ représente un atome d'hydrogène, un atome d'halogène, un groupe (C₁-C₆)alcoxy, un groupe (C₁-C₆)alkyle, amino ou NRaRb; les groupes alkyle et alcoxy pouvant éventuellement être substitués par un ou plusieurs atomes d'halogène, un groupe hydroxy, amino, ou un groupe (C₁-C₆)alcoxy ;
R₂ représente un groupe hétérocyclique, ce groupe étant éventuellement substitué par un ou plusieurs groupes choisis indépendamment les uns des autres parmi les atomes ou groupes suivants : hydroxy, (C₁-C₆)alkyle, (C₁-C₆)alcoxy, halogène, cyano, NRaRb, -CO-R₅, - CO-NR₆R₇, -CO-O-R₈, -NR₉-CO-R₁₀, les groupes (C₁-C₆)alkyle et (C₁-C₆)alkoxy étant éventuellement substitués par un ou plusieurs atomes d'halogène ou groupes hydroxy, NRaRb, oxido ;
R₃ représente un atome d'hydrogène, un groupe (C₁-C₆)alkyle, un groupe (C₁-C₆)alcoxy ou un atome d'halogène ;
R₄ représente un atome d'hydrogène, un groupe (C₁-C₄)alkyle, un groupe (C₁-C₄)alcoxy ou un atome de fluor ;
R₅ représente un atome d'hydrogène, un groupe phényle ou un groupe (C₁-C₆)alkyle ;
R₆ et R₇, identiques ou différents, représentent un atome d'hydrogène ou un groupe (C₁-C₆)alkyle, ou forment avec l'atome d'azote qui les porte un cycle de 4 à 7 chaînons incluant éventuellement un autre hétéroatome choisi parmi N, O ou S ;
R₈ représente un groupe (C₁-C₆)alkyle ;
R₉ et R₁₀, identiques ou différents, représentent un atome d'hydrogène ou un groupe (C₁-C₆)alkyle ;
Ra et Rb représentent, indépendamment l'un de l'autre, un atome d'hydrogène, un groupe (C₁-C₆)alkyle ou forment avec l'atome d'azote qui les porte un cycle de 4 à 7 chaînons, incluant éventuellement un autre hétéroatome choisi parmi N, O ou S ;
un groupe hétérocyclique représente un groupe mono ou bicyclique comportant de 5 à 10 atomes dont de 1 à 4 hétéroatomes choisis parmi N, O et S, ce groupe cyclique étant aromatique, insaturé ou partiellement insaturé ou oxydé et étant relié par un atome de carbone ;
leurs racémiques, énantiomères, diastéréoisomères et leurs mélanges;
à l'état de base ou de sel d'addition à un acide, ainsi qu'à l'état d'hydrate ou de solvat.

2. Composés de formule (I) selon la revendication 1, **caractérisé en ce que** :
X représente un groupe hétérocyclique éventuellement substitué par un ou plusieurs groupes choisis indépendamment les uns des autres parmi les atomes d'halogène,
à l'état de base ou de sel d'addition à un acide.

3. Composés de formule (I) selon la revendication 1 ou 2, **caractérisé en ce que**
R₁, R₃, R₄ représentent un atome d'hydrogène,
à l'état de base ou de sel d'addition à un acide.

4. Composés de formule (I) selon l'une quelconque des revendications précédentes, **caractérisé en ce que** :
R₂ représente un groupe hétérocyclique, ce groupe étant éventuellement substitué par un ou plusieurs groupes choisis indépendamment les uns des autres parmi les atomes ou groupes suivants : (C₁-C₆)alkyle, NRaRb, le ou les groupes (C₁-C₆)alkyle étant éventuellement substitués par un ou plusieurs atomes d'halogène ou groupes hydroxy,
Ra et Rb représentent, indépendamment l'un de l'autre, un atome d'hydrogène, un groupe (C₁-C₆)alkyle,
à l'état de base ou de sel d'addition à un acide.

5. Composés de formule (I) selon l'une quelconque des revendications précédentes, **caractérisé en ce que** :
X représente un groupe pyridine, isoxazole, thiazole, thiadiazole, pyrazole, thiophène, ou oxazole, ces groupes étant éventuellement substitués par un atome de fluor,
R₂ représente un groupe pyridine, thiophéne, imidazole, pyrrole, furanne, oxazole, triazole, ou pyrazole, ces groupes étant éventuellement substitués par un groupe NH₂ ou hydroxyméthyle,
R₁, R₃, R₄ représentent un atome d'hydrogène
à l'état de base ou de sel d'addition à un acide.

6. Composé de formule (I) selon l'une quelconque des revendications précédentes, **caractérisé en ce qu'**il est choisi parmi :
• 6-(6-Aminopyridin-2-yl)-*N*-(pyridin-2-yl)imidazo[1,2-*a*]pyridine-2-carboxamide et son chlorhydrate (2:1)
• 6-(1*H*-Imidazol-4-yl)-*N*-(pyridin-2-yl)imidazo[1,2-*a*]pyridine-2-carboxamide
• *N*,6-Di(pyridin-2-yl)imidazo[1,2-*a*]pyridine-2-carboxamide
• *N*-(Pyridin-2-yl)-6-(thiophèn-3-yl)imidazo[1,2-*a*]pyridine-2-carboxamide
• *N*-(Isoxazol-4-yl)-6-(pyridin-2-yl)imidazo[1,2-*a*]pyridine-2-carboxamide
• *N*-(6-Fluoropyridin-2-yl)-6-(pyridin-2-yl)imidazo[1,2-*a*]pyridine-2-carboxamide
• 6-(Pyridin-2-yl-*N*-(thiazol-2-yl)imidazo[1,2-*a*]pyridine-2-carboxamide
• *N*-(Isoxazol-3-yl)-6-(pyridin-2-yl)imidazo[1,2-*a*]pyridine-2-carboxamide
• 6-(Pyridin-2-yl-*N*-(1,3,4-thiadiazol-2-yl)imidazo[1,2-*a*]pyridine-2-carboxamide
• *N*-(1*H*-Pyrazol-3-yl)-6-(pyridin-2-yl)imidazo[1,2-*a*]pyridine-2-carboxamide
• 6-(Pyridin-2-yl-*N*-(thiophén-3-yl)imidazo[1,2-*a*]pyridine-2-carboxamide
• 6-(1*H*-Pyrrol-3-yl)-*N*-(thiazol-2-yl)imidazo[1,2-*a*]pyridine-2-carboxamide
• *N*-(Isoxazol-4-yl)-6-(1*H*-pyrazol-3-yl)imidazo[1,2-*a*]pyridine-2-carboxamide
• 6-(Furan-2-yl)-*N*-(thiazol-2-yl)imidazo[1,2-*a*]pyridine-2-carboxamide
• 6-(Furan-2-yl)-*N*-(isoxazol-4-yl)imidazo[1,2-*a*]pyridine-2-carboxamide
• 6-(Oxazol-2-yl)-*N*-(thiazol-2-yl)imidazo[1,2-*a*]pyridine-2-carboxamide
• *N*-(Isoxazol-3-yl)-6-(oxazol-2-yl)imidazo[1,2-*a*]pyridine-2-carboxamide
• *N*-(Isoxazol-4-yl)-6-(oxazol-2-yl)imidazo[1,2-*a*]pyridine-2-carboxamide
• 6-(Furan-3-yl)-*N*-(thiazol-2-yl)imidazo[1,2-*a*]pyridine-2-carboxamide
• 6-(Furan-3-yl)-*N*-(isoxazol-4-yl)imidazo[1,2-*a*]pyridine-2-carboxamide
• 6-[5-(Hydroxyméthyl)furan-2-yl]-*N*-(thiazol-2-yl)imidazo[1,2-*a*]pyridine-2-carboxamide
• 6-[5-(Hydroxyméthyl)furan-2-yl]-*N*-(isoxazol-3-yl)imidazo[1,2-*a*]pyridine-2-carboxamide
• 6-[5-(Hydroxyméthyl)furan-2-yl]-*N*-(isoxazol-4-yl)imidazo[1,2-*a*]pyridine-2-carboxamide
• 6-(1*H*-Imidazol-4-yl)-*N*-(isoxazol-3-yl)imidazo[1,2-*a*]pyridine-2-carboxamide
• 6-(6-Aminopyridin-2-yl)-*N*-(thiazol-2-yl)imidazo[1,2-*a*]pyridine-2-carboxamide
• 6-(1*H*-Pyrrol-3-yl)-*N*-(thiophén-3-yl)imidazo[1,2-*a*]pyridine-2-carboxamide
• 6-(Furan-2-yl)-*N*-(pyridin-2-yl)imidazo[1,2-*a*]pyridine-2-carboxamide
• *N*-(6-Fluoropyridin-2-yl)-6-(furan-2-yl)imidazo[1,2-*a*]pyridine-2-carboxamide
• 6-(Furan-2-yl)-*N*-(thiophén-3-yl)imidazo[1,2-*a*]pyridine-2-carboxamide
• 6-(Furan-2-yl)-*N*-(isoxazol-3-yl)imidazo[1,2-*a*]pyridine-2-carboxamide
• 6-(Furan-2-yl)-*N*-(1,3,4-thiadiazol-2-yl)imidazo[1,2-*a*]pyridine-2-carboxamide
• 6-(Oxazol-2-yl)-*N*-(pyridin-2-yl)imidazo[1,2-*a*]pyridine-2-carboxamide
• *N*-(6-Fluoropyridin-2-yl)-6-(1,3-oxazol-2-yl)imidazo[1,2-*a*]pyridine-2-carboxamide
• 6-(Oxazol-2-yl)-*N*-(thiophén-3-yl)imidazo[1,2-*a*]pyridine-2-carboxamide
• 6-(Furan-3-yl)-*N*-(pyridin-2-yl)imidazo[1,2-*a*]pyridine-2-carboxamide
• *N*-(6-Fluoropyridin-2-yl)-6-(furan-3-yl)imidazo[1,2-*a*]pyridine-2-carboxamide
• 6-(Furan-3-yl)-*N*-(thiophén-3-yl)imidazo[1,2-*a*]pyridine-2-carboxamide
• 6-(Furan-3-yl)-*N*-(isoxazol-3-yl)imidazo[1,2-*a*]pyridine-2-carboxamide
• 6-(Furan-3-yl)-*N*-(1,3,4-thiadiazol-2-yl)imidazo[1,2-*a*]pyridine-2-carboxamide
• 6-[5-(Hydroxyméthyl)furan-2-yl]-*N*-(pyridin-2-yl)imidazo[1,2-*a*]pyridine-2-carboxamide
• 6-[5-(Hydroxyméthyl)furan-2-yl]-*N*-(thiophén-3-yl)imidazo[1,2-*a*]pyridine-2-carboxamide
• 6-(Furan-2-yl)-*N*-(1*H*-pyrazol-3-yl)imidazo[1,2-*a*]pyridine-2-carboxamide
• *N*-(Thiophén-3-yl)-6-(1*H*-1,2,4-triazol-3-yl)imidazo[1,2-*a*]pyridine-2-carboxamide
• 6-(6-Aminopyridin-2-yl)-*N*-(6-fluoropyridin-2-yl)imidazo[1,2-*a*]pyridine-2-carboxamide et son chlorhydrate (2:1)
• 6-(6-Aminopyridin-2-yl)-*N*-(thiophén-3-yl)imidazo[1,2-*a*]pyridine-2-carboxamide et son chlorhydrate (2:1)
• 6-(6-Aminopyridin-2-yl)-*N*-(isoxazol-3-yl)imidazo[1,2-*a*]pyridine-2-carboxamide et son chlorhydrate (2:1)
• 6-(6-Aminopyridin-2-yl)-*N*-(1,3,4-thiadiazol-2-yl)imidazo[1,2-*a*]pyridine-2-carboxamide et son chlorhydrate (2:1)
• 6-(6-Aminopyridin-2-yl)-*N*-(isoxazol-4-yl)imidazo[1,2-*a*]pyridine-2-carboxamide et son chlorhydrate (2:1)
• *N*-(Pyridin-2-yl)-6-(1*H*-pyrrol-3-yl)imidazo[1,2-*a*]pyridine-2-carboxamide et son trifluoroacétate (1:1)
• *N*-(Isoxazol-3-yl)-6-(1*H*-pyrrol-3-yl)imidazo[1,2-*a*]pyridine-2-carboxamide
• 6-(1*H*-Pyrrol-3-yl)-*N*-(1,3,4-thiadiazol-2-yl)imidazo[1,2-*a*]pyridine-2-carboxamide
• *N*-(Oxazol-2-yl)-6-(1*H*-pyrrol-3-yl)imidazo[1,2-*a*]pyridine-2-carboxamide
• *N*-(Isoxazol-4-yl)-6-(1*H*-pyrrol-3-yl)imidazo[1,2-*a*]pyridine-2-carboxamide
• 6-(1*H*-Pyrazol-3-yl)-*N*-(pyridin-2-yl)imidazo[1,2-*a*]pyridine-2-carboxamide
• 6-(1*H*-Pyrazol-3-yl)-*N*-(thiophén-3-yl)imidazo[1,2-*a*]pyridine-2-carboxamide et son trifluoroacétate (1:1)
• 6-(1*H*-Pyrazol-3-yl)-*N*-(1,3-thiazol-2-yl)imidazo[1,2-*a*]pyridine-2-carboxamide et son trifluoroacétate (1:1)
• *N*-(Isoxazol-3-yl)-6-(1*H*-pyrazol-3-yl)imidazo[1,2-*a*]pyridine-2-carboxamide
• 6-(1*H*-Pyrazol-3-yl)-*N*-(1,3,4-thiadiazol-2-yl)imidazo[1,2-*a*]pyridine-2-carboxamide et son trifluoroacétate (1:1)
• *N*,6-Di(1*H*-pyrazol-3-yl)imidazo[1,2-*a*]pyridine-2-carboxamide
• 6-(Furan-2-yl)-*N*-(1,3-oxazol-2-yl)imidazo[1,2-*a*]pyridine-2-carboxamide et son trifluoroacétate (1:1)
• 6-(Oxazol-2-yl)-*N*-(1,3,4-thiadiazol-2-yl)imidazo[1,2-*a*]pyridine-2-carboxamide
• 6-(Oxazol-2-yl)-*N*-(1*H*-pyrazol-3-yl)imidazo[1,2-*a*]pyridine-2-carboxamide
• 6-(Furan-3-yl)-*N*-(1,3-oxazol-2-yl)imidazo[1,2-*a*]pyridine-2-carboxamide et son trifluoroacétate (1:1)
• 6-[5-(Hydroxyméthyl)furan-2-yl]-*N*-(1*H*-pyrazol-3-yl)imidazo[1,2-*a*]pyridine-2-carboxamide
• 6-(1*H*-Imidazol-4-yl)-*N*-(thiophén-3-yl)imidazo[1,2-*a*]pyridine-2-carboxamide
• 6-(1*H*-Imidazol-4-yl)-*N*-(1,3-thiazol-2-yl)imidazo[1,2-*a*]pyridine-2-carboxamide
• *N*-(Pyridin-2-yl)-6-(1*H*-1,2,3-triazol-4-yl)imidazo[1,2-*a*]pyridine-2-carboxamide
• *N*-(Thiophén-3-yl)-6-(1*H*-1,2,3-triazol-4-yl)imidazo[1,2-*a*]pyridine-2-carboxamide ;
et leurs sels d'addition à un acide.

7. Médicament, **caractérisé en ce qu'**il comprend un composé de formule (I) selon l'une quelconque des revendications 1 à 6, ou un sel d'addition de ce composé à un acide pharmaceutiquement acceptable.

8. Composition pharmaceutique, **caractérisée en ce qu'**elle comprend un composé de formule (I) selon l'une quelconque des revendications 1 à 6, ou un sel pharmaceutiquement acceptable, de ce composé, ainsi qu'au moins un excipient pharmaceutiquement acceptable.

9. Utilisation d'un composé de formule (I) selon l'une quelconque des revendications 1 à 6, pour la préparation d'un médicament destiné au traitement et à la prévention des maladies neurodégénératives.

10. Utilisation d'un composé de formule (I) selon l'une quelconque des revendications 1 à 6, pour la préparation d'un médicament destiné au traitement et à la prévention des traumatismes cérébraux et de l'épilepsie.

11. Utilisation d'un composé de formule (I) selon l'une quelconque des revendications 1 à 6, pour la préparation d'un médicament destiné au traitement et à la prévention des maladies psychiatriques.

12. Utilisation d'un composé de formule (I) selon l'une quelconque des revendications 1 à 6, pour la préparation d'un médicament destiné au traitement et à la prévention des maladies inflammatoires.

13. Utilisation d'un composé de formule (I) selon l'une quelconque des revendications 1 à 6, pour la préparation d'un médicament destiné au traitement et à la prévention de l'ostéoporose.

14. Utilisation d'un composé de formule (I) selon l'une quelconque des revendications 1 à 6, pour la préparation d'un médicament destiné au traitement et à la prévention des cancers.

15. Utilisation d'un composé de formule (I) selon l'une quelconque des revendications 1 à 6 pour la préparation d'un médicament destiné au traitement et à la prévention de la maladie de Parkinson, d'Alzheimer, des tauopathies, de la sclérose en plaque.

16. Utilisation d'un composé de formule (I) selon l'une quelconque des revendications 1 à 6, pour la préparation d'un médicament destiné au traitement et à la prévention de la schizophrénie, la dépression, la dépendance à une substance les troubles du déficit de l'attention et de l'hyperactivité.

17. Composés
Acide 6-(6-{[(1,1-diméthyléthoxy)carbonyl]amino}pyridin-2-yl)imidazo[1,2-*a*]pyridine-2-carboxylique
Acide 6-(pyridin-2-yl)imidazo[1,2-*a*]pyridine-2-carboxylique
Acide 6-(1-triphénylméthyl-1*H*-imidazol-4-yl)imidazo[1,2-*a*]pyridine-2-carboxylique
Acide 6-(2-{[(1,1-diméthyléthoxy)carbonyl]amino}-thiazol-4-yl)imidazo[1,2-*a*]pyridine-2-carboxylique
Acide 6-(1*H*-pyrrol-3-yl)imidazo[1,2-*a*]pyridine-2-carboxylique
Acide 6-(1*H*-pyrazol-3-yl)imidazo[1,2-*a*]pyridine-2-carboxylique
Acide 6-(1*H*-pyrazol-4-yl)imidazo[1,2-*a*]pyridine-2-carboxylique
Acide 6-(furan-2-yl)imidazo[1,2-*a*]pyridine-2-carboxylique
Acide 6-(furan-3-yl)imidazo[1,2-*a*]pyridine-2-carboxylique
Acide 6-[5-(hydroxyméthyl)furan-2-yl]imidazo[1,2-*a*]pyridine-2-carboxylique
Acide 6-(thiophèn-3-yl)imidazo[1,2-*a*]pyridine-2-carboxylique
Acide 6-(oxazol-2-yl)imidazo[1,2-*a*]pyridine-2-carboxylique
Acide 6-(1*H*-1,2,4-triazol-3-yl)imidazo[1,2-*a*]pyridine-2-carboxylique
Acide 6-(1*H*-1,2,3-triazol-4-yl)imidazo[1,2-*a*]pyridine-2-carboxylique
6-iodo-*N*-(isoxazol-4-yl)imidazo[1,2-*a*]pyridine-2-carboxamide
6-iodo-*N*-(thiazol-2-yl)imidazo[1,2-*a*]pyridine-2-carboxamide
6-Iodo-*N*-(pyridin-2-yl)imidazo[1,2-*a*]pyridine-2-carboxamide
6-Triméthylstannyl-*N*-(pyridin-2-yl)imidazo[1,2-*a*]pyridine-2-carboxamide Bromhydrate (1:1) de 6-(4,4,5,5-tétraméthyl-1,3,2-dioxaborolan-2-yl)imidazo[1,2-*a*]pyridine-2-carboxylate d'éthyle
Acide 2-éthoxycarbonyl-imidazo[1,2-*a*]pyridine-6-boronique.

18. Utilisation des composés selon la revendication 17, pour la synthèse de produits de formule générale (I) telle que définie dans la revendication 1.

## Claims

1. Compounds corresponding to formula (I): 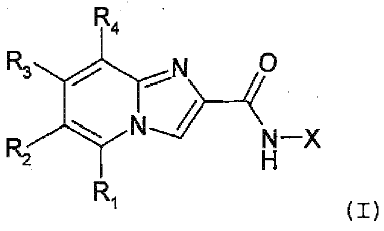 in which:
X represents a heterocyclic group optionally substituted with one or more groups chosen, independently of each other, from the following groups or atoms: halogen, (C₁-C₆) alkoxy, (C₁-C₆) alkyl, NRaRb, cyano, oxido, COOR₈, the alkyl and alkoxy groups possibly being substituted with one or more halogen atoms;
R₁ represents a hydrogen atom, a halogen atom, a group (C₁-C₆)alkoxy, a group (C₁-C₆)alkyl, amino or NRaRb; the alkyl and alkoxy groups possibly being substituted with one or more halogen atoms, a hydroxyl or amino group, or a group (C₁-C₆)alkoxy;
R₂ represents a heterocyclic group, this group possibly being substituted with one or more groups chosen, independently of each other, from the following groups or atoms: hydroxyl, (C₁-C₆) alkyl, (C₁-C₆)alkoxy, halogen, cyano, NRaRb, -CO-R₅, -CO-NR₆R₇, -CO-O-R₈, -NR₉-CO-R₁₀, the groups (C₁-C₆)alkyl and (C₁-C₆)alkoxy being optionally substituted with one or more halogen atoms or hydroxyl, NRaRb or oxido groups;
R₃ represents a hydrogen atom, a group (C₁-C₆)alkyl, a group (C₁-C₆)alkoxy or a halogen atom;
R4 represents a hydrogen atom, a group (C₁-C₄)alkyl, a group (C₁-C₄)alkoxy or a fluorine atom;
R₅ represents a hydrogen atom, a phenyl group or a group (C₁-C₆) alkyl;
R₆ and R₇, which may be identical or different, represent a hydrogen atom or a group (C₁-C₆)alkyl, or form, with the nitrogen atom that bears them, a 4- to 7-membered ring optionally including another heteroatom chosen from N, O and S;
R₈ represents a group (C₁-C₆)alkyl;
R₉ and R₁₀, which may be identical or different, represent a hydrogen atom or a group (C₁-C₆)alkyl;
Ra and Rb represent, independently of each other, a hydrogen atom, a group (C₁-C₆)alkyl or form, with the nitrogen atom that bears them, a 4- to 7-membered ring optionally including another heteroatom chosen from N, O and S;
a heterocyclic group represents a monocyclic or bicyclic group comprising from 5 to 10 atoms including from 1 to 4 heteroatoms chosen from N, O and S, this cyclic group being aromatic, unsaturated or partially unsaturated or oxidized, and is connected via a carbon atom;
the racemic mixtures, enantiomers, diastereoisomers and mixtures thereof in the form of the base or of an acid-addition salt, and in the form of a hydrate or solvate.

2. Compounds of formula (I) according to Claim 1, **characterized in that**:
X represents a heterocyclic group optionally substituted with one or more groups chosen, independently of each other, from halogen atoms,
in the form of the base or of an acid-addition salt.

3. Compounds of formula (I) according to Claim 1 or 2, **characterized in that**:
R₁, R₃ and R₄ represent a hydrogen atom,
in the form of the base or of an acid-addition salt.

4. Compounds of formula (I) according to any one of the preceding claims, **characterized in that**:
R₂ represents a heterocyclic group, this group possibly being substituted with one or more groups chosen, independently of each other, from the following groups or atoms: (C₁-C₆) alkyl, NRaRb, the group(s) (C₁-C₆)alkyl being optionally substituted with one or more halogen atoms or hydroxyl groups,
Ra and Rb represent, independently of each other, a hydrogen atom or a group (C₁-C₆)alkyl,
in the form of the base or of an acid-addition salt.

5. Compounds of formula (I) according to any one of the preceding claims, **characterized in that**:
X represents a pyridine, isoxazole, thiazole, thiadiazole, pyrazole, thiophene or oxazole group, these groups being optionally substituted with a fluorine atom,
R₂ represents pyridine, thiophene, imidazole, pyrrole, furan, oxazole, triazole or pyrazole group, these groups being optionally substituted with an NH₂ or hydroxymethyl group,
R₁, R₃ and R₄ represent a hydrogen atom,
in the form of the base or of an acid-addition salt.

6. Compound of formula (I) according to any one of the preceding claims, **characterized in that** it is chosen from:
• 6-(6-Aminopyridin-2-yl)-*N*-(pyridin-2-yl)imidazo[1,2-a]pyridine-2-carboxamide and its hydrochloride (1:2)
• 6-(1*H*-Imidazol-4-yl)-*N*-(pyridin-2-yl)imidazo[1,2-*a*]pyridine-2-carboxamide
• *N,*6-Di(pyridin-2-yl)imidazo[1,2-*a*]pyridine-2-carboxamide
• *N*-(Pyridin-2-yl)-6-(thiophen-3-yl)imidazo [1,2-*a*]pyridine-2-carboxamide
• *N*-(Isoxazol-4-yl)-6-(pyridin-2-yl)imidazo[1,2-*a*]pyridine-2-carboxamide
• *N*-(6-Fluoropyridin-2-yl)-6-(pyridin-2-yl)imidazo[1,2-*a*]pyridine-2-carboxamide
• 6-(Pyridin-2-yl-*N*-(thiazol-2-yl)imidazo[1,2-*a*]pyridine-2-carboxamide
• *N*-(Isoxazol-3-yl)-6-(pyridin-2-yl)imidazo[1,2-*a*]pyridine-2-carboxamide
• 6-(Pyridin-2-yl-*N*-(1,3,4-thiadiazol-2-yl)imidazo[1,2-*a*]pyridine-2-carboxamide
• *N*-(1*H*-Pyrazol-3-yl)-6-(pyridin-2-yl)imidazo[1,2-*a*]pyridine-2-carboxamide
• 6-(Pyridin-2-yl-*N*-(thiophen-3-yl)imidazo[1,2-*a*]pyridine-2-carboxamide
• 6-(1*H*-Pyrrol-3-yl)-*N*-(thiazol-2-yl)imidazo[1,2-*a*]pyridine-2-carboxamide
• *N*-(Isoxazol-4-yl)-6-(1*H*-pyrazol-3-yl)imidazo[1,2-*a*]pyridine-2-carboxamide
• 6-(Furan-2-yl)-*N*-(thiazol-2-yl)imidazo[1,2-*a*]pyridine-2-carboxamide
• 6-(Furan-2-yl)-*N*-(isoxazol-4-yl)imidazo[1,2-*a*]pyridine 2-carboxamide
• 6-(Oxazol-2-yl)-*N*-(thiazol-2-yl)imidazo[1,2-*a*]pyridine 2-carboxamide
• *N*-(Isoxazol-3-yl)-6-(oxazol-2-yl)imidazo[1,2-*a*]pyridine-2-carboxamide
• *N*-(Isoxazol-4-yl)-6-(oxazol-2-yl)imidazo[1,2-*a*]pyridine-2-carboxamide
• 6-(Furan-3-yl)-*N*-(thiazol-2-yl)imidazo[1,2-*a*]pyridine-2-carboxamide
• 6-(Furan-3-yl)-*N*-(isoxazol-4-yl)imidazo[1,2-*a*]pyridine-2-carboxamide
• 6-[5-(Hydroxymethyl)furan-2-yl]-*N*-(thiazol-2-yl)imidazo[1,2-*a*]pyridine-2-carboxamide
• 6-[5-(Hydroxymethyl)furan-2-yl]-*N*-(isoxazol-3-yl)imidazo[1,2-*a*]pyridine-2-carboxamide
• 6-[5-(Hydroxymethyl)furan-2-yl]-*N*-(isoxazol-4-yl)imidazo[1,2-*a*]pyridine-2-carboxamide
• 6-(1*H*-Imidazol-4-yl)-*N*-(isoxazol-3-yl)imidazo[1,2-*a*]pyridine-2-carboxamide
• 6-(6-Aminopyridin-2-yl)-*N*-(thiazol-2-yl)imidazo[1,2-*a*]pyridine-2-carboxamide
• 6-(1*H*-Pyrrol-3-yl)-*N*-(thiophen-3-yl)imidazo[1,2-*a*]pyridine-2-carboxamide
• 6-(Furan-2-yl)-*N*-(pyridin-2-yl)imidazo[1,2-*a*]pyridine-2-carboxamide
• *N*-(6-Fluoropyridin-2-yl)-6-(furan-2-yl)imidazo[1,2-*a*]pyridine-2-carboxamide
• 6-(Furan-2-yl)-*N*-(thiophen-3-yl)imidazo[1,2-*a*]pyridine-2-carboxamide
• 6-(Furan-2-yl)-*N*-(isoxazol-3-yl)imidazo[1,2-*a*]pyridine-2-carboxamid
• 6-(Furan-2-yl)-*N*-(1,3,4-thiadiazol-2-yl)imidazo[1,2-*a*]pyridine-2-carboxamide
• 6-(Oxazol-2-yl)-*N*-(pyridin-2-yl)imidazo[1,2-*a*]pyridine-2-carboxamide
• *N*-(6-Fluoropyridin-2-yl)-6-(1,3-oxazol-2-yl)imidazo[1,2-*a*]pyridine-2-carboxamide
• 6-(Oxazol-2-yl)-*N*-(thiophen-3-yl)imidazo[1,2-*a*]pyridine-2-carboxamide
• 6-(Furan-3-yl)-*N*-(pyridin-2-yl)imidazo[1,2-*a*]pyridine-2-carboxamide
• *N*-(6-Fluoropyridin-2-yl)-6-(furan-3-yl)imidazo[1,2-a]pyridine-2-carboxamide
• 6-(Furan-3-yl)-*N*-(thiophen-3-yl)imidazo[1,2-*a*]pyridine-2-carboxamide
• 6-(Furan-3-yl)-*N*-(isoxazol-3-yl)imidazo[1,2-*a*]pyridine-2-carboxamide
• 6-(Furan-3-yl)-*N*-(1,3,4-thiadiazol-2-yl)imidazo[1,2-*a*]pyridine-2-carboxamide
• 6-[5-(Hydroxymethyl)furan-2-yl]-*N*-(pyridin-2-yl)imidazo[1,2-*a*]pyridine-2-carboxamide
• 6-[5-(Hydroxymethyl)furan-2-yl]-*N*-(thiophen-3-yl)imidazo[1,2-*a*]pyridine-2-carboxamide
• 6-(Furan-2-yl)-*N*-(1*H*-pyrazol-3-yl)imidazo[1,2-*a*]pyridine-2-carboxamide
• *N*-(Thiophen-3-yl)-6-(1*H*-1,2,4-triazol-3-yl)imidazo[1,2-*a*]pyridine-2-carboxamide
• 6-(6-Aminopyridin-2-yl)-*N*-(6-fluoropyridin-2-yl)imidazo[1,2-*a*]pyridine-2-carboxamide and its hydrochloride (1:2)
• 6-(6-Aminopyridin-2-yl)-*N*-(thiophen-3-yl)imidazo[1,2-*a*]pyridine-2-carboxamide and its hydrochloride (1:2)
• 6-(6-Aminopyridin-2-yl)-*N*-(isoxazol-3-yl)imidazo[1,2-*a*]pyridine-2-carboxamide and its hydrochloride (1:2)
• 6-(6-Aminopyridin-2-yl)-*N*-(1,3,4-thiadiazol-2-yl)imidazo[1,2-*a*]pyridine-2-carboxamide and its hydrochloride (1:2)
• 6-(6-Aminopyridin-2-yl)-*N*-(isoxazol-4-yl)imidazo[1,2-*a*]pyridine-2-carboxamide and its hydrochloride (1:2)
• *N*-(Pyridin-2-yl)-6-(1*H*-pyrrol-3-yl)imidazo[1,2-*a*]pyridine-2-carboxamide and its trifluoroacetate (1:1)
• *N*-(Isoxazol-3-yl)-6-(1*H*-pyrrol-3-yl)imidazo[1,2-*a*]pyridine-2-carboxamide
• 6-(1*H*-Pyrrol-3-yl)-*N*-(1,3,4-thiadiazol-2-yl)imidazo[1,2-*a*]pyridine-2-carboxamide
• *N*-(Oxazol-2-yl)-6-(1*H*-pyrrol-3-yl)imidazo[1,2-a]pyridine-2-carboxamide
• *N*-(Isoxazol-4-yl)-6-(1*H*-pyrrol-3-yl)imidazo[1,2-a]pyridine-2-carboxamide
• 5-(1*H*-Pyrazol-3-yl)-*N*-(pyridin-2-yl)imidazo[1,2-a]pyridine-2-carboxamide
• 6-(1*H*-Pyrazol-3-yl)-*N*-(thiophen-3-yl)imidazo[1,2-a]pyridine-2-carboxamide and its trifluoroacetate (1:1)
• 6-(1*H*-Pyrazol-3-yl)-*N*-(1,3-thiazol-2-yl)imadazo[1,2-*a*]pyridine-2-carboxamide and its trifluoroacetate (1:1)
• *N*-(Isoxazol-3-yl)-6-(1*H*-pyrazol-3-yl)imidazo[1,2-*a*]pyridine-2-carboxamide
• 6-(1*H*-Pyrazol-3-yl)-*N*-(1,3,4-thiadiazol-2-yl)imidazo[1,2-*a*]pyridine-2-carboxamide and its trifluoroacetate (1:1)
• *N*,6-Di(1*H*-pyrazol-3-yl)imidazo[1,2-*a*]pyridine-2-carboxamide
• 6-(Furan-2-yl)-*N*-(1,3-oxazol-2-yl)imidazo[1,2-*a*]pyridine-2-carboxamide and its trifluoroacetate (1:1)
• 6-(Oxazol-2-yl)-*N*-(1,3,4-thiadiazol-2-yl)imidazo[1,2-*a*]pyridine-2-carboxamide
• 6-(Oxazol-2-yl)-*N*-(1*H*-pyrazol-3-yl)imidazo[1,2-*a*]pyridine-2-carboxamide
• 6-(Furan-3-yl)-*N*-(1,3-oxazol-2-yl)imidazo[1,2-*a*]pyridine-2-carboxamide and its trifluoroacetate (1:1)
• 6-[5-(Hydroxymethyl)furan-2-yl]-*N*-(1*H*-pyrazol-3-yl)imidazo[1,2-*a*]pyridine-2-carboxamide
• 6-(1*H*-Imidazol-4-yl)-*N*-(thiophen-3-yl)imidazo[1,2-*a*]pyridine-2-carboxamide
• 6-(1*H*-Imidazol-4-yl)-*N*-(1,3-thiazol-2-yl)imidazo[1,2-*a*]pyridine-2-carboxamide
• *N*-(Pyridin-2-yl)-6-(1*H*-1,2,3-triazol-4-yl)imidazo[1,2-*a*]pyridine-2-carboxamide
• *N*-(Thiophen-3-yl)-6-(1*H*-1,2,3-triazol-4-yl)imidazo[1,2-*a*]pyridine-2-carboxamide;
and the acid-addition salts thereof.

7. Medicament, **characterized in that** it comprises a compound of formula (I) according to any one of Claims 1 to 6, or an addition salt of this compound with a pharmaceutically acceptable acid.

8. Pharmaceutical composition, **characterized in that** it comprises a compound of formula (I) according to any one of Claims 1 to 6, or a pharmaceutically acceptable salt of this compound, and also at least one pharmaceutically acceptable excipient.

9. Use of a compound of formula (I) according to any one of Claims 1 to 6, for the preparation of a medicament for treating or preventing neurodegenerative diseases.

10. Use of a compound of formula (I) according to any one of Claims 1 to 6, for the preparation of a medicament for treating or preventing cerebral trauma and epilepsy.

11. Use of a compound of formula (I) according to any one of Claims 1 to 6, for the preparation of a medicament for treating or preventing psychiatric diseases.

12. Use of a compound of formula (I) according to any one of Claims 1 to 6, for the preparation of a medicament for treating or preventing inflammatory diseases.

13. Use of a compound of formula (I) according to any one of Claims 1 to 6, for the preparation of a medicament for treating or preventing osteoporosis.

14. Use of a compound of formula (I) according to any one of Claims 1 to 6, for the preparation of a medicament for treating or preventing cancers.

15. Use of a compound of formula (I) according to any one of Claims 1 to 6, for the preparation of a medicament for treating or preventing Parkinson's disease, Alzheimer's disease, tauopathies and multiple sclerosis.

16. Use of a compound of formula (I) according to any one of Claims 1 to 6, for the preparation of a medicament for treating or preventing schizophrenia, depression, substance dependency and attention-deficit hyperactivity disorder.

17. Compounds
6-(6-{[(1,1-Dimethylethoxy)carbonyl]amino}pyridin-2-yl)imidazo[1,2-a]pyridine-2-carboxylic acid
6-(Pyridin-2-yl)imidazo[1,2-a]pyridine-2-carboxylic acid
6-(1-Triphenylmethyl-1*H*-imidazol-4-yl)imidazo[1,2-*a*]pyridine-2-carboxylic acid
6-(2-{[(1,1-Dimethylethoxy)carbonyl]amino}thiazol-4-yl)imidazo[1,2-*a*]pyridine-2-carboxylic acid
6-(1*H*-Pyrrol-3-yl)imidazo[1,2-*a*]pyridine-2-carboxylic acid
6-(1*H*-Pyrazol-3-yl)imidazo[1,2-*a*]pyridine-2-carboxylic acid
6-(1*H*-Pyrazol-4-yl)imidazo[1,2-*a*]pyridine-2-carboxylic acid
6-(Furan-2-yl)imidazo[1,2-*a*]pyridine-2-carboxylic acid
6-(Furan-3-yl)imidazo[1,2-*a*]pyridine-2-carboxylic acid
6-[5-(Hydroxymethyl)furan-2-yl]imidazo[1,2-*a*]pyridine-2-carboxylic acid
6-(Thiophen-3-yl)imidazo[1,2-*a*]pyridine-2-carboxylic acid
6-(Oxazol-2-yl)imidazo[1,2-*a*]pyridine-2-carboxylic acid
6-(1*H*-1,2,4-Triazol-3-yl)imidazo[1,2-*a*]pyridine-2-carboxylic acid
6-(1*H*-1,2,3-Triazol-4-yl)imidazo[1,2-*a*]pyridine-2-carboxylic acid
6-Iodo-*N*-(isoxazol-4-yl)imidazo[1,2-*a*]pyridine-2-carboxamide
6-Iodo-*N*-(thiazol-2-yl)imidazo[1,2-*a*]pyridine-2-carboxamide
6-Iodo-*N*-(pyridin-2-yl)imidazo[1,2-*a*]pyridine-2-carboxamide
6-Trimethylstannyl-*N*-(pyridin-2-yl)imidazo[1,2-*a*]pyridine-2-carboxamide
Ethyl 6-(4,4,5,5-tetramethyl-1,3,2-dioxaborolan-2-yl)-imidazo[1,2-*a*]pyridine-2-carboxylate hydrobromide (1:1) 2-Ethoxycarbonylimidazo[1,2-*a*]pyridine-6-boronic acid.

18. Use of the compounds according to Claim 17, for the synthesis of products of general formula (I) as defined in Claim 1.

## Patentansprüche

1. Verbindungen der Formel (I): 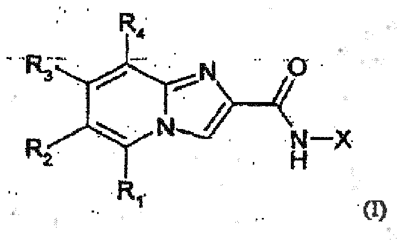 worin:
X für eine heterocyclische Gruppe steht, die gegebenenfalls durch eine oder mehrere Gruppen substituiert ist, die unabhängig voneinander unter den folgenden Atomen bzw. Gruppen ausgewählt sind: Halogen, (C₁-C₆)-Alkoxy, (C₁-C₆)-Alkyl, NRaRb, Cyano, Oxido, COOR₈, wobei die Alkyl- und Alkoxygruppen gegebenenfalls durch ein oder mehrere Halogenatome substituiert sein können;
R₁ für ein Wasserstoffatom, ein Halogenatom, eine (C₁-C₆)-Alkoxygruppe, eine (C₁-C₆)-Alkylgruppe, Amino oder NRaRb steht; wobei die Alkyl- und Alkoxygruppen gegebenenfalls durch ein oder mehrere Halogenatome, eine Hydroxygruppe, eine Aminogruppe oder eine (C₁-C₆)-Alkoxygruppe substituiert sein können;
R₂ für eine heterocyclische Gruppe steht, die gegebenenfalls durch eine oder mehrere Gruppen substituiert ist, die unabhängig voneinander unter den folgenden Atomen bzw. Gruppen ausgewählt sind: Hydroxy, (C₁-C₆) -Alkyl, (C₁-C₆) -Alkoxy, Halogen, Cyano, NRaRb, -CO-R₅, -CO-NR₆R₇, -CO-O-R₈, -NR₉-CO-R₁₀, wobei die (C₁-C₆)-Alkyl- und (C₁-C₆)-Alkoxygruppen gegebenenfalls durch ein bzw. eine oder mehrere Halogenatome oder Hydroxy-, NRaRb- oder Oxidogruppen substituiert sind;
R₃ für ein Wasserstoffatom, eine (C₁-C₆)-Alkylgruppe, eine (C₁-C₆) -Alkoxygruppe oder ein Halogenatom steht;
R₄ für ein Wasserstoffatom, eine (C₁-C₄)-Alkylgruppe, eine (C₁-C₄)-Alkoxygruppe oder ein Fluoratom steht;
R₅ für ein Wasserstoffatom, eine Phenylgruppe oder eine (C₁-C₆) -alkylgruppe steht;
R₆ und R₇ gleich oder verschieden sind und für ein Wasserstoffatom oder eine (C₁-C₆) -Alkylgruppe stehen oder mit dem Stickstoffatom, an das sie gebunden sind, einen 4- bis 7-gliedrigen Ring, der gegebenenfalls ein weiteres unter N, O und S ausgewähltes Heteroatom enthält, bilden;
R₈ für eine (C₁-C₆)-Alkylgruppe steht;
R₉ und R₁₀ gleich oder verschieden sind und für ein Wasserstoffatom oder eine (C₁-C₆) -Alkylgruppe stehen;
Ra und Rb unabhängig voneinander für ein Wasserstoffatom oder eine (C₁-C₆)-Alkylgruppe stehen oder mit dem Stickstoffatom, an das sie gebunden sind, einen 4- bis 7-gliedrigen Ring, der gegebenenfalls ein weiteres unter N, O und S ausgewähltes Heteroatom enthält, bilden;
eine heterocyclische Gruppe für eine mono- oder bicyclische Gruppe mit 5 bis 10 Atomen einschließlich 1 bis 4 Heteroatomen, die unter N, O und S ausgewählt sind, steht, wobei diese cyclische Gruppe aromatisch, ungesättigt oder teilweise ungesättigt oder oxidiert ist und über ein Kohlenstoffatom gebunden ist;
Racemate, Enantiomere, Diastereoisomere und Gemische davon;
in Basen- oder Säureadditionssalzform sowie in Hydrat- oder Solvatform.

2. Verbindungen der Formel (I) nach Anspruch 1, **dadurch gekennzeichnet, dass**:
X für eine heterocyclische Gruppe steht, die gegebenenfalls durch eine oder mehrere Gruppen substituiert ist, die unabhängig voneinander unter Halogenatomen ausgewählt sind,
in Basen- oder Säureadditionssalzform.

3. Verbindungen der Formel (I) nach Anspruch 1 oder 2, **dadurch gekennzeichnet, dass**:
R₁, R₃ und R₄ für ein Wasserstoffatom stehen,
in Basen- oder Säureadditionssalzform.

4. Verbindungen der Formel (I) nach einem der vorhergehenden Ansprüche, **dadurch gekennzeichnet, dass**:
R₂ für eine heterocyclische Gruppe steht, die gegebenenfalls durch eine oder mehrere Gruppen substituiert ist, die unabhängig voneinander unter den folgenden Atomen bzw. Gruppen ausgewählt sind: (C₁-C₆)-Alkyl, NRaRb, wobei die (C₁-C₆) -Alkylgruppe bzw. die (C₁-C₆)-Alkylgruppen gegebenenfalls durch ein bzw. eine oder mehrere Halogenatome oder Hydroxygruppen substituiert ist bzw. sind, wobei Rₐ and R_{b} unabhängig voneinander für ein Wasserstoffatom oder eine (C₁-C₆)-Alkylgruppe stehen; in Basen- oder Säureadditionssalzform.

5. Verbindungen der Formel (I) nach einem der vorhergehenden Ansprüche, **dadurch gekennzeichnet, dass**:
X für eine Pyridin-, Isoxazol-, Thiazol-, Thiadiazol-, Pyrazol-, Thiophen- oder Oxazolgruppe steht, wobei diese Gruppen gegebenenfalls durch ein Fluoratom substituiert sind,
R₂ für eine Pyridin-, Thiophen-, Imidazol-, Pyrrol-, Furan-, Oxazol-, Triazol- oder Pyrazolgruppe steht, wobei diese Gruppen gegebenenfalls durch eine NH₂- oder Hydroxymethylgruppe substituiert sind,
R₁, R₃ und R₄ für ein Wasserstoffatom stehen,
in Basen- oder Säureadditionssalzform.

6. Verbindung der Formel (I) nach einem der vorhergehenden Ansprüche, **dadurch gekennzeichnet, dass** sie unter:
• 6-(6-Aminopyridin-2-yl)-*N*-(pyridin-2-yl)-imidazo[1,2-*a*]pyridin-2-carboxamid und dessen Hydrochlorid (1:2)
• 6-(1*H*-Imidazol-4-yl)-*N*-(pyridin-2-yl)imidazo-[1,2-*a*]pyridin-2-carboxamid
• N,6-Di(pyridin-2-yl)imidazo[1,2-*a*]pyridin-2-carboxamid
• *N*-(Pyridin-2-yl)-6-(thiophen-3-yl)imidazo[1,2-*a*]pyridin-2-carboxamid
• *N*-(Isoxazol-4-yl)-6-(pyridin-2-yl)imidazo[1,2-*a*]pyridin-2-carboxamid
• *N*-(6-Fluoropyridin-2-yl)-6-(pyridin-2-yl)-imidazo[1,2-*a*]pyridin-2-carboxamid
• 6-(Pyridin-2-yl-*N*-(thiazol-2-yl)imidazo[1,2-*a*]-pyridin-2-carboxamid
• *N*-(Isoxazol-3-yl)-6-(pyridin-2-yl)imidazo[1,2-*a*]pyridin-2-carboxamid
• 6-(Pyridin-2-yl-*N*-(1,3,4-thiadiazol-2-yl)-imidazo[1,2-*a*]pyridin-2-carboxamid
• *N*-(1*H*-Pyrazol-3-yl)-6-(pyridin-2-yl)imidazo-[2,2-*a*]pyridin-2-carboxamid
• 6-(Pyridin-2-yl-*N*-(thiophen-3-yl)imidazo[1,2-*a*]pyridin-2-carboxamid
• 6-(1*H*-Pyrrol-3-yl)-*N*-(thiazol-2-yl)imidazo[1,2-*a*]pyridin-2-carboxamid
• *N*-(Isoxazol-4-yl)-6-(1*H*-pyrazol-3-yl)imidazo-[1,2-*a*]pyridin-2-carboxamid
• 6-(Furan-2-yl)-*N*-(thiazol-2-yl)imidazo[1,2-*a*]-pyridin-2-carboxamid
• 6-(Furan-2-yl)-*N*-(isoxazol-4-yl)imidazo[1,2-*a*]-pyridin-2-carboxamid
• 6-(Oxazol-2-yl)-*N*-(thiazol-2-yl)imidazo[1,2-*a*]-pyridin-2-carboxamid
• *N*-(Isoxazol-3-yl)-6-(oxazol-2-yl)imidazo[1,2-*a*]pyridin-2-carboxamid
• *N*-(Isoxazol-4-yl)-6-(oxazol-2-yl)imidazo[1,2-*a*]pyridin-2-carboxamid
• 6-(Furan-3-yl)-*N*-(thiazol-2-yl)imidazo[1,2-*a*]-pyridin-2-carboxamid
• 6-(Furan-3-yl)-*N*-(isoxazol-4-yl)imidazo[1,2-*a*]-pyridin-2-carboxamid
• 6-[5-(Hydroxymethyl)furan-2-yl]-*N*-(thiazol-2-yl)imidazo[1,2-*a*]pyridin-2-carboxamid
• 6-[5-(Hydroxymethyl)furan-2-yl]-*N*-(isoxazol-3-yl)imidazo[1,2-*a*]pyridin-2-carboxamid
• 6-[5-(Hydroxymethyl)furan-2-yl]-*N*-(isoxazol-4-yl)imidazo[1,2-*a*]pyridin-2-carboxamid
• 6-(1*H*-Imidazol-4-yl)-*N*-(isoxazol-3-yl)imidazo-[1,2-*a*]pyridin-2-carboxamid
• 6-(6-Aminopyridin-2-yl)-*N*-(thiazol-2-yl)-imidazo[1,2-*a*]pyridin-2-carboxamid
• 6-(1*H*-Pyrrol-3-yl)-*N*-(thiophen-3-yl)imidazo-[1,2-*a*]pyridin-2-carboxamid
• 6-(Furan-2-yl)-*N*-(pyridin-2-yl)imidazo[1,2-*a*] - pyridin-2-carboxamid
• *N*-(6-Fluoropyridin-2-yl)-6-(furan-2-yl)imidazo-[1,2-*a*]pyridin-2-carboxamid
• 6-(Furan-2-yl)-*N*-(thiophen-3-yl)imidazo[1,2-*a*]-pyridin-2-carboxamid
• 6-(Furan-2-yl)-*N*-(isoxazol-3-yl)imidazo[1,2-*a*]-pyridin-2-carboxamid
• 6-(Furan-2-yl)-*N*-(1,3,4-thiadiazol-2-yl)-imidazo[1,2-*a*]pyridin-2-carboxamid
• 6-(Oxazol-2-yl)-*N*-(pyridin-2-yl)imidazo[1,2-*a*]-pyridin-2-carboxamid
• *N*-(6-Fluorpyridin-2-yl)-6-(1,3-oxazol-2-yl)-imidazo[1,2-*a*]pyridin-2-carboxamid
• 6-(Oxazol-2-yl)-*N*-(thiophen-3-yl)imidazo[1,2-*a*]pyridin-2-carboxamid
• 6-(Furan-3-yl)-*N*-(pyridin-2-yl)imidazo[1,2-*a*]-pyridin-2-carboxamid
• *N*-(6-Fluorpyridin-2-yl)-6-(furan-3-yl)imidazo-[1,2-*a*]pyridin-2-carboxamid
• 6-(Furan-3-yl)-*N*-(thiophen-3-yl)imidazo[1,2-*a*]-pyridin-2-carboxamid
• 6-(Furan-3-yl)-*N*-(isoxazol-3-yl)imidazo[1,2-*a*]-pyridin-2-carboxamid
• 6-(Furan-3-yl)-*N*-(1,3,4-thiadiazol-2-yl)-imidazo[1,2-*a*]pyridin-2-carboxamid
• 6-[5-(Hydroxymethyl)furan-2-yl]-*N*-(pyridin-2-yl)imidazo[1,2-*a*]pyridin-2-carboxamid
• 6-[5-(Hydroxymethyl)furan-2-yl]-*N*-thiophen-3-yl)imidazo[1,2-*a*]pyridin-2-carboxamid
• 6-(Furan-2-yl)-*N*-(1*H*-pyrazol-3-yl)imidazo[1,2-*a*]pyridin-2-carboxamid
• *N*-(Thiophen-3-yl)-6-(1*H*-1,2,4-triazol-3-yl)-imidazo[1,2-*a*]pyridin-2-carboxamid
• 6-(6-Aminopyridin-2-yl)-*N*-(6-fluorpyridin-2-yl)imidazo[1,2-*a*]pyridin-2-carboxamid und dessen Hydrochlorid (1:2)
• 6-(6-Aminopyridin-2-yl)-*N*-(thiophen-3-yl)-imidazo[1,2-*a*]pyridin-2-carboxamid und dessen Hydrochlorid (1:2)
• 6-(6-Aminopyridin-2-yl)-*N*-(isoxazol-3-yl - imidazo[1,2-*a*]pyridin-2-carboxamid und dessen Hydrochlorid (1:2)
• 6-(6-Aminopyridin-2-yl)-*N*-(1,3,4-thiadiazol-2-yl)imidazo[1,2-*a*]pyridin-2-carboxamid und dessen Hydrochlorid (1:2)
• 6-(6-Aminopyridin-2-yl)-*N*-(isoxazol-4-yl)-imidazo[1,2-*a*]pyridin-2-carboxamid und dessen Hydrochlorid (1:2)
• *N*-(Pyridin-2-yl)-6-(1*H*-pyrrol-3-yl)imidazo[1,2-*a*]pyridin-2-carboxamid und dessen Trifluoracetat (1:1)
• *N*-(Isoxazol-3-yl)-6-(1*H*-pyrrol-3-yl)imidazo-[1,2-*a*]pyridin-2-carboxamid
• 6-(1*H*-Pyrrol-3-yl)-*N*-(1,3,4-thiadiazol-2-yl)-imidazo[1,2-*a*]pyridin-2-carboxamid
• *N*-(Oxazol-2-yl)-6-(1*H*-pyrrol-3-yl)imidazo[1,2-*a*]pyridin-2-carboxamid
• *N*-(Isoxazol-4-yl)-6-(1*H*-pyrrol-3-yl)imidazo-[1,2-*a*]pyridin-2-carboxamid
• 6-(1*H*-Pyrazol-3-yl)-*N*-(pyridin-2-yl)imidazo-[1,2-*a*]pyridin-2-carboxamid
• 6-(1*H*-Pyrazol-3-yl)-*N*-(thiophen-3-yl)imidazo-[1,2-*a*]pyridin-2-carboxamid und dessen Trifluoracetat (1:1)
• 6-(1*H*-Pyrazol-3-yl)-*N*-(1,3-thiazol-2-yl)-imidazo[1,2-*a*]pyridin-2-carboxamid und dessen Trifluoracetat (1:1)
• *N*-(Isoxazol-3-yl)-6-(1*H*-pyrazol-3-yl)imidazo-[1,2-*a*]pyridin-2-carboxamid
• 6-(1*H*-Pyrazol-3-yl)-*N*-(1,3,4-thiadiazol-2-yl)-imidazo[1,2-*a*]pyridin-2-carboxamid und dessen Trifluoracetat (1:1)
• N,6-Di(1*H*-pyrazol-3-yl)imidazo[1,2-*a*]pyridin-2-carboxamid
• 6-(Furan-2-yl)-*N*-(1,3-oxazol-2-yl)imidazo[1,2-*a*]pyridin-2-carboxamid und dessen Trifluoracetat (1:1)
• 6-(Oxazol-2-yl)-*N*-(1,3,4-thiadiazol-2-yl)-imidazo[1,2-*a*]pyridin-2-carboxamid
• 6-(Oxazol-2-yl)-*N*-(1*H*-pyrazol-3-yl)imidazo[1,2-*a*]pyridin-2-carboxamid
• 6-(Furan-3-yl)-*N*-(1,3-oxazol-2-yl)imidazo[1,2-*a*]pyridin-2-carboxamid und dessen Trifluoracetat (1:1)
• 6-[5-(Hydroxymethyl)furan-2-yl]-*N*-(1*H*-pyrazol-3-yl)imidazo[1,2-*a*]pyridin-2-carboxamid
• 6-(1*H*-Imidazol-4-yl)-*N*-(thiophen-3-yl)imidazo-[1,2-*a*]pyridin-2-carboxamid
• 6-(1*H*-Imidazol-4-yl)-*N*-(1,3-thiazol-2-yl)-imidazo[1,2-*a*]pyridin-2-carboxamid
• *N*-(Pyridin-2-yl)-6-(1*H*-1,2,3-triazol-4-yl)-imidazo[1,2-*a*]pyridin-2-carboxamid
• *N*-(Thiophen-3-yl)-6-(1*H*-1,2,3-triazol-4-yl)-imidazo[1,2-*a*]pyridin-2-carboxamid
und den Säureadditionssalzen davon ausgewählt ist.

7. Medikament, **dadurch gekennzeichnet, dass** es eine Verbindung der Formel (I) nach einem der Ansprüche 1 bis 6 oder ein Additionssalz dieser Verbindung mit einer pharmazeutisch unbedenklichen Säure enthält.

8. Pharmazeutische Zusammensetzung, **dadurch gekennzeichnet, dass** sie eine Verbindung der Formel (I) nach einem der Ansprüche 1. bis 6 oder ein pharmazeutisch unbedenkliches Salz dieser Verbindung sowie mindestens einen pharmazeutisch unbedenklichen Hilfsstoff enthält.

9. Verwendung einer Verbindung der Formel (I) nach einem der Ansprüche 1 bis 6 zur Herstellung eines Medikaments zur Behandlung und Prävention von neurodegenerativen Erkrankungen.

10. Verwendung einer Verbindung der Formel (I) nach einem der Ansprüche 1 bis 6 zur Herstellung eines Medikaments zur Behandlung und Prävention von Hirntraumen und Epilepsie.

11. Verwendung einer Verbindung der Formel (I) nach einem der Ansprüche 1 bis 6 zur Herstellung eines Medikaments zur Behandlung und Prävention von psychiatrischen Erkrankungen.

12. Verwendung einer Verbindung der Formel (I) nach einem der Ansprüche 1 bis 6 zur Herstellung eines Medikaments zur Behandlung und Prävention von entzündlichen Erkrankungen.

13. Verwendung einer Verbindung der Formel (I) nach einem der Ansprüche 1 bis 6 zur Herstellung eines Medikaments zur Behandlung und Prävention von Osteoporose.

14. Verwendung einer Verbindung der Formel (I) nach einem der Ansprüche 1 bis 6 zur Herstellung eines Medikaments zur Behandlung und Prävention von Krebserkrankungen.

15. Verwendung einer Verbindung der Formel (I) nach einem der Ansprüche 1 bis 6 zur Herstellung eines Medikaments zur Behandlung und Prävention von Parkinson-Krankheit, Alzheimer-Krankheit, Tauopathien und multipler Sklerose.

16. Verwendung einer Verbindung der Formel (I) nach einem der Ansprüche 1 bis 6 zur Herstellung eines Medikaments zur Behandlung und Prävention von Schizophrenie, Depression, Substanzabhängigkeit und Aufmerksamkeitsdefizit-Hyperaktivitätssyndrom.

17. Verbindungen
6-(6-{[(1,1-Dimethylethoxy)carbonyl]amino}pyridin-2-yl)imidazo[1,2-a]pyridin-2-carbonsäure
6-(Pyridin-2-yl)imidazo[1,2-*a*]pyridin-2-carbonsäure
6-(1-Triphenylmethyl-1*H*-imidazol-4-yl)imidazo[1,2-a]pyridin-2-carbonsäure
6-(2-{[(1,1-Dimethylethoxy)carbonyl]amino}thiazol-4-yl)imidazo[1,2-*a*]pyridin-2-carbonsäure
6-(1*H*-Pyrrol-3-yl)imidazo[1,2-a]pyridin-2-carbonsäure
6- (1*H*-pyrazol-3-yl)imidazo[1,2-*a*]pyridin-2-carbonsäure
6-(1*H*-Pyrazol-4-yl)imidazo[1,2-*a*]pyridin-2-carbonsäure
6-(Furan-2-yl)imidazo[1,2-*a*]pyridin-2-carbonsäure
6-(Furan-3-yl)imidazo[1,2-*a*]pyridin-2-carbonsäure
6-[5-(Hydroxymethyl)furan-2-yl]imidazo[1,2-*a*]-pyridin-2-carbonsäure
6-(Thiophen-3-yl)imidazo[1,2-*a*]pyridin-2-carbonsäure
6-(Oxazol-2-yl)imidazo[1,2-*a*]pyridin-2-carbonsäure
6-(1*H*-1,2,4-Triazol-3-yl)imidazo[1,2-*a*]pyridin-2-carbonsäure
6-(1*H*-1,2,3-Triazol-4-yl)imidazo[1,2-*a*]pyridin-2-carbonsäure
6-Iod-*N*-(isoxazol-4-yl)imidazo[1,2-*a*]pyridin-2-carboxamid
6-Iod-*N*-(thiazol-2-yl)imidazo [1,2-*a*]pyridin-2-carboxamid
6-Iod-*N*-(pyridin-2-yl)imidazo[1,2-*a*]pyridin-2-carboxamid
6-Trimethylstannyl-*N*-(pyridin-2-yl)imidazo[1,2-*a*]-pyridin-2-carboxamid
6-(4,4,5,5-tetramethyl-1,3,2-dioxaborolan-2-yl)-imidazo[1,2-*a*]pyridin-2-carbonsäureethylester-hydrobromid (1:1)
2-Ethoxycarbonylimidazo[1,2-*a*]pyridin-6-boronsäure.

18. Verwendung der Verbindungen nach Anspruch 17 zur Synthese von Produkten der allgemeinen Formel (I) gemäß Anspruch 1.
